# EUROPEAN PATENT SPECIFICATION

(11) **EP 3 802 542 B1**
(45) Date of publication and mention of the grant of the patent: **28.05.2025**
(21) Application number: 19731171.5
(22) Date of filing: 06.06.2019
(51) Int. Cl.: C07D 487/04, A01N 43/90

(54) **PESTICIDALLY ACTIVE HETEROCYCLIC DERIVATIVES WITH SULFOXIMINE CONTAINING SUBSTITUENTS**
HETEROCYCLISCHE DERIVATEN MIT SCHWEFEL SUBSTITENTEN ALS PESTIZIDE
DÉRIVÉS HÉTÉROCYCLIQUES AVEC SUBSTITUENTS DE SOUFRE ET LEUR UTILISATION COMME PESTICIDES

(30) Priority: 06.06.2018 IN 201811021198; 30.08.2018 EP 18191693
(43) Date of publication of application: 14.04.2021
(60) Divisional application: 24156865.8
(73) Proprietor: Syngenta Crop Protection AG, 4058 Basel (CH)
(72) Inventor: EDMUNDS, Andrew, 4332 Stein (CH); MUEHLEBACH, Michel, 4332 Stein (CH); RENDLER, Sebastian, 4332 Stein (CH); BUCHHOLZ, Anke, 4332 Stein (CH); EMERY, Daniel, 4332 Stein (CH); SIKERVAR, Vikas, Ilhas Goa 403 110 (IN); RAWAL, Girish, Ilhas Goa 403 110 (IN); SEN, Indira, Ilhas Goa 403 110 (IN)
(86) International application number: PCT/EP2019/064795
(87) International publication number: WO 2019/234158

(56) References cited:
- EP-A1- 3 252 046

## Description

The present invention relates to pesticidally active, in particular insecticidally active heterocyclic derivatives containing sulfoximine substituents, to processes for their preparation, to compositions comprising those compounds, and to their use for controlling animal pests, including arthropods and in particular insects or representatives of the order *Acarina.*

Pesticidally active hetero-bicyclic substituted cyclopropyl derivatives with sulfur-containing substitutents are known and described in the literature, for example, in WO 2018/077565 , WO 2018/070502, WO 2017/146226, WO 2017/089190, WO 2017/084879, WO 2016/121997, WO 2016/104746, WO 2016/096584, WO 2016/046071, WO 2016/071214, WO 2016/039441, WO 2016/026848, WO 2016/023954, WO 2014/142292, and WO 2016/020286. Pesticidally active heterocyclic sulfoximine derivatives have previously been described in the literature, for example, in WO 2015/071180.

It has now surprisingly been found that certain novel bicyclic sulfoximine-containing substituted cyclopropyl derivatives have favorable properties as pesticides.

The present invention relates to compounds of formula (I) 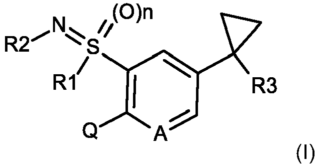 wherein
A is CH or N;
R₁ is C₁-C₄-alkyl;
R₂ is hydrogen, cyano, -C(O)R₇, -C(O)OR₈, C₁-C₆-alkyl, -CONR₉R₁₀, or SO₂R₁₁;
wherein
R₇ is hydrogen, C₁-C₆-alkyl, or C₁-C₆-haloalkyl; and R₈ is C₁-C₆-alkyl or C₁-C₆-haloalkyl;
R₉, R₁₀ independently of one another are hydrogen or C₁-C₆-alkyl;
R₁₁ is C₁-C₆-alkyl;
R₃ is hydrogen, C₁-C₃-alkyl, C₁-C₃-haloalkyl, cyano, -CO₂H, -CO₂NH₂, C₁-C₄alkoxycarbonyl, C₁-C₄-alkylaminocarbonyl, or C₁-C₄-dialkylaminocarbonyl;
n is 0 or 1;
Q is a radical selected from the group consisting of formulae Q₂, Q₃, Q₄ and Q₅ 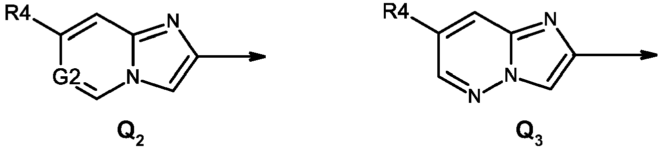 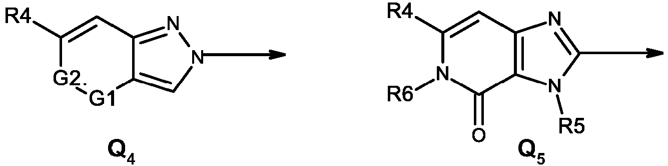
wherein the arrow denotes the point of attachment to the ring incorporating the radical A;
and wherein
R₄ is trifluoromethyl, trifluoromethylsulfanyl or trifluoromethylsulfonyl;
R₅ is C₁-C₄-alkyl;
R₆ is OCH₃, CH₂CH₃ or cyclopropyl; and
G₁ and G₂ are, independently from each other, N or CH;
or an agrochemically acceptable salt, stereoisomer, enantiomer, tautomer or N-oxide of a compound of formula I.

Compounds of formula I which have at least one basic centre can form, for example, acid addition salts, for example with strong inorganic acids such as mineral acids, for example perchloric acid, sulfuric acid, nitric acid, nitrose acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, isopropyl, n-butyl, sec-butyl, iso-butyl, tert-butyl, pentyl, hexyl, and their branched isomers. For example, there also may be mentioned any one of the radicals 1-methylbutyl, 2-methylbutyl, 3-methylbutyl, 2, 2-dimethylpropyl, 1-ethylpropyl, n-hexyl, n-pentyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl. As noted below, haloalkyl, haloalkylsulfanyl, haloalkylsulfinyl, haloalkylsulfonyl, alkoxy and haloalkoxy radicals are derived from the alkyl radicals.

Halogen is generally fluorine, chlorine, bromine or iodine. This also applies, correspondingly, to halogen in combination with other meanings, such as haloalkyl.

The term "C₁-Cₙhaloalkyl" as used herein refers to a straight-chain or branched saturated C₁-Cₙalkyl radical having 1 to n carbon atoms (as mentioned above), where some or all of the hydrogen atoms in these radicals may be replaced by fluorine, chlorine, bromine and/or iodine, i.e., for example,, for example, any one of of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3- pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl 1,1-difluoro-2,2,2-trichloroethyl, and 2,2,3,3-tetrafluoroethyl; preferably trichloromethyl, difluorochloromethyl, difluoromethyl, trifluoromethyl and dichlorofluoromethyl.

The term "C₁-Cₙalkoxy" as used herein refers to a straight-chain or branched saturated C₁-Cₙalkyl radical having 1 to n carbon atoms (as mentioned above) which is attached via an oxygen atom, i.e., for example, any one of the radicals methoxy, ethoxy, propoxy, i-propoxy, n-butoxy, isobutoxy, sec-butoxy, tert-butoxy, 1-methylpropoxy, 2-methylpropoxy and 1,1-dimethylethoxy and also the isomeric pentyloxy and hexyloxy radicals; preferably methoxy and ethoxy.

The term "C₁-Cₙhaloalkoxy" as used herein refers to a straight-chain or branched saturated C₁-Cₙhaloalkyl radical having 1 to n carbon atoms (as mentioned above) which is attached via an oxygen atom similar to C₁-Cₙalkoxy.

The term "C₁-Cₙalkylsulfanyl" as used herein refers to a straight chain or branched saturated alkyl radical having 1 to n carbon atoms (as mentioned above) which is attached via a sulfur atom, i.e., for example, any one of methylthio, ethylthio, n-propylthio, 1-methylethylthio, butylthio, 1-methylpropylthio, 2-methylpropylthio or 1,1-dimethylethylthio.

The term "C₁-Cₙhaloalkylsulfanyl" as used herein refers to a C₁-Cₙalkylsulfanyl radical as mentioned above which is partially or fully substituted by fluorine, chlorine, bromine and/or iodine, i.e., for example, any one of fluoromethylthio, difluoromethylthio, trifluoromethylthio, chlorodifluoromethylthio, bromodifluoromethylthio, 2-fluoroethylthio, 2-chloroethylthio, 2-bromoethylthio, 2-iodoethylthio, 2,2-difluoroethylthio, 2,2,2-trifluoroethylthio, 2,2,2-trichloroethylthio, 2-chloro-2-fluoroethylthio, 2-chloro-2,2-difluoroethylthio, 2,2-dichloro-2-fluoroethylthio, pentafluoroethylthio, 2-fluoropropylthio, 3-fluoropropylthio, 2-chloropropylthio, 3-chloropropylthio, 2-bromopropylthio, 3-bromopropylthio, 2,2-difluoropropylthio, 2,3-difluoropropylthio, 2,3-dichloropropylthio, 3,3,3- trifluoropropylthio, 3,3,3-trichloropropylthio, 2,2,3,3,3-pentafluoropropylthio, heptafluoropropylthio, 1-(fluoromethyl)-2-fluoroethylthio, 1-(chloromethyl)-2-chloroethylthio, 1-(bromomethyl)-2-bromoethylthio, 4-fluorobutylthio, 4-chlorobutylthio, or 4- bromobutylthio.

Similar considerations apply to the terms "C₁-Cₙhaloalkylsulfinyl" and "C₁-Cₙhaloalkylsulfonyl" which refer to the C₁-Cₙhaloalkylsulfanyl (as mentioned above), but with the sulfur in a different oxidation state, for example, sulfoxide -S(O)C₁-Cₙhaloalkyl or sulfone -S(O)₂C₁-Cₙhaloalkyl, respectively. Accordingly, for example, groups such as trifluoromethylsulfinyl, trifluoromethylsulfonyl or 2,2,2-trifluoroethylsulfonyl.

The term "C₃-C₆cycloalkyl" as used herein refers to 3-6 membered cycloalkyl groups such as cyclopropane, cyclobutane, cyclopropane, cyclopentane and cyclohexane.

The term "C₁-Cₙalkoxycarbonyl" refers to a straight-chain or branched saturated C₁-Cₙalkoxy radical having 1 to n carbon atoms (as mentioned above) which is attached via a carbonyl group.

The term "C₁-Cₙalkylaminocarbonyl" refers to a straight-chain or branched saturated C₁-Cₙalkyl radical having 1 to n carbon atoms (as mentioned above) which is attached via the nitrogen atom of an aminocarbonyl group.

The term "C₁-Cₙ dialkylaminocarbonyl" refers to two straight-chain or branched saturated C₁-Cₙalkyl radicals having 1 to n carbon atoms (as mentioned above), the same or different, which are attached via the nitrogen atom of an aminocarbonyl group

Terminal single bonds (Free radicals) represent methyl groups within the context of a given molecular structure or represent a point of attachment in the context of a variable group definition.

The compounds of formula I according to the present invention also include hydrates which may be formed during the salt formation.

Embodiments according to the invention, wherein R₁, R₂, R₃, R₄, Q (Q₂, Q₃, Q₄, Q₅), R₅, R₆, R₇, R₈, R₉, R₁₀, R₁₁, A, G₁ and G₂ are defined as for compounds of formula (I), according to the present invention, are provided as set out below.

Compounds of formula I, wherein Q is Q₁ 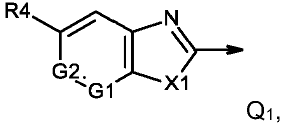 are reference examples.

In one embodiment, the present invention provides compounds of formula I, or an agrochemically acceptable salt, stereoisomer, enantiomer, tautomer or N-oxide thereof, as defined above.

Preferably, the present invention provides compounds of formula I wherein:
A is CH or N;
R₁ is ethyl, propyl or isopropyl;
R₂ is is hydrogen, cyano, C₁-C₃-alkyl, C₁-C₃-alkylcarbonyl, C₁-C₃-alkoxycarbonyl, or C₁-C₃-haloalkylcarbonyl;
R₃ is hydrogen, C₁-C₃-haloalkyl, cyano, -CO₂H, -CO₂NH₂, C₁-C₄-dialkylaminocarbonyl; and
n is 1.

More preferably, the present invention provides compounds of formula I wherein:
A is CH or N;
R₁ is ethyl;
R₂ is hydrogen;
R₃ is hydrogen, C₁-C₂-haloalkyl, cyano, -CO₂NH₂, or C₁-C₂-dialkylaminocarbonyl; and
n is 1.

Further preferred are compounds of formula I wherein:
A is CH or N;
R₁ is ethyl;
R₂ is hydrogen;
R₃ is hydrogen, cyano or CO₂NH₂; and
n is 1.

Most preferably, the present invention provides compounds of formula I wherein:
A is CH or N;
R₁ is ethyl;
R₂ is hydrogen;
R₃ is hydrogen or cyano; and
n is 1.

In one embodiment, the present invention provides compounds of formula I, wherein:
Q is a radical selected from Q₂, Q₄ and Q₅ 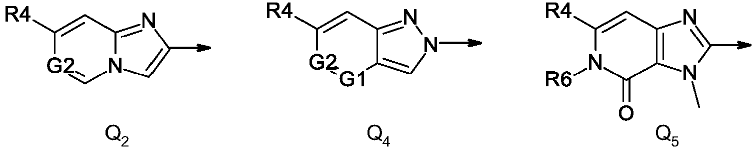
wherein the arrow denotes the point of attachment to the ring incorporating the radical A;
and wherein
R₄ is trifluoromethyl, trifluoromethylsulfanyl or trifluoromethylsulfonyl;
R₆ is OCH₃, CH₂CH₃ or cyclopropy; and
G₁ and G₂ are, independently from each other, N or CH.

In a further embodiment, the present invention provides compounds of formula I wherein:
Q is a radical selected from Q₂ and Q₅ 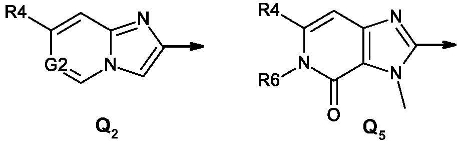
wherein the arrow denotes the point of attachment to the ring incorporating the radical A;
and wherein
R₄ is trifluoromethyl, trifluoromethylsulfanyl or trifluoromethylsulfonyl
R₆ is OCH₃, CH₂CH₃ or cyclopropyl; and
G₂ is N or CH.

The present invention also provides compounds of formula I wherein:
Q is a radical selected from Q₅ 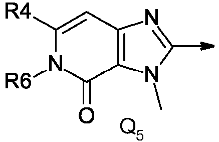
wherein the arrow denotes the point of attachment to the ring incorporating the radical A;
and wherein
R₄ is trifluoromethyl, trifluoromethylsulfanyl or trifluoromethylsulfonyl; and
R₆ is ethyl, methoxy or cyclopropyl.

The present invention further provides compounds of formula Iwherein:
A is CH or N;
R₁ is ethyl, propyl or isopropyl;
R₂ is is hydrogen, cyano, C₁-C₃ alkyl, C₁-C₃alkylcarbonyl, C₁-C₃alkoxycarbonyl, C₁-C₃haloalkylcarbonyl;
R₃ is hydrogen, C₁-C₃haloalkyl, cyano, CO₂H, CO₂NH₂, C₁-C₄dialkylaminocarbonyl;
n is 1;
Q is a radical selected from Q₂, Q₄ and Q₅ 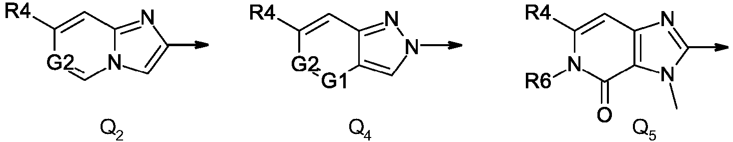
wherein the arrow denotes the point of attachment to the ring incorporating the radical A;
and wherein
R₄ is trifluoromethyl, trifluoromethylsulfanyl or trifluoromethylsulfonyl;
R₆ is OCH₃, CH₂CH₃ or cyclopropyl; and
G₁ and G₂ are, independently from each other, N or CH.

In a preferred embodiment, the present invention relates to compounds of formula I wherein:
A is CH or N;
R₁ is ethyl;
R₂ is hydrogen;
R₃ is hydrogen, C₁-C₂haloalkyl, cyano, CO₂NH₂, C₁-C₂dialkylaminocarbonyl;
n is 1;
Q is a radical selected from Q₂ and Q₅ 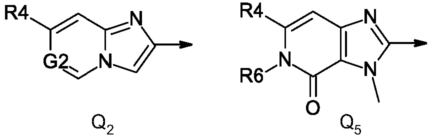
wherein the arrow denotes the point of attachment to the ring incorporating the radical A;
and wherein
R₄ is trifluoromethyl, trifluoromethylsulfanyl or trifluoromethylsulfonyl
R₆ is OCH₃, CH₂CH₃ or cyclopropyl; and
G₂ is N or CH.

Further preferred are compounds of formula I wherein:
A is CH or N;
R₁ is ethyl;
R₂ is hydrogen;
R₃ is hydrogen, cyano or CO₂NH₂;
n is 1;
Q is a radical selected from Q₅ 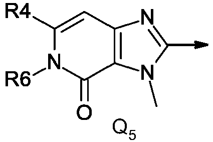
wherein the arrow denotes the point of attachment to the ring incorporating the radical A;
and wherein
R₄ is trifluoromethyl, trifluoromethylsulfanyl or trifluoromethylsulfony; and
R₆ is ethyl, methoxy or cyclopropyl.

Also preferred are compounds of formula I wherein:
Q is a radical selected from Q₅ 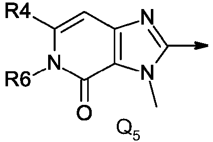
wherein the arrow denotes the point of attachment to the ring incorporating the radical A;
R₄ is trifluoromethyl, trifluoromethylsulfanyl or trifluoromethylsulfonyl; and
R₆ is OCH₃, CH₂CH₃ or cyclopropyl.

Further preferred are compounds of formula Iwherein:
Q is a radical Q₂ 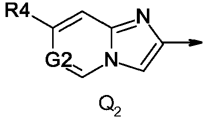
wherein the arrow denotes the point of attachment to the ring incorporating the radical A;
R₄ is trifluoromethyl, trifluoromethylsulfanyl or trifluoromethylsulfonyl; and
G₂ is N or CH.

Further preferred are compounds of formula I wherein:
Q is Q₃; and
R₄ is trifluoromethyl, trifluoromethylsulfanyl or trifluoromethylsulfonyl.

In another embodiment, there are provided compounds of formula I wherein:
Q is Q₄ 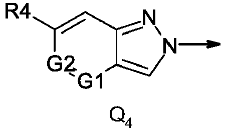
wherein the arrow denotes the point of attachment to the ring incorporating the radical A;
R₄ is trifluoromethyl, trifluoromethylsulfanyl or trifluoromethylsulfonyl; and
G₁ is N and G₂ is CH, G₁ is CH and G₂ is N, G₁ and G₂ are CH, or G₁ and G₂ are N; preferably G₁ is CH and G₂ is N.

In another aspect the present invention provides a composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of the foregoing embodiments 1 - 19, and, optionally, an auxiliary or diluent.

In a further aspect the present invention provides a method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of the foregoing embodiments 1 - 19 or a composition as defined above.

In a yet further aspect, the present invention provides a method for the protection of plant propagation material from the attack by insects, acarines, nematodes or molluscs, which comprises treating the propagation material or the site, where the propagation material is planted, with a composition as defined above.

The process according to the invention for preparing compounds of formula (I), wherein R₁, R₂, R₃, R₄, Q (Q₂, Q₃, Q₄, Q₅), R₅, R₆, R₇, R₈, R₉, R₁₀, R₁₁, A, G₁ and G₂ are defined as for compounds of formula (I) according to the present invention; and wherein compounds of formula (I) wherein Q is Q1 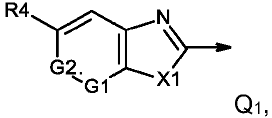 are reference examples; is carried out in principle by methods known to those skilled in the art, and as further described below.

The compounds of formula (I) according to the present invention can be made as shown in the following Schemes, in which, unless otherwise stated, the definition of each variable is as defined above for compounds of formula (I) according to the present invention.

The subgroup of compounds of the formula I wherein n is 0, i.e. sulfilimine la, wherein R₁, R₂, R₃, A, and Q are as defined above, may be prepared by reacting the corresponding sulfide of the formula II, wherein R₁, R₂, R₃, A, and Q are as defined above, under imination reaction conditions (step A, Scheme 1). The particular subgroup of compounds of the formula I wherein n is 1, defined as the sulfoximine I, wherein R₁, R₂, R₃, A, and Q are as defined above, may be obtained by oxidation of the sulfilimine compounds of the formula la, wherein R₁, R₂, R₃, A, and Q are as defined above (step B).

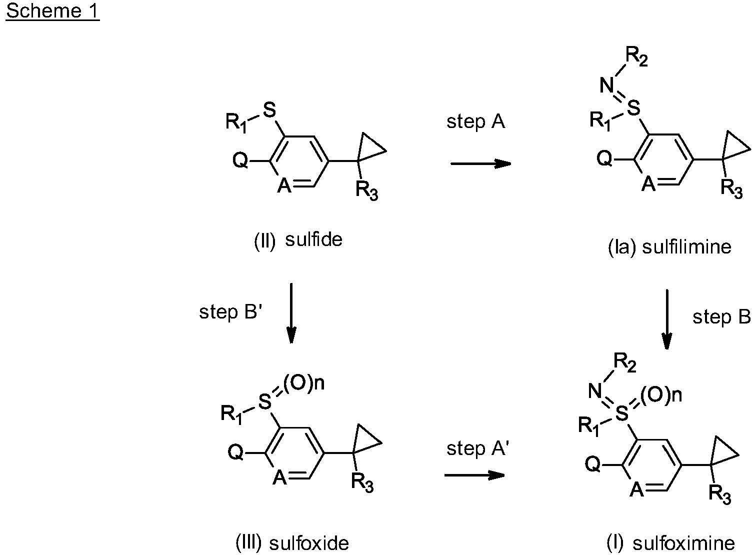

Conversely, the order of the two steps may be reverted whereby the sulfoximine compounds of the formula I, wherein R₁, R₂, R₃, A, and Q are as defined above are as defined above, may be prepared from sulfoxides of the formula III, wherein R₁, R₂, R₃, A, and Q are as defined above, under appropriate imination reaction conditions (step A').

Typical preparation methods and reaction conditions to access the compounds of the formula I, either towards the sulfilimines la (step A) or the sulfoximines I (step A'), may be found, for example, in H. Okamura, C. Bolm, Org. Lett., 2004, 6, 1305-1307; H. Okamura, C. Bolm, Chem. Lett., 2004, 33, 482-487; D. Leca, K. Song, M. Amatore, L. Fensterbank, E. Lacôte, M. Malacria, Chem. Eur. J., 2004, 10, 906-916; or M. Reggelin, C. Zur, Synthesis, 2000, 1-64. Typical imination reagents/conditions may be defined as NaN₃/H₂SO₄, O-mesitylenesulfonyl-hydroxylamine (MSH), or metal-catalyzed methods [see O.G. Mancheno, C. Bolm, Chem. Eur. J., 2007, 13, 6674-6681] such as R₂-N₃/FeCl₂, R₂-NH₂/Fe(acac)₃/PhI=O, PhI=N-R₂/ Fe(OTf)₂, PhI=N-R₂/CuOTf, PhI=N-R₂/Cu(OTf)₂, PhI=N-R₂/CuPF₆, PhI(OAc)₂/R₂-NH₂/ MgO/Rh₂(OAc)₄ or oxaziridines (e.g. 3-(4-cyano-phenyl)-oxaziridine-2-carboxylic acid tert-butyl ester).

Of particular interest are metal-free imination methods of sulfides of the formula II and/or sulfoxides of the formula III to prepare sulfilimines of the formula la (step A) and/or sulfoximines of the formula I (step A'). Such imination reactions involve R₂-NH₂ and an oxidant, for example, PhI(OAc)₂/R₂-NH₂ as described in G.Y. Cho, C. Bolm, Tetrahedron Lett., 2005, 46, 8007-8008; or N-bromosuccinimide (NBS)/R₂-NH₂ and a base such as sodium or potassium ter-butoxide as described in C. Bolm et al., Synthesis, 2010, No 17, 2922-2925. Oxidants such as N-iodosuccinimide (NIS) or iodine may be also used alternatively as described, for example, in O.G. Mancheno, C. Bolm, Org. Lett. 2007, 9, 3809-3811. An example of hypochlorite salts being used as oxidant, such as sodium hypochlorite NaOCl or calcium hypochlorite Ca(OCl)₂, was described in WO2008/106006.

For the transformation of a sulfilimine la to a sulfoximine I (step B), classical oxidation reagents may involve, for example, KMnO₄, NaMnO₄, mCPBA, NaIO₄/RuO₂, NaIO₄/RuCl₃, H₂O₂, oxone. In particular, the use of ruthenium salts in combination with alkali metal periodates and alternatively the use of alkali metal permanganates was described in WO2008/097235 and WO2008/106006.

Detailed preparation conditions useful for the synthesis of such sulfilimine and/or sulfoximine compounds of formula la respectively I are also given, for example, in WO2006/061200 or WO 2007/080131.

Alternatively, the subgroup of sulfoximine compounds of the formula I wherein R₂ is hydrogen and n is 1, and in which R₁, R₃, A, and Q are as defined in formula I, 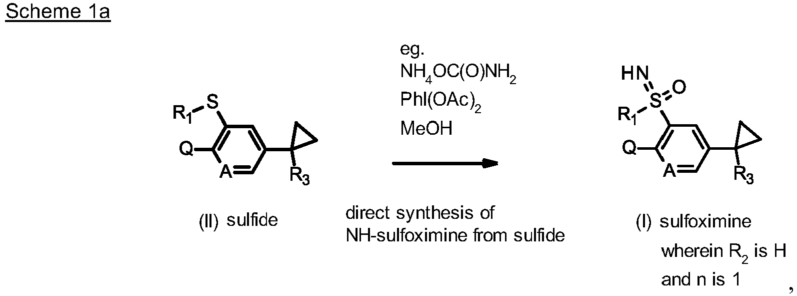 can be prepared (scheme 1a) by reacting sulfide compounds of formula II, wherein R₁, R₃, A, and Q are as defined in formula I, with a suitable nitrogen source such as, for example, ammonia, ammonium carbamate or ammonium acetate (preferably ammonium carbamate), in the presence of hypervalent iodine reagents, such as diacetoxyiodobenzene, in solvents such as toluene, acetonitrile or methanol, at temperatures between 0 and 100°C, preferably around room temperature, in analogy to descriptions found, for example, in Chem. Commun. 53, 348-351; 2017 (and references cited therein).

A compound of the formula Ib, wherein R₁, R₃, A, and Q are as defined above and wherein n is 1 and R₂ is CN, may be transformed into a compound of the formula Ic, wherein R₁, R₃, A, and Q are as defined above are as defined above and wherein n is 1 and R₂ is C(O)CF₃, by treatment with trifluoroacetic anhydride in a solvent such as dichloromethane as described, for example, in O.G. Mancheno, C. Bolm, Org. Lett. 2007, 9, 3809-3811. The compound of the formula Ic, wherein R₁, R₃, A, and Q are as defined above and wherein n is 1 and R₂ is C(O)CF₃, may be transformed into a compound of the formula I, wherein R₁, R₃, A, and Q are as defined above and wherein n is 1 and R₂ is hydrogen, by treatment with a base such as sodium or potassium carbonate in a polar protic solvent such as methanol or ethanol as described, for example, in H. Okamura, C. Bolm, Org. Lett. 2004, 6, 1305-1307. A compound of the formula Id, wherein R₁, R₃, A, and Q are as defined above are as defined above and wherein n is 1 and R₂ is C(O)NH₂, may be prepared from a compound of the formula Ib, wherein R₁, R₃, A and Q are as defined above and wherein n is 1 and in which R₂ is CN, by treatment with a strong acid such as concentrated sulfuric acid in an organic solvent such as acetonitrile as described, for example, in WO09/111309. The chemistry is summarised in scheme 2.

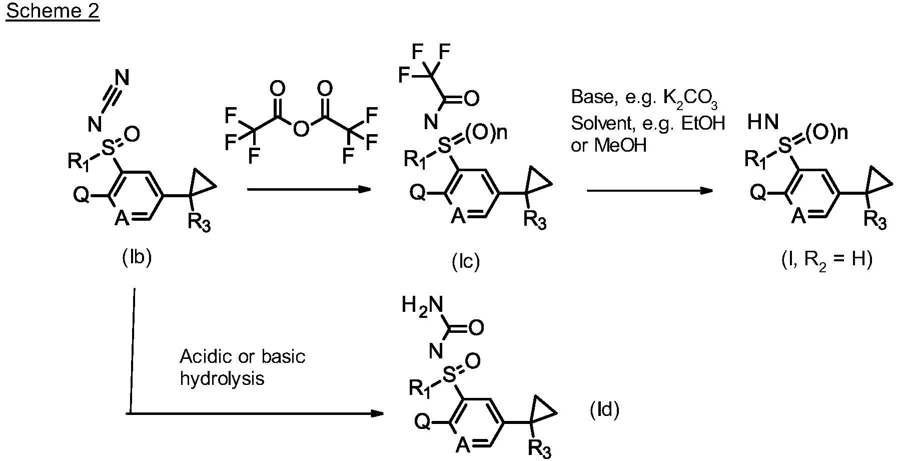

A compound of the formula Ie, wherein R₁, R₃, A and Q are as defined above and wherein n is 1 and R₂ is C₁-C₆alkyl, may be prepared from a compound of the formula I, wherein R₁, R₃, A and Q are as defined above and wherein n is 1 and in which R₂ is hydrogen, by treatment with an alkylating agent of formula LG-R₂, wherein LG is a leaving group such as a halogen (especially bromine or iodine), a sulfonate OSO₂R₁₂ (especially mesylate or tosylate), wherein R₁₂ is C₁-C₆alkyl, C₁-C₆halo-alkyl, or phenyl optionally substituted by nitro or C₁-C₃alkyl, or a sulfate (such as dimethylsulfate), preferably in the presence of a suitable base, such as alkali metal carbonates, for example sodium carbonate or potassium carbonate, or alkali metal hydrides such as sodium hydride, or alkali metal hydroxides such as sodium hydroxide and potassium hydroxide, in an inert solvent at temperatures between -20 and 150°C, preferably between 0 and 80°C. Examples of solvent to be used include ethers such as tetrahydrofuran, ethylene glycol dimethyl ether (1,2-dimethoxyethane), tert-butylmethyl ether, and 1,4-dioxane, aromatic hydrocarbons such as toluene and xylene, nitriles such as acetonitrile or polar aprotic solvents such as N,N-dimethylformamide, N,N-dimethylacetamide, N-methyl-2-pyrrolidone or dimethyl sulfoxide.

A compound of the formula If, wherein R₁, R₃, A and Q are as defined above and wherein n is 1 and R₂ is C(O)R₇ and R₇ is C₁-C₆alkyl or C₁-C₆haloalkyl, may be prepared from a compound of the formula I, wherein R₁, R₃, A and Q are as defined above and wherein n is 1 and R₂ is hydrogen, by treatment with a reagent of formula LG₁-C(O)R₇ or an anhydride reagent of formula R₇C(O)-O-C(O)R₇, wherein R₇ is as defined above and LG₁ is a leaving group such as a halogen (especially chlorine), optionally in presence of an acylating catalyst, such as 4-dimethylaminopyridine (DMAP), preferably in presence of a base, such as triethylamine, diisopropylethylamine or pyridine, in an inert solvent at temperatures between 0 and 50°C. Examples of solvent to be used include ethers such as tetrahydrofuran, ethylene glycol dimethyl ether, tert-butylmethyl ether, and 1,4-dioxane, aromatic hydrocarbons such as toluene and xylene, halogenated hydrocarbons such as dichloromethane and chloroform, nitriles such as acetonitrile or polar aprotic solvents such as N,N-dimethylformamide, N,N-dimethylacetamide, N-methyl-2-pyrrolidone or dimethyl sulfoxide. The reaction may be carried out in the presence of an excess of base, which then may also act as a solvent or diluent.

A compound of the formula Ig, wherein R₁, R₃, A and Q are as defined above and wherein n is 1 and in which R₂ is C(O)OR₈ and R₈ is C₁-C₆alkyl, C₁-C₆haloalkyl may be prepared from a compound of the formula I, wherein R₁, R₃, A and Q are as defined above and wherein n is 1 and R₂ is hydrogen, by treatment with a reagent of formula LG₂-C(O)OR₈, wherein R₈ is as defined above and LG₂ is a leaving group such as a halogen (especially chlorine), optionally in presence of an acylating catalyst, such as 4-dimethylaminopyridine (DMAP), preferably in presence of a base, such as triethylamine, diisopropylethylamine or pyridine, in an inert solvent at temperatures between 0 and 50°C. Examples of solvent to be used include ethers such as tetrahydrofuran, ethylene glycol dimethyl ether, tert-butylmethyl ether, and 1,4-dioxane, aromatic hydrocarbons such as toluene and xylene, halogenated hydrocarbons such as dichloromethane and chloroform, nitriles such as acetonitrile or polar aprotic solvents such as N,N-dimethylformamide, N,N-dimethylacetamide, N-methyl-2-pyrrolidone or dimethyl sulfoxide. The reaction may be carried out in the presence of an excess of base, which then may also act as a solvent or diluent.

A compound of the formula Ih, wherein R₁, R₃, A, and Q are as defined above and wherein n is 1 and which R₂ is CONR₉R₁₀ and R₉, R₁₀ independently of one another are hydrogen or C₁-C₆alkyl, may be prepared from a compound of the formula I, wherein R₁, R₃, A and Q are as defined above and wherein n is 1 and in which R₂ is hydrogen, by treatment with a reagent of formula LG₃-C(O)NR₉R₁₀, wherein R₉ and R₁₀ are as defined above and LG₃ is a leaving group such as a halogen (especially chlorine), optionally in presence of an acylating catalyst, such as 4-dimethylaminopyridine (DMAP), preferably in presence of a base, such as triethylamine, diisopropylethylamine or pyridine, in an inert solvent at temperatures between 0 and 50°C. Examples of solvent to be used include ethers such as tetrahydrofuran, ethylene glycol dimethyl ether, tert-butylmethyl ether, and 1,4-dioxane, aromatic hydrocarbons such as toluene and xylene, halogenated hydrocarbons such as dichloromethane and chloroform, nitriles such as acetonitrile or polar aprotic solvents such as N,N-dimethylformamide, N,N-dimethylacetamide, N-methyl-2-pyrrolidone or dimethyl sulfoxide. The reaction may be carried out in the presence of an excess of base, which then may also act as a solvent or diluent.

A compound of the formula Ii, wherein R₁, R₃, A, and Q are as defined above and wherein n is 1 and in which R₂ is SO₂R₁₁ and R₁₁ is C₁-C₆alkyl, may be prepared from a compound of the formula I, wherein R₁, R₃, A and Q are as defined above and wherein n is 1 and in which R₂ is hydrogen, by treatment with a reagent of formula LG₄- SO₂R₁₁, wherein R₁₁ is as defined above and LG₄ is a leaving group such as a halogen (especially chlorine), optionally in presence of an acylating catalyst, such as 4-dimethylaminopyridine (DMAP), preferably in presence of a base, such as triethylamine, diisopropylethylamine or pyridine, in an inert solvent at temperatures between 0 and 50°C. Examples of solvent to be used include ethers such as tetrahydrofuran, ethylene glycol dimethyl ether, tert-butylmethyl ether, and 1,4-dioxane, aromatic hydrocarbons such as toluene and xylene, halogenated hydrocarbons such as dichloromethane and chloroform, nitriles such as acetonitrile or polar aprotic solvents such as N,N-dimethylformamide, N,N-dimethylacetamide, N-methyl-2-pyrrolidone or dimethyl sulfoxide. The reaction may be carried out in the presence of an excess of base, which then may also act as a solvent or diluent.A compound of the formula Ij, wherein R₁, R₂, R₃, and Q are as defined above and wherein n is 1 and in which R₂ is C(O)R₇ and R₇ is hydrogen, may be prepared from a compound of the formula I, wherein R₁, R₃, A and Q are as defined above and wherein n is 1, and R₂ is hydrogen, by treatment with a trialkyl orthoformate, such as trimethyl orthoformate, optionally in the presence of an acid activator such as para-toluenesulfonic acid, optionally in the presence of an inert organic solvent, and at temperatures between 0 and 180°C. The reaction may be carried out in the presence of an excess of trialkyl orthoformate, which then may also act as a solvent or diluent. Such a process is illustrated, for example, in WO 2006/037945. The transforsmtions described above are illustrated in scheme 3.

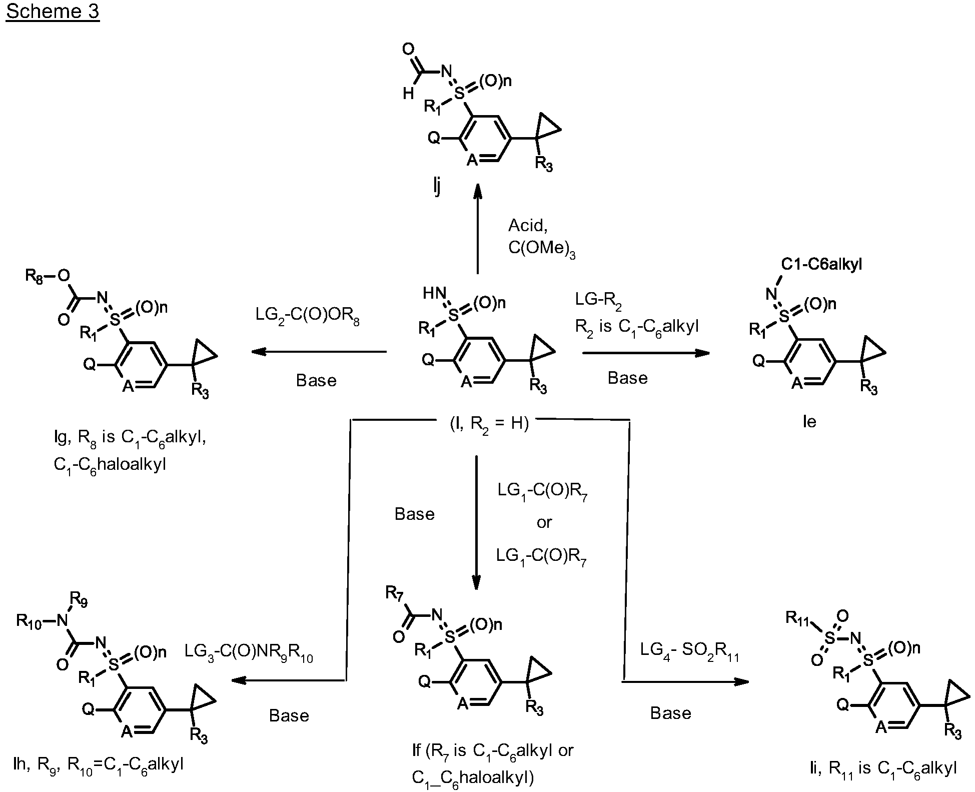

Compounds of formula II wherein R₃ is hydrogen are in some cases known, for example: 2-(5-cyclopropyl-3-ethylsulfanyl-2-pyridyl)-5-(trifluoromethoxy)-1,3-benzoxazole, CAS [2128705-99-5], WO 2017146226; 2-(5-cyclopropyl-3-ethylsulfanyl-2-pyridyl)-5-(difluoromethylsulfanyl)-1,3-benzoxazole, CAS [2128706-06-7], WO 2017146226; 2-(5-cyclopropyl-3-ethylsulfanyl-2-pyridyl)-5-(difluoromethoxy)-1,3-benzoxazole, CAS [2128706-01-2], WO 2017146226; 5-cyclopropyl-2-(5-cyclopropyl-3-ethylsulfanyl-2-pyridyl)-3-methyl-6-(trifluoromethyl)imidazo[4,5-c]pyridin-4-one, CAS [2098699-63-7] , WO 2017089190; 5-cyclopropyl-2-(4-cyclopropyl-2-ethylsulfanyl-phenyl)-3-methyl-6-(trifluoromethyl)imidazo[4,5-c]pyridin-4-one, CAS [2098699-80-8], WO 2017089190 ; 2-(5-cyclopropyl-3-ethylsulfanyl-2-pyridyl)-5-(1,1,2,2,2-pentafluoroethyl)-1,3-benzoxazole, CAS [1975147-97-7], WO 2016121997; 2-(5-cyclopropyl-3-ethylsulfanyl-2-pyridyl)-5-(trifluoromethylsulfanyl)-1,3-benzoxazole, CAS [1975147-94-4], WO 2017146226; 2-(5-cyclopropyl-3-ethylsulfanyl-2-pyridyl)-1-methyl-5-(trifluoromethyl)benzimidazole, CAS [1975147-91-1], WO 2016121997; 2-(5-cyclopropyl-3-ethylsulfanyl-2-pyridyl)-6-(trifluoromethyl)oxazolo[5,4-b]pyridine, CAS [1975147-87-5], WO 2016121997; 2-(5-cyclopropyl-3-ethylsulfanyl-2-pyridyl)-3-methyl-6-(1,1,2,2,2-pentafluoroethyl)imidazo[4,5-b]pyridine, CAS [1975147-85-3], WO 2016121997; 6-(5-cyclopropyl-3-ethylsulfanyl-2-pyridyl)-7-methyl-3-(2,2,2-trifluoroethoxy)imidazo[4,5-c]pyridazine, CAS [1957168-99-8], WO 2016104746; 2-(5-cyclopropyl-3-ethylsulfanyl-2-pyridyl)-3-methyl-6-(trifluoromethyl)imidazo[4,5-b]pyridine, CAS [1951416-89-9 ], WO 2016096584; 2-(4-cyclopropyl-2-ethylsulfanyl-phenyl)-7-(trifluoromethyl)imidazo[1,2-c]pyrimidine,CAS [1923785-41-4], WO 2016071214; 2-(5-cyclopropyl-3-ethylsulfanyl-2-pyridyl)-7-(trifluoromethyl)imidazo[1,2-c]pyrimidine, CAS [1923784-36-4], WO 2016071214; 2-(4-cyclopropyl-2-ethylsulfanyl-phenyl)-5-ethyl-3-methyl-6-(trifluoromethyl)imidazo[4,5-c]pyridin-4-one, CAS [1879051-89-4 ], WO 2016023954; 2-(4-cyclopropyl-2-ethylsulfanyl-phenyl)-3,5-dimethyl-6-(trifluoromethyl)imidazo[4,5-c]pyridin-4-one, CAS [1879051-88-3], WO 2016023954.

Compounds of formula II wherein R₃ is H, i.e compounds of formula IIa 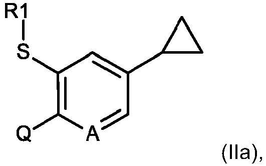 can be generally prepared by reaction of a compound of formula IV, wherein A, Q and R₁ are as defined in formula I above, with a compound of formula V as shown in scheme 4:

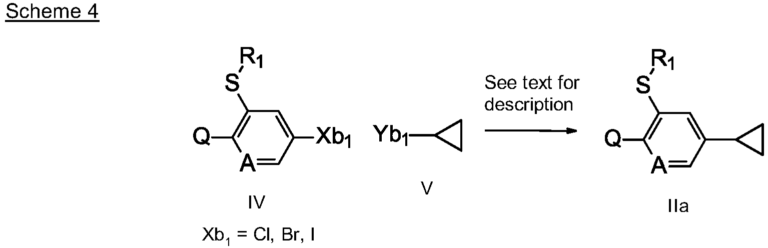

More specifically, compounds of formula IIa can be prepared (as depicted in scheme 4) by reacting compounds of formula IIa with compounds of formula V, wherein X_{b1} can be a halogen, preferentially chlorine, bromine or iodine and Y_{b1} can be a boron-derived functional group, as for example B(OH)₂ or B(OR_{b1})₂ wherein R_{b1} can be a C₁-C₆alkyl group or the two groups OR_{b1} can form together with the boron atom a five- or six-membered ring, as for example a pinacol boronic ester (Suzuki cross-coupling, see for example Tetrahedron Letters, 43(39), 6987-6990; 2002). In formula IIa, A, R₁ and Q are as described in formula **I.** The reaction can be catalyzed by a palladium based catalyst, for example tetrakis(triphenylphosphine) palladium(0), bis(triphenylphosphine)palladium(II) dichloride, chloro(2-dicyclohexylphosphino-2',4',6'-triisopropyl-1,1'-biphenyl)[2-(2'-amino-1,1'-biphenyl)]palladium(II) (XPhos palladacycle), (1,1'bis(diphenylphosphino)-ferrocene)dichloropalladium-dichloromethane (1:1 complex) or palladium acetate plus phosphine ligands (such as, for example, triphenylphosphine or tricyclohexylphosphine) in presence of a base, like sodium carbonate, tripotassium phosphate or cesium fluoride, in a solvent (such as toluene, 1,2-dimethoxy-ethane DME, tetrahydrofuran or dioxane) or a solvent mixture, like, for example a mixture of 1,2-dimethoxyethane (or dioxane, toluene, or tetrahydrofuran) and water, preferably under inert atmosphere. The reaction temperature can preferably range from ambient temperature to the boiling point of the reaction mixture, or alternatively heating may be performed under microwave irradiation.

Alternatively, compounds of formula IV, wherein X_{b1} can be a halogen, preferentially chlorine, bromine or iodine, may be reacted with compounds of formula V, wherein Y_{b1} is a magnesium halide group, such as -MgBr (Kumada cross-coupling), optionally in the presence of additives, such as zind halides (Journal of Organic Chemistry, 75(19), 6677-6680; 2010). The reaction may be catalyzed by a palladium based catalyst, or may involve a nickel based catalyst, such as 1,3-is(diphenylphosphino)propanenickel dichloride (dppp)NiCl₂.

Also known are reactions between compounds of formula IV with compounds of formula V, wherein Y_{b1} is a zinc halide group, such as -ZnBr (Negishi cross-coupling), as illustrated for example in Synthetic Communications, 28(2), 225-232; 1998**.** The reaction may be catalyzed by a palladium based catalyst, such as for example, (1,1'bis(diphenylphosphino)-ferrocene)dichloropalladium Pd(dppf)Cl₂ or bis(triphenylphosphine)palladium(II) dichloride, optionally in the presence of phosphine additives (such as, for example, 2-dicyclohexyl-phosphino-2',6'-dimethoxy-biphenyl S-PHOS), in a solvent, like, for example 1,2-dimethoxyethane, dioxane, toluene, or tetrahydrofuran, preferably under inert atmosphere. The reaction temperature can preferentially range from ambient temperature to the boiling point of the reaction mixture.

Compounds of formula II, wherein R₃ is cyano are in some cases known, for example: 1-[5-ethylsulfanyl-6-[5-methoxy-3-methyl-4-oxo-6-(trifluoromethyl)imidazo[4,5-c]pyridin-2-yl]-3-pyridyl]cyclopropanecarbonitrile, CAS [2225113-68-6], WO 2018/077565; 1-[3-ethylsulfanyl-4-[5-methoxy-3-methyl-4-oxo-6-(trifluoromethyl)imidazo[4,5-c]pyridin-2-yl]phenyl]cyclopropanecarbonitrile, CAS [CAS [2225113-73-3], WO 2018/077565; 1-[4-[5-cyclopropyl-3-methyl-4-oxo-6-(trifluoromethyl)imidazo[4,5-c]pyridin-2-yl]-3-ethylsulfanyl-phenyl]cyclopropanecarbonitrile, CAS [2098699-74-0], WO 2017/089190; 1-[6-[5-cyclopropyl-3-methyl-4-oxo-6-(trifluoromethyl)imidazo[4,5-c]pyridin-2-yl]-5-ethylsulfanyl-3-pyridyl]cyclopropanecarbonitrile, CAS [2098699-59-1], WO 2017/089190; 1-[5-ethylsulfanyl-6-[5-(trifluoromethylsulfanyl)-1,3-benzoxazol-2-yl]-3-pyridyl]cyclopropanecarbonitrile, CAS [1975148-53-8 ], WO 2016/121997; 1-[3-ethylsulfanyl-4-[7-(trifluoromethyl)imidazo[1,2-c]pyrimidin-2-yl]phenyl]cyclopropanecarbonitrile, CAS [1923785-47-0], WO 2016/071214; 1-[5-ethylsulfanyl-6-[7-(trifluoromethyl)imidazo[1,2-c]pyrimidin-2-yl]-3-pyridyl]cyclopropanecarbonitrile, CAS [1923784-42-2], WO 2016/071214.

Other compounds of formula II, wherein R₃ is cyano and R₁, A, and Q are as defined in formula I, i.e compounds of formula IIb 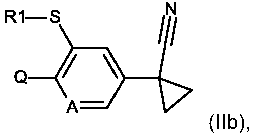 can be prepared as shown in scheme 5:

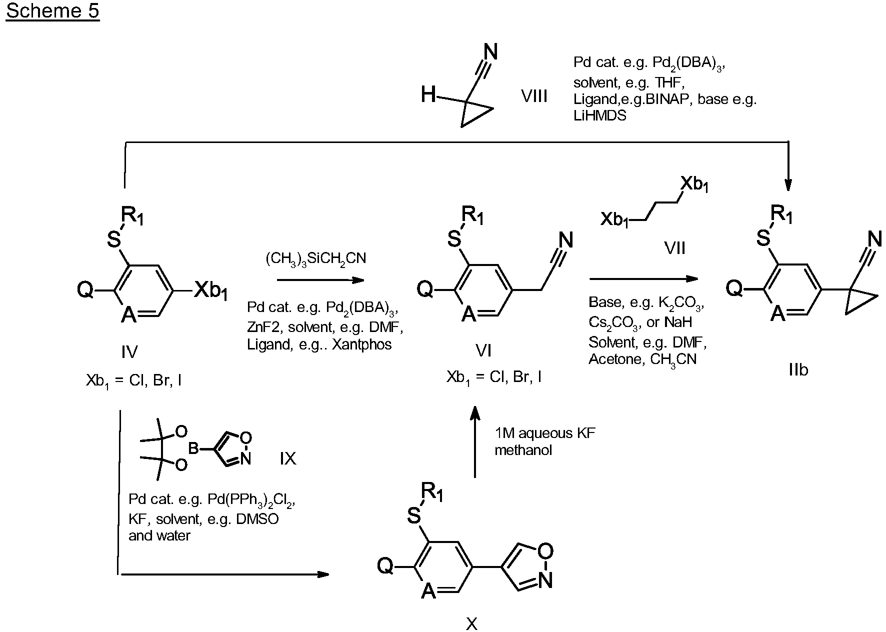

As shown in scheme 5, treatment of compounds of formula IV, wherein R₁, Q, and A are as described in formula I, and wherein Xb₁ is preferably halogen (even more preferably chlorine, bromine or iodine), with trimethylsilyl-acetonitrile TMSCN, in the presence of zinc(II)fluoride, and a palladium(0)catalyst such as tris(dibenzylideneacetone)dipalladium(0)-chloroform adduct (Pd₂(dba)₃), a ligand, for example Xantphos, in an inert solvent, such as DMF at temperatures between 100-160 °C, optionally under microwave heating, leads to compounds of formula VI. Such chemistry has been described in the literature, e.g. in Org. Lett., 16(24), 6314-6317; 2014***.*** Compounds of formula IV can be treated with compounds of formula VII, wherein Xb₁ is as described above, in the presence of a base such as sodium hydride, K₂CO₃, or Cs₂CO₃, in an inert solvent such as DMF, acetone, or acetonitrile, to give compounds of formula IIb. Alternatively, compounds of formula IIb can be prepared directly from compounds of formula IV by treatment with compounds of formula VIII with Pd₂(dba)₃, a ligand, such as BINAP a strong base such as LiHMDS, in an inert solvent such as THF at temperatures between 40-70 °C. Such chemistry has been described in, for example, J. Am. Chem. Soc., 127(45), 15824-15832; 2005***.*** Another process to compounds of formula IIb involves reaction of compounds of formula IV, wherein A, R₁ and Q are as described under formula I, and in which Xb₁ is halogen, preferably chlorine, bromine, iodine, with 4-isoxazoleboronic acid or 4-isoxazoleboronic acid pinacol ester (compound of formula IX), in the presence of potassium fluoride KF, and a palladium catalyst such as bis(triphenylphosphine)palladium(II) dichloride Pd(PPh₃)₂Cl₂, in an inert solvent, such as dimethylsulfoxide DMSO, optionally in mixture with water, at temperatures between 40-150°C, optionally under microwave heating, leads to compounds of formula IX, wherein Q, R₁ and A are as described under formula I above. Reaction of compounds of formula IX, with aqueous potassium fluoride KF (concentration between 0.5 and 3M, preferably 1M), in an inert solvent, such as dimethylsulfoxide DMSO or methanol, at temperatures between 20-150°C, optionally under microwave heating, leads to compounds of formula VI, wherein A, R₁ and Q are as described under formula I above. Conversion of compounds of formula V to compounds of formula IIb is as described above. Such chemistry has been described in the literature, e.g. in J Am Chem Soc 2011, 133, 6948-6951.

Compounds of the formula IIb may further be utilized for the preparation of compounds of formula II (scheme 6) where R₃ is further functionalized. Indeed, compounds of formula IIb, wherein A, R₁, X₁ and R₂ are as defined in formula I above, may be partially hydrolyzed, under acidic or basic conditions known to persons skilled in the art to compounds of formula IIc, or fully hydrolysed to compounds of formula IId. Treatment of compounds of formula IIc with reagents such as SF₄ or Fluolead (4-tert-butyl-2, 6-dimethyl phenylsulfur trifluoride), optionally in the presence of HF, leads to compounds of formula IIe (as described in for example, Organic Letters, 16, 6314-6317; 2014***.***

Activation of compounds of formula (Ild), wherein R₁, Q and A are as defined in formula I, by methods known to those skilled in the art and described in, for example, Tetrahedron, 2005, 61 (46), 10827-10852, to form an activated species (IIf), wherein Q, R₁ and A are as defined in formula I, and wherein X₀ is halogen, preferably chlorine. For example, compounds (IIf) where X₀ is halogen, preferably chlorine, are formed by treatment of (IId) with, for example, oxalyl chloride (COCl)₂ or thionyl chloride SOCl₂ in the presence of catalytic quantities of N,N-dimethylformamide DMF in inert solvents such as methylene chloride CH₂Cl₂ or tetrahydrofuran THF at temperatures between 20 to 100°C, preferably 25°C. Alternatively, treatment of compounds of formula (IId) with, for example, 1-ethyl-3-(3-dimethylaminopropyl)carbodiimide EDC or dicyclohexyl carbodiimide DCC will generate an activated species (XII), wherein X₀ is X₀₁ or X₀₂ respectively, in an inert solvent, such as pyridine or tetrahydrofuran THF, optionally in the presence of a base, such as triethylamine, at temperatures between 50-180°C; Such activated intermediates of formula lif can be reacted with nucleophiles such as amines of formula HNR₁₀₁R₀₁₂, wherein R₁₀₁ and R₁₀₂ are hydrogen or C₁-C₆alkyl, or HOR₁₀₃ where R103 is C₁-C₆alkyl, optionaly in the presence of a base such as triethylamine, pyridine or DMAP, in an inert solvent such as CH₂Cl₂, THF or acetonitrile, to give compounds of formula lig and lih respectively. Such reactions are well known to thoses skilled in the art. The post transformation of the cyanocyclopropyls are illustrated in Scheme 6.

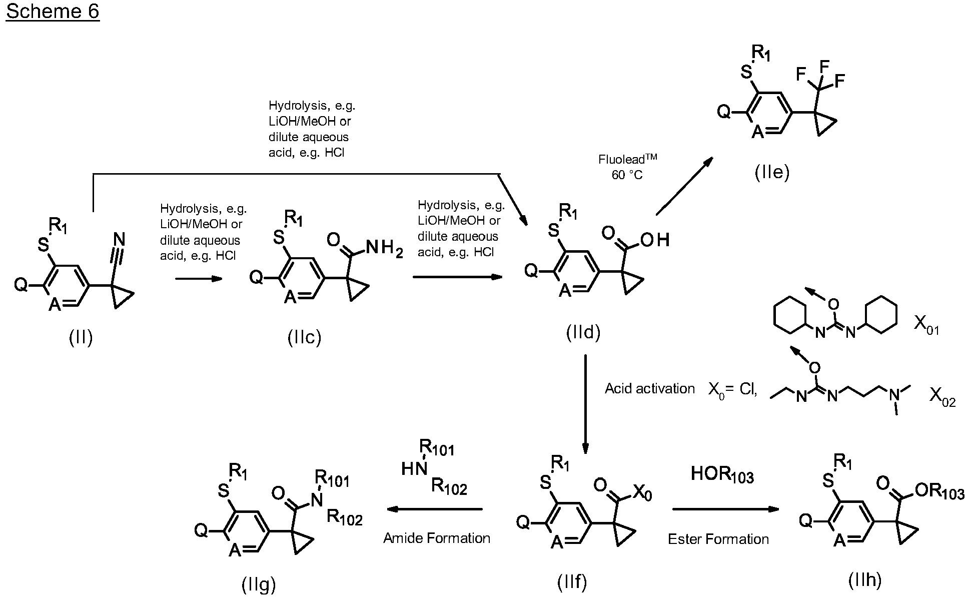

Compounds of the formula II, wherein Q is Q₁, defining compounds of the formula II-Q₁ 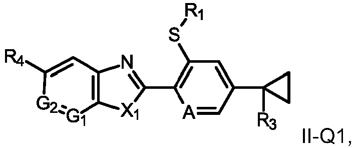 wherein R₁, R₃, R₄, X₁, G₁, and G₂ are as defined in formula I can be prepared as shown in scheme 7.

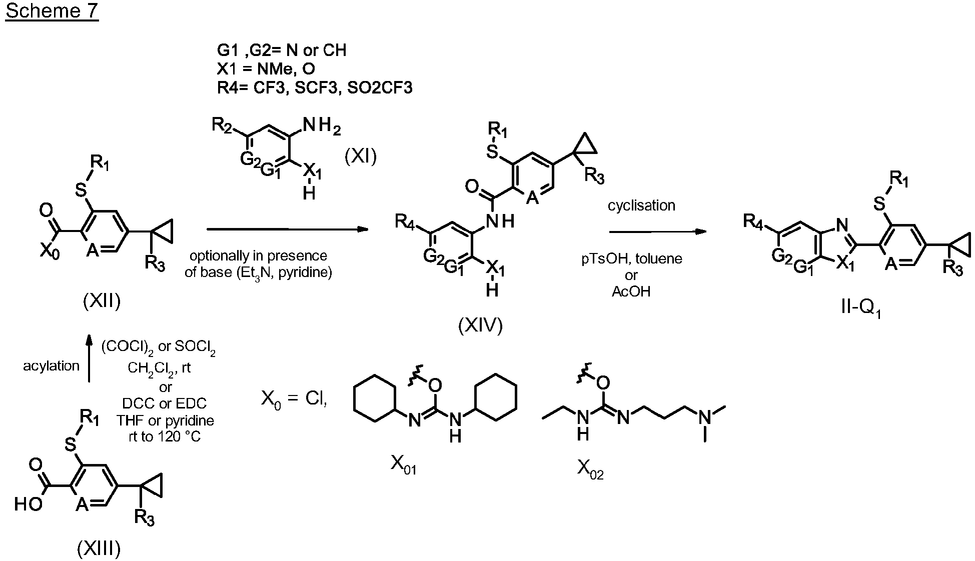

As shown in scheme 7, compounds of formula II-Q1 can be prepared by cyclizing compounds of the formula (XIV), wherein R₁, R₃, R₄, A, X₁, G₁, and G₂ are as defined in formula I, for example through heating in acetic acid or trifluoroacetic acid (preferably when X₁ is NR₅, wherein R₅ is C₁-C₄alkyl), at temperatures between 0 and 180°C, preferably between 20 and 150°C, optionally under microwave irradiation. Cyclization of compounds of formula (XIV) may also be achieved in the presence of an acid catalyst, for example methanesulfonic acid, or para-toluenesulfonic acid p-TsOH, in an inert solvent such as N-methyl pyrrolidone, toluene or xylene, at temperatures between 25-180°C, preferably 100-170°C. Such processes have been described previously, for example, in WO 2016096584. Alternatively, compounds of formula (XIV) may be converted into compounds of formula II-Q1 (preferably when X₁ is O) using triphenylphosphine, di-isopropyl azodicarboxylate (or di-ethyl azodicarboxylate) in an inert solvent such as tetrahydrofuran THF at temperatures between 20-50°C. Such Mitsunobu conditions have been previously described for these transformations (see WO 2009/131237 and WO 2016/121997).

Compounds of the formula (XIV), wherein R₁, R₃, R₄, A, X₁, G₁, and G₂ are as defined in formula I, may be prepared via acylation by;
i) Activation of compounds of formula (XIII), wherein R₁, R₃ and A are as defined in formula I, by methods known to those skilled in the art and described in, for example, Tetrahedron, 2005, 61 (46), 10827-10852, to form an activated species (XII), wherein R₁, R₃ and A are as defined in formula I, and wherein X₀ is halogen, preferably chlorine. For example, compounds (XII) where X₀ is halogen, preferably chlorine, are formed by treatment of (XIII) with, for example, oxalyl chloride (COCl)₂ or thionyl chloride SOCl₂ in the presence of catalytic quantities of N,N-dimethylformamide DMF in inert solvents such as methylene chloride CH₂Cl₂ or tetrahydrofuran THF at temperatures between 20 to 100°C, preferably 25°C. Alternatively, treatment of compounds of formula (XIII) with, for example, 1-ethyl-3-(3-dimethylaminopropyl)carbodiimide EDC or dicyclohexyl carbodiimide DCC will generate an activated species (XII), wherein X₀ is X₀₁ or X₀₂ respectively, in an inert solvent, such as pyridine or tetrahydrofuran THF, optionally in the presence of a base, such as triethylamine, at temperatures between 50-180°C; followed by
ii) Treament of the activated species (XII) with compounds of the formula (XI), wherein X₁, G₁, G₂ and R₄ are as defined in formula I, in the presence of a base, such as triethylamine, N,N-diisopropyl-ethylamine or pyridine, in an inert solvents such as dichloromethane, tetrahydrofuran, dioxane, N,N-dimethylformamide, N,N-dimethylacetamide, acetonitrile, ethyl acetate or toluene, at temperatures between 0 and 50°C, to form the compounds of formula (XIV). Compounds of formula XIII where R₃ is H , i.e compound wherein is R₁ is ethyl, R₃ is H, and A is N or CH are known and described in WO 2017146226, or WO 2016121997(for 5-cyclopropyl-3-ethylsulfanyl-pyridine-2-carboxylic acid, CAS [1975148-58-3], and WO 2016023954 (for 4-cyclopropyl-2-ethylsulfanyl-benzoic acid, CAS [1879052-58-0]. The compound of formula XIII wherein R₃ is cyano, R₁ is ethyl and A is CH (4-(1-cyanocyclopropyl)-2-ethylsulfanyl-benzoic acid, CAS [2225113-79-9]), and the compound of formula XIII wherein R₃ is cyano, R₁ is ethyl and A is N (5-(1-cyanocyclopropyl)-3-ethylsulfanyl-pyridine-2-carboxylic acid, CAS [2225113-77-7]) are known and described in and described in WO 2018/077565.

Compounds of formula (XI), wherein X₁, G₁, G₂ and R₂ are as defined in formula I, have been previously described, for example, in WO 2012/086848, WO 2015/000715, and WO 2016/116338.

Compounds of the formula II, wherein Q is Q₂, defining compounds of the formula II-Q₂,
wherein R₁, R₃, R₄, A, and G₂ are as defined in formula I, 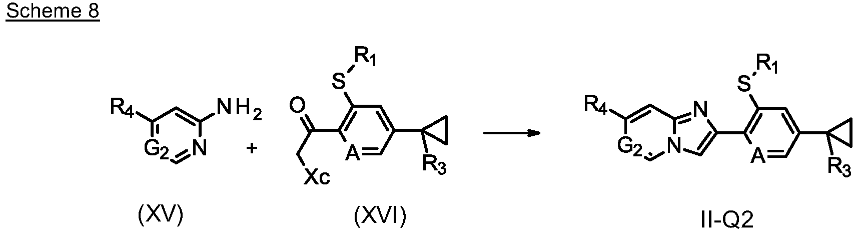 may be prepared (scheme 8) by condensing compounds of the formula (XVI), wherein R₁, R₃, A are as defined in formula I, and in which Xc is is a leaving group such as, for example, chlorine, bromine or iodine (preferably chlorine or bromine), with compounds of the formula (XVI), wherein G₂ and R₂ are as defined in formula I, in an inert solvent, for example ethanol or acetonitrile, optionally in the presence of a suitable base, such as sodium, potassium or cesium carbonate, or magenesium oxide at temperatures between 80 and 150°C, optionally under microwave heating conditions. Such processes have been described previously, for example, in WO 2012/49280 or WO 2003/031587. Compounds of formula (XV), wherein G₂ and R₂ are as defined in formula I, are either known compounds, commercially available or may be prepared by known methods known to those skilled in the art.

Compounds of the formula (XVI), wherein R₁, R₃, and A are as defined in formula I, and in which Xc is is a leaving group such as, for example, chlorine, bromine or iodine (preferably chlorine or bromine), 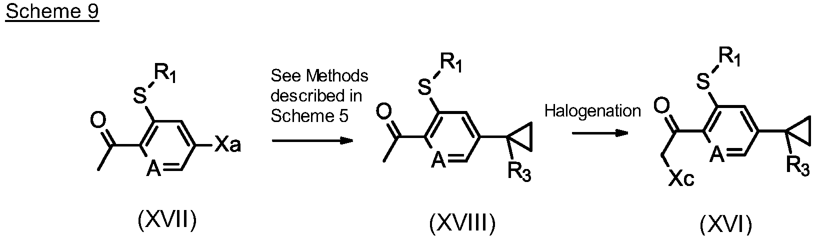 may be prepared (scheme 9) by treatment of compounds of formula (XVIII), wherein R₁, R₃, and A are as defined in formula I, with a halogenating agent ("Xc⁺" source), e.g. N-bromosuccinimide, N-iodosuccinimide, N-chlorosuccinimide, I₂, CuBr₂, Br₂ in acetic acid, PhNMe₃⁺Br₃⁻, typically in a solvent such as methanol, acetonitrile, tetrahydrofurane, ethyl acetate, chloroform or dichloromethane, or mixtures thereof, at temperatures between 0°C and 150°C, preferably between room temperature and 120°C, optionally under microwave heating conditions. Such processes have been described previously, for example, in WO2016/071214.

Compounds of formula (XVIII), wherein R₁, R₃ and A are as defined in formula I, may be prepared by reacting compounds of formula (XVII), wherein R₁ and A are as defined in formula I, and in which Xa is a leaving group such as, for example, chlorine, bromine or iodine (preferably chlorine or bromine), under conditions already described above (see scheme 5, transformation of compounds IV into IIb).

Compounds of formula (XVII), wherein R₁ and A are as defined in formula I, and wherein Xa is a leaving group such as, for example, chlorine, bromine or iodine, in particular those compounds wherein Xa is a halogen (even more preferably chlorine, bromine or iodine; particularly preferred is chlorine or bromine), are either known compounds, commercially available or may be prepared by known methods, described in the literature, as for example in WO 2016/071214.

Alternatively, compounds of the formula II, wherein Q is Q₅, defining compounds of the formula II-Q₅, wherein R₁, R₃, R₄, R₆ and A are as defined in formula I, 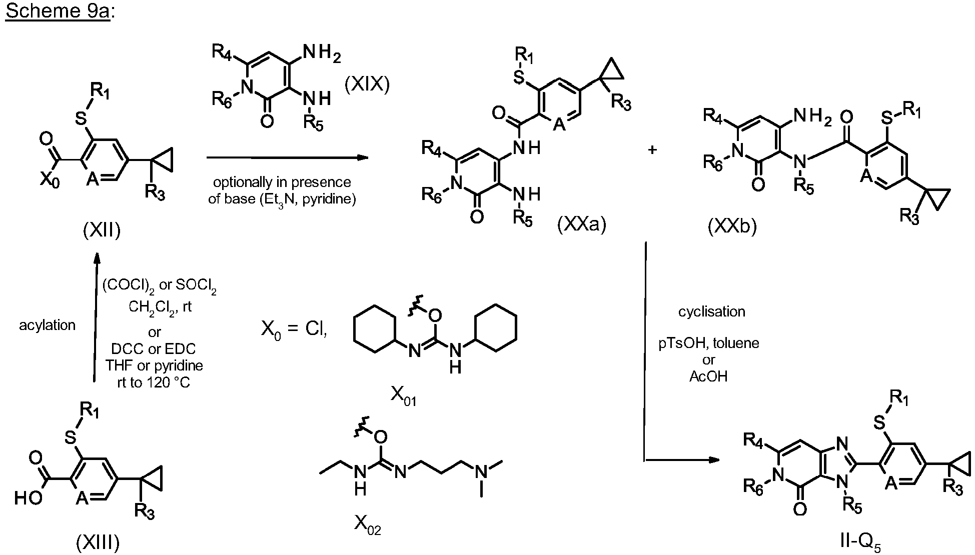 may be prepared (scheme 9a) by cyclizing compounds of the formula (XXa), wherein R₁, R₃, R₄, R₅, R₆, and A are as defined in formula I, or regioisomers of the formula (XXb) with identical substituent definitions, or a mixture thereof in any ratio, under conditions already described above (see scheme 7, transformation of compounds (XIV) into II-Q1).

Compounds of the formula (XXa), wherein R₁, R₃, R₄, R₅, R₆, and A are as defined in formula I, or regioisomers of the formula (XXb) with identical substituent definitions, or a mixture thereof in any ratio, may be prepared by treatment of the activated species (XII) described above with compounds of the formula (XIX), wherein R₄, R₅ and R₆ are as defined in formula I, under conditions already described above (see scheme 7, transformation of compounds (XII) and (XIII) into compounds (XIV)).

Compounds of formula (XIX), wherein R₄, R₅ and R₆ are as defined in formula I, have been previously described, for example, in WO 2016/023954, WO 2016/142326, WO 2017/133994, and WO 2018077565

Alternatively, compounds of the formula II, wherein Q is Q₃, defining compounds of the formula II-Q₃, wherein R₁, R₄, R₃, and A are as defined in formula I, 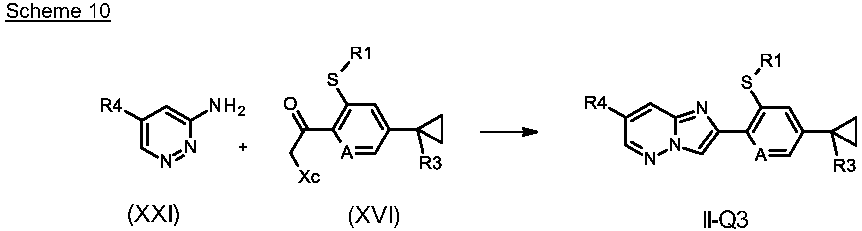 may be prepared (scheme 10) by condensing compounds of the formula (XVI) described above, wherein R₁, R₃ and A are as defined in formula I, and in which Xc is is a leaving group such as, for example, chlorine, bromine or iodine (preferably chlorine or bromine), with compounds of the formula (XXI), wherein R₄ is as defined in formula I, in an inert solvent, for example ethanol, toluene or acetonitrile, optionally in the presence of a suitable base, such as sodium, potassium or cesium carbonate (or sodium or potassium hydrogene carbonate), or magnesium oxide at temperatures between 80 and 150°C, optionally under microwave heating conditions. Such processes have been described previously, for example, in WO 2011/074658. Compounds of formula (XXI), wherein R₄ is as defined in formula I, are either known compounds, commercially available or may be prepared by known methods known to those skilled in the art (see for example WO 2011/074658 and WO 2010/083145).

Compounds of the formula II, wherein Q is Q₄, defining compounds of the formula II-Q₄, 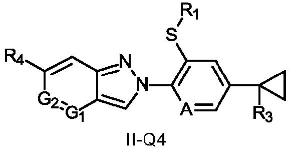 wherein R₁, R₃, R₄, G₁, G₂ and A are as defined in formula I, 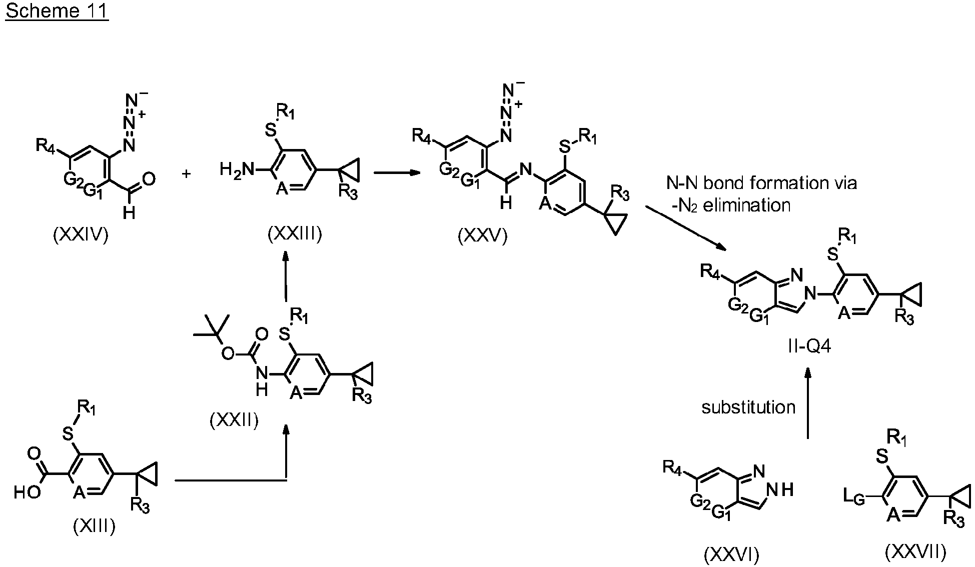 may also be prepared (scheme 11) *via* N-N bond forming reaction of azido imines of compounds of the formula (XXV), wherein R₁, R₄, R₇, R₈, A, G₁, G₂ and R₂ are as defined in formula I, under pyrolytic condition which facilitates extrusion of N₂ . Alternatively, this reaction may be conducted in presence of a metal catalyst, for example a Cu(I) catalyst, such as Cul, CuBr, CuCl or CuCN , or more generally with transition metals, in combination with a ligand such as tetramethylethylenediamine, 2,2'-bipyridine or 1,10-phenanthroline. Suitable solvents may include use of e toluene, chlorobenzene, or xylene, at temperatures between room temperature and 200°C, preferably between 100 and 160°C, optionally under microwave heating conditions. Such reductive cyclisation reaction conditions were described in, for example, Organic Letters, 2011, Vol. 13, No. 13, 3542-3545, and US 2017/0260183,

Compounds of the formula (XXV), wherein R₁, R₃, R₄, G₁, G₂ and A are as defined in formula I, may be prepared by reaction between compounds of formula (XXIII), wherein R₁, R₃ and A are as defined in formula I, and compounds of formula (XXIV), wherein G₁, G₂ and R₄ are as defined in formula I, usually upon heating at temperatures between room temperature and 200°C, preferably between 40 and 160°C, optionally under microwave heating conditions, in suitable solvents that may include, for example, toluene or xylene. The formation of compounds of formula (XXV) may require water removal, either by azeotropical distillation, or by means of a drying agent such as for example TiCl₄ or molecular sieves. Such formation of Schiff bases of formula (XXV) is known to those skilled in the art, and was described in, for example, WO 2017/134066. Compounds of formula XXIV are reported in literature (see CAS 2211908-96-0 reported in WO 2018/052136).

Compounds of the formula XXIII may be prepared by the reaction of compound of formula XXII in the presence of acid such as HCl, H₂SO₄, H₃PO₄, HNO₃, TFA. Such deprotection reaction of N-linked carbamate are well known to those skilled in the art and described for example in RSC Advances, 5(5), 3200-3205; 2015

Compounds of formula XXII may be prepared by the reaction of compounds of formula XIII and organo-azide or an ammonia derivatives for example NH₄OH, NH₃, NH₂Boc in the presence of a suitable base and in the presence or absence of Lewis acids and solvent at temperatures between 50 °C and 200 °C. Examples of organo-azide include TMSN₃, sodium azide, diphenyl phosphoryl azide or tosyl azide and suitable solvent may be t-BuOH, toluene, xylene, THF or acetonitrile. Example of suitable Lewis acid may include Zn(OTf)₂. Such reactions of converting carboxylic acids to amines are well known to those skilled in the art by the name of Curtius reaction and reported in Org. Lett., 2005, 7, 4107-4110; Journal of Medicinal Chemistry, 49(12), 3614-3627; 2006**.**

Alternatively, compounds of the formula II-Q₄, wherein R₁, R₃, R₄, G₁, G₂ and A are as defined in formula I, may be prepared by reacting compounds of formula (XXVI), wherein R₄, G₁, G₂ are as defined in formula I, and in which L_{G} is a leaving group such as, for example, chlorine, bromine or iodine (preferably chlorine or bromine), or an aryl-, alkyl- or haloalkylsulfonate such as trifluoromethanesulfonate, with compounds of formula (XXVII), wherein G₁, G₂ and R₄ are as defined in formula I, in the presence of base such as for example cesium, sodium, potassium or lithium carbonate, or sodium hydride, optionally in the presence of a metal catalyst such as copper(I) iodide or a palladium catalyst, with or without additives such as L-proline, N,N'-dimethylethylenediamine or a phosphorus-based ligand, in an inert solvent such as acetonitrile, N,N-dimethylformamide, N-methyl-2-pyrrolidone or dimethyl sulfoxide at temperatures between room temperature and 200°C, optionally under microwave heating conditions. Such aromatic nucleophilic substitution reaction conditions were described in, for example, WO 2017/134066. Compounds of formula XXVII may be obtained by a Sandmeyer reaction, i.e. diazotation of compound XXIII with NaNO2 or tBu-ONO in an inert solvent such as t-BuOH or water, followed by addition of an inoraganic halaide such as CuBr2, CuCl, or KI. Such Sandmeyer reactions are well known in the literature (see for example Synthesis, 2007, 2534-2538, Org. Lett., 2008, 10, 3961-3964, and references cited therein.

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reaction is advantageously carried out in a temperature range from approximately -80°C to approximately +140°C, preferably from approximately -30°C to approximately +100°C, in many cases in the range between ambient temperature and approximately +80°C.

A compound of formula I can be converted in a manner known per se into another compound of formula I by replacing one or more substituents of the starting compound of formula I in the customary manner by (an)other substituent(s) according to the invention.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula I can be prepared in a manner known per se. Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known per se into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula I according to the following Tables Y-1 to Y-8, X-1 to X-8, U-1 to U-2 and V-1 to V-6 below can be prepared according to the methods described above. The examples which follow, wherein R₁, R₂, R₃, R₄, Q (Q₂, Q₃, Q₄, Q₅), R₅, R₆, R₇, R₈, R₉, R₁₀, R₁₁, A, G₁ and G₂ are defined as for compounds of formula (I), according to the present invention, are intended to illustrate the invention and show preferred compounds of formula I.

The compounds of Tables Y-1 to Y-8, X-1 to X-8, U-1 to U-2 and V-1 to V-6 which relate to compounds of formula I, wherein Q is Q₁ 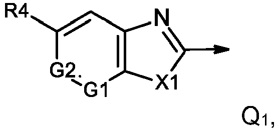 are considered as reference examples.

Tables Y-1 to Y-8 refer to compounds of formula

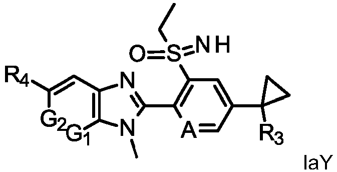

**Table Z: Substituent definitions of R₃ and R₄:**

| Index | R₄ | R3 |
|---|---|---|
| 1 | CF₃ | CN |
| 2 | SCF₃ | CN |
| 3 | SO₂CF₃ | CN |
| 4 | CF₃ | H |
| 5 | SCF₃ | H |
| 6 | SO₂CF₃ | H |
| 7 | CF₃ | CONH₂ |
| 8 | SCF₃ | CONH₂ |

Table Y-1 provides 8 compounds Y-1.001 to Y-1.008 of formula IaY wherein A is CH, G₁ is CH, G₂ is CH and R₃, R₄ are as defined in table Z.

For example, compound Y-1.001 has the following structure;

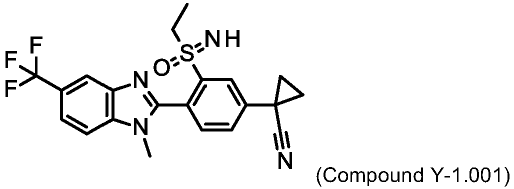

Table Y-2 provides 8 compounds Y-2.001 to Y-2.008 of formula IaY wherein A is CH, G₁ is CH, G₂ is N and R₃, R₄ are as defined in table Z.

For example, compound Y-2.001 has the following structure;

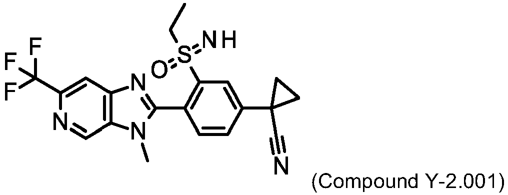

Table Y-3 provides 8 compounds Y-3.001 to Y-3.008 of formula IaY wherein A is CH, G₁ is N, G₂ is CH and R₃, R₄ are as defined in table Z.

For example, compound Y-3.001 has the following structure;

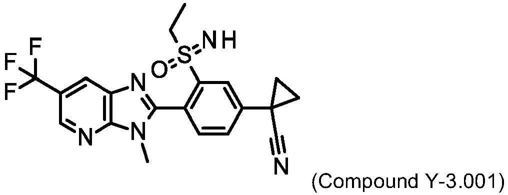

Table Y-4 provides 8 compounds Y-4.001 to Y-4.008 of formula IaY wherein A is CH, G₁ is N, G₂ is N and R₃, R₄ are as defined in table Z.

For example, compound Y-4.001 has the following structure;

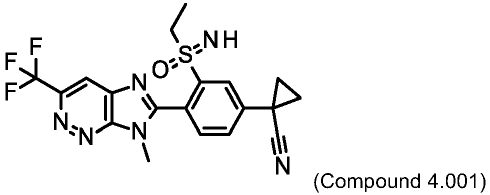

Table Y-5 provides 8 compounds Y-5.001 to Y-5.008 of formula IaY wherein A is N, G₁ is CH, G₂ is CH and R₃, R₄ are as defined in table Z.

For example, compound Y-5.001 has the following structure;

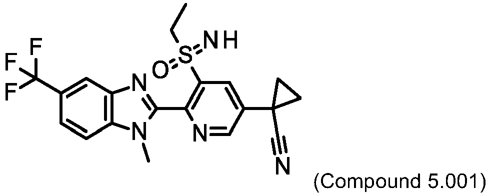

Table Y-6 provides 8 compounds Y-6.001 to Y-6.008 of formula IaY wherein A is N, G₁ is CH, G₂ is N and R₃, R₄ are as defined in table Z.

For example, compound Y-6.001 has the following structure;

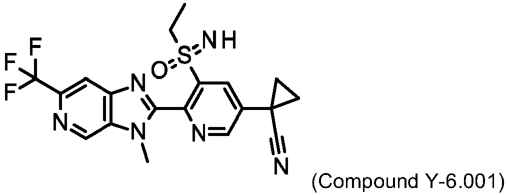

Table Y-7 provides 8 compounds Y-7.001 to Y-7.008 of formula IaY wherein A is N, G₁ is N, G₂ is CH and R₃, R₄ are as defined in table Z.

For example, compound Y-7.001 has the following structure;

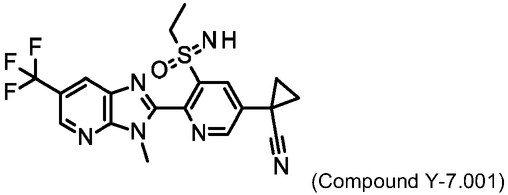

Table Y-8 provides 8 compounds Y-8.001 to Y-8.008 of formula IaY wherein A is N, G₁ is N, G₂ is N and R₃, R₄ are as defined in table Z.

For example, compound Y-8.001 has the following structure;

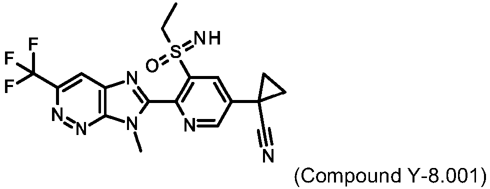

Tables X-1 to X-8 refer to compounds of formula

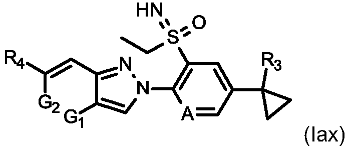

Table X-1 provides 8 compounds X-1.001 to X-1.008 of formula lax wherein A is CH, G₁ is CH, G₂ is CH and R₃, R₄ are as defined in table Z.

For example, compound X-1.001 has the following structure;

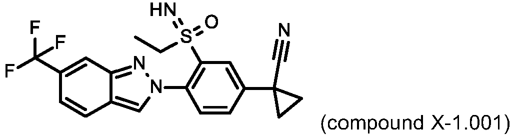

Table X-2 provides 8 compounds X-2.001 to X-2.008 of formula lax wherein A is CH, G₁ is CH, G₂ is N and R₃, R₄ are as defined in table Z.

Table X-3 provides 8 compounds X-3.001 to X-3.008 of formula lax wherein A is CH, G₁ is N, G₂ is CH and R₃, R₄ are as defined in table Z.

Table X-4 provides 8 compounds X-4.001 to X-4.008 of formula lax wherein A is CH, G₁ is N, G₂ is N and R₃, R₄ are as defined in table Z.

Table X-5 provides 8 compounds X-5.001 to X-5.008 of formula lax wherein A is N, G₁ is CH, G₂ is CH and R₃, R₄ are as defined in table Z.

Table X-6 provides 8 compounds X-6.001 to X-6.008 of formula Ib wherein A is N, G₁ is CH, G₂ is N and R₃, R₄ are as defined in table Z.

Table X-7 provides 8 compounds X-7.001 to X-7.008 of formula Ib wherein A is N, G₁ is N, G₂ is CH and R₃, R₄ are as defined in table Z.

Table X-8 provides 8 compounds X-8.001 to X-8.008 of formula Ib wherein A is N, G₁ is N, G₂ is N and R₃, R₄ are as defined in table Z.

The tables V-1 to V-6 below illustrate specific compounds of the invention of formula I (wherein R₂ is H, and n is 1).

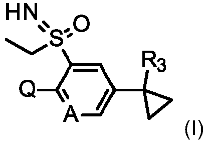

Table V-1 provides 9 compounds V-1.001 to V-1.009 of formula I wherein is A is CH, R₃ is CN and Q are as defined in table W.

**Table W: Substituent definitions of Q:**

| Index | Q | Index | Q | Index | Q |
|---|---|---|---|---|---|
| 1 | 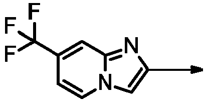 | 4 | 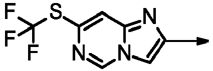 | 7 | 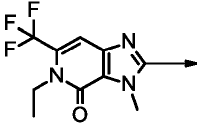 |
| 2 | 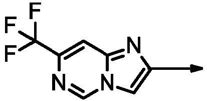 | 5 | 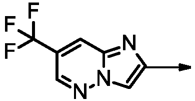 | 8 | 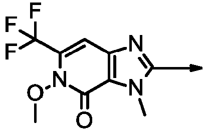 |
| 3 | 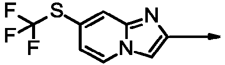 | 6 | 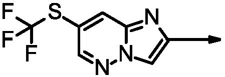 | 9 | 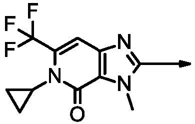 |

For example compound V-1.002 has the following structure:

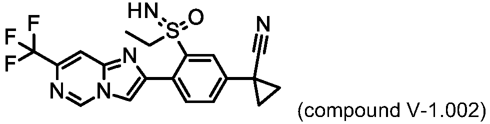

Table V-2 provides 9 compounds V-2.001 to V-2.009 of formula I wherein A is N, R₃ is CN and Q are as defined in table W.

For example compound V-2.005 has the following structure:

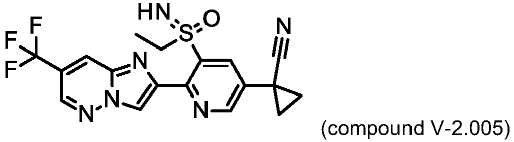

Table V-3 provides 9 compounds V-3.001 to V-3.009 of formula I wherein A is CH, R₃ is H and Q are as defined in table W.

For example compound V-3.008 has the following structure:

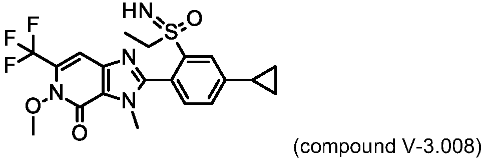

Table V-4 provides 9 compounds V-4.001 to V-4.009 of formula I wherein A is N, R₃ is H and Q are as defined in table W.

For example compound V-4.001 has the following structure:

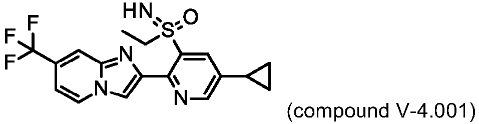

Table V-5 provides 9 compounds V-5.001 to V-5.009 of formula I wherein A is CH, R₃ is CONH₂ and Q are as defined in table W.

For example compound V-5.007 has the following structure:

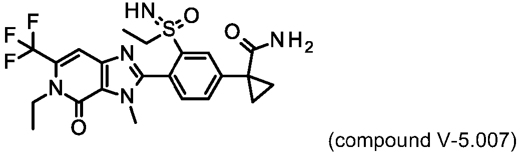

Table V-6 provides 9 compounds V-6.001 to V-6.009 of formula I wherein A is N, R₃ is CONH₂ and Q are as defined in table W

For example compound V-6.006 has the following structure:

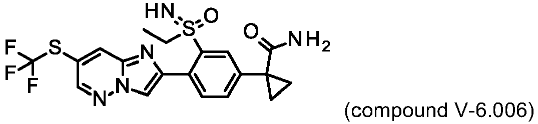

Tables U-1 to U-2 refer to compounds of formula

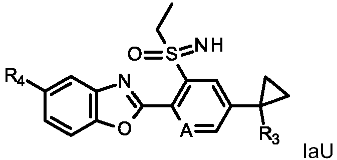

Table U-1 provides 8 compounds U-1.001 to U-1.008 of formula IaU wherein A is CH, and R₃, R₄ are as defined in table Z.

For example, compound U-1.001 has the following structure;

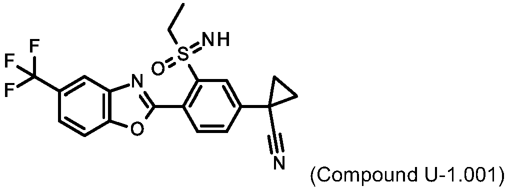

Table U-2 provides 8 compounds U-2.001 to U-2.008 of formula IaU wherein A is N, and R₃, R₄ are as defined in table Z.

The compounds of formula I according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects molluscs, nematodes or representatives of the order Acarina. The insecticidal, molluscicidal, nematicidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

Compounds of formula (I) according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile, improved physico-chemical properties, or increased biodegradability or environmental profile). In particular, it has been surprisingly found that certain compounds of formula (I) show an advantageous safety profile with respect to non-target organisms, for example, non-target arthropods, in particular pollinators such as honey bees, solitary bees, and bumble bees. Most particularly, Apis mellifera.

In this regard, certain compounds of formula (I) of the invention can be distinguished from known compounds by virtue of greater efficacy at low application rates, which can be verified by the person skilled in the art using experimental procedures similar to or adapted from those outlined in the biological examples, using lower application rates if necessary, for example 50 ppm, 12.5 ppm, 6 ppm, 3 ppm, 1.5 ppm, 0.8 ppm or 0.2 ppm.

Further it has surprisingly found that that compounds of formula (I) show advantageous physico-chemical properties for application in crop protection, in particular reduced melting point, reduced lipophilicity and increased water solubility. Such properties have been found to be advantageous for plant uptake and systemic distribution, see for example A. Buchholz, S. Trapp, Pest Manag Sci 2016; 72: 929-939) in order to control certain pest species named below.

Examples of the abovementioned animal pests are:
from the order *Acarina,* for example,
Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
Haematopinus spp., Linognathus spp., Pediculus spp., Pemphigus spp. and Phylloxera spp.;
from the order *Coleoptera,* for example,
Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.;
from the order *Diptera,* for example,
Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euschistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
Acromyrmex, Arge spp, Atta spp., Cephus spp., Diprion spp., Diprionidae, Gilpinia polytoma, Hoplo-campa spp., Lasius spp., Monomorium pharaonis, Neodiprion spp., Pogonomyrmex spp, Slenopsis invicta, Solenopsis spp. and Vespa spp.;
from the order *Isoptera,* for example,
Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Gra-pholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
Damalinea spp. and Trichodectes spp.;
from the order *Orthoptera,* for example,
Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
Liposcelis spp.;
from the order *Siphonaptera,* for example,
Ceratophyllus spp., Ctenocephalides spp. and Xenopsylla cheopis;
from the order *Thysanoptera,* for example,
Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. *B*. *elatior, B. semperflorens, B. tubéreux*), *Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. (*C. maritime*), *Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I. Walleriana*), *Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. *(P. peltatum, P. Zonale), Viola* spp. (pansy), *Petunia* spp., *Phlox* spp., *Plecthranthus* spp., *Poinsettia* spp., *Parthenocissus* spp. *(P. quinquefolia, P. tricuspidata), Primula* spp., *Ranunculus* spp., *Rhododendron* spp., Rosa spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. *(A. sativum, A.. cepa, A. oschaninii, A. Porrum, A. ascalonicum, A. fistulosum), Anthriscus cerefolium, Apium graveolus, Asparagus officinalis, Beta vulgarus, Brassica* spp. (*B. Oleracea, B. Pekinensis, B. rapa), Capsicum annuum, Cicer arietinum, Cichorium endivia, Cichorum* spp. (*C*. *intybus, C. endivia*), *Citrillus lanatus, Cucumis* spp. (*C. sativus, C. melo*), *Cucurbita* spp. (*C*. *pepo, C. maxima*), *Cyanara* spp. (*C*. *scolymus, C. cardunculus*), *Daucus carota, Foeniculum vulgare, Hypericum* spp., *Lactuca sativa, Lycopersicon* spp. (*L. esculentum, L. lycopersicum*), *Mentha* spp., *Ocimum basilicum, Petroselinum crispum, Phaseolus* spp. (*P. vulgaris, P*. *coccineus*), *Pisum sativum, Raphanus sativus, Rheum rhaponticum, Rosemarinus* spp., *Salvia* spp., *Scorzonera hispanica, Solanum melongena, Spinacea oleracea, Valerianella* spp. (*V. locusta, V. eriocarpa*) and *Vicia faba.*

Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 2002/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 2003/018810).

Examples of such toxins or transgenic plants capable of synthesising such toxins are disclosed, for example, in EP-A-0 374 753, WO1993/07278, WO1995/34656, EP-A-0 427 529, EP-A-451 878 and WO 2003/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 1990/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 2003/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
6. **1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch). The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 1995/33818 and EP-A-0 353 191. The methods of producing such transgenic plants are generally known to the person skilled in the art and are described, for example, in the publications mentioned above.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO1995/33818) or protein or polypeptide factors involved in plant pathogen defence (so-called "plant disease resistance genes", as described in WO 2003/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO2005/113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, *C*. *lurida*), *Rhizotrogus spp.* (e.g. European chafer, *R. majalis*), *Cotinus spp.* (e.g. Green June beetle, *C*. *nitida*), *Popillia spp.* (e.g. Japanese beetle, *P. japonica*), *Phyllophaga spp.* (e.g. May/June beetle), *Ataenius spp.* (e.g. Black turfgrass ataenius, *A. spretulus*), *Maladera spp.* (e.g. Asiatic garden beetle, *M. castanea*) and *Tomarus spp*.), ground pearls (*Margarodes* spp.), mole crickets (tawny, southern, and short-winged; *Scapteriscus* spp., *Gryllotalpa africana*) and leatherjackets (European crane fly, *Tipula spp*.).

The present invention may also be used to control insect pests of turfgrass that are thatch dwelling, including armyworms (such as fall armyworm *Spodoptera frugiperda,* and common armyworm *Pseudaletia unipuncta),* cutworms, billbugs (*Sphenophorus spp.,* such as *S*. *venatus verstitus* and S. *parvulus),* and sod webworms (such as *Crambus spp.* and the tropical sod webworm, *Herpetogramma phaeopteralis).*

The present invention may also be used to control insect pests of turfgrass that live above the ground and feed on the turfgrass leaves, including chinch bugs (such as southern chinch bugs, *Blissus insularis),* Bermudagrass mite *(Eriophyes cynodoniensis),* rhodesgrass mealybug *(Antonina graminis),* two-lined spittlebug (*Propsapia bicincta),* leafhoppers, cutworms (*Noctuidae* family), and greenbugs. The present invention may also be used to control other pests of turfgrass such as red imported fire ants (*Solenopsis invicta)* that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, *n*-hexane, *n*-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 l/ha, especially from 10 to 1000 l/ha.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25 % | 50% | 75% |
| sodium lignosulfonate | 5% | 5% | - |
| sodium lauryl sulfate | 3% | - | 5% |
| sodium diisobutylnaphthalenesulfonate | - | 6 % | 10 % |
| phenol polyethylene glycol ether (7-8 mol of ethylene oxide) | - | 2% | - |
| highly dispersed silicic acid | 5% | 10 % | 10 % |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50% | 75% |
| light mineral oil | 5% | 5% | 5% |
| highly dispersed silicic acid | 5% | 5% | - |
| Kaolin | 65 % | 40 % | - |
| Talcum | - | | 20 % |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3% |
| calcium dodecylbenzenesulfonate | 3% |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5% | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8 % |
| polyethylene glycol (mol. wt. 200) | 3 % |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6% |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5% |
| copolymer butanol PO/EO | 2% |
| Tristyrenephenole with 10-20 moles EO | 2% |
| 1,2-benzisothiazolin-3-one (in the form of a 20% solution in water) | 0.5 % |
| monoazo-pigment calcium salt | 5% |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹ H NMR measurements were recorded on a Brucker 400MHz spectrometer, chemical shifts are given in ppm relevant to a TMS standard. Spectra measured in deuterated solvents as indicated. Either one of the LCMS methods below was used to characterize the compounds. The characteristic LCMS values obtained for each compound were the retention time ("Rt", recorded in minutes) and the measured molecular ion (M+H)⁺ or (M-H)⁻.

### LCMS and GCMS Methods:

### Method 1:

Spectra were recorded on a Mass Spectrometer from Waters (SQD, SQDII Single quadrupole mass spectrometer) equipped with an electrospray source (Polarity: positive and negative ions, Capillary: 3.00 kV, Cone range: 30 V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment , diode-array detector and ELSD detector. Column: Waters UPLC HSS T3, 1.8 □m, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 10-100% B in 1.2 min; Flow (ml/min) 0.85

### Method 2:

Spectra were recorded on a Mass Spectrometer from Waters (SQD, SQDII Single quadrupole mass spectrometer) equipped with an electrospray source (Polarity: positive and negative ions), Capillary: 3.00 kV, Cone range: 30V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment , diode-array detector and ELSD detector. Column: Waters UPLC HSS T3, 1.8 □m, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 10-100% B in 2.7 min; Flow (ml/min) 0.85

### Method 3:

Spectra were recorded on a Mass Spectrometer from Waters (SQD, SQDII or ZQ Single quadrupole mass spectrometer) equipped with an electrospray source (Polarity: positive and negative ions), Capillary: 3.00 kV, Cone range: 30 V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment , diode-array detector and ELSD detector. Column: Waters UPLC HSS T3, 1.8 □m, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 0-10% B in 2.5 min; Flow (ml/min) 0.85

### Method 4:

Spectra were recorded on a Mass Spectrometer from Waters (SQD, SQDII Single quadrupole mass spectrometer) equipped with an electrospray source (Polarity: positive and negative ions), Capillary: 3.00 kV, Cone range: 30 V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment , diode-array detector and ELSD detector. Column: Waters UPLC HSS T3, 1.8 □m, 30 x 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 40-100% B in 1.2 min; Flow (ml/min) 0.85

### Method 5:

Spectra were recorded on a Mass Spectrometer from Waters (Acquity SDS Mass Spectrometer) equipped with an electrospray source (Polarity: Positive and Negative Polarity Switch, Capillary: 3.00 kV, Cone Voltage: 41.00 V, Source temperature: 150°C, Desolvation Gas Flow: 1000 L/Hr., Desolvation temperature: 500°C, Gas Flow @Cone: 50 L/hr., Mass range: 110-800 Da, PDA wavelength range: 210-400 nm. Column: Acquity UPLC HSS T3 C18, length 30 mm, diameter 2.1 mm, particle size 1.8 µm. Column oven temperature 40°C. Solvent gradient: A= Water with 0.1% formic acid : Acetonitrile (95:5 v/v). B= Acetonitrile with 0.05% formic acid. Gradient= 0 min 90% A, 10% B; 0.2 min 50% A, 50% B; 0.7-1.3 min 0% A, 100% B; 1.4-1.6 min 90% A, 10% B. Flow rate 0.6 mL/min.

### Example H1: Preparation of 1-[5-(ethylsulfonimidoyl)-6-[7-methyl-3-(trifluoromethyl)imidazo[4,5-c]pyridazin-6-y]-3-pyridyl]cyclopropanecarbonitrile (Table P, Example P10)

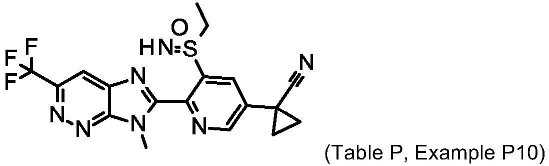

### Step 1: Preparation of methyl 5-(1-cyano-2-ethoxy-2-oxo-ethyl)-3-ethylsulfanyl-pyridine-2-carboxylate

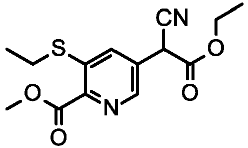

Methyl 5-bromo-3-ethylsulfanyl-pyridine-2-carboxylate, prepared as described in patent WO 2017/089190 (32 g, 115.88 mmol) was dissolved in dimethyl sulfoxide (350 mL). Then ethyl 2-cyanoacetate (18.5 mL, 173.82 mmol), potassium carbonate (40.442 g, 289.70 mmol) and tetrabutylammonium bromide (3.81 g, 11.588 mmol) were added successively at room temperature. The resulting suspension were stirred one night at 90°C and then cooled to room temperature. Water and ethyl acetate were added, the resulting mixture was cooled down at 0°C and hydrochloric acid (2M) was added slowly to acidify the reaction to pH 4-5. The aqueous layer was extracted with ethyl acetate three times. The combined organic layers were, dried over sodium sulfate, filtered and concentrated *in vacuo.* The crude obtained was heated at 80°C in ethanol (250 ml) for 1 hour. The solution obtained was cooled to 0°C, stirred 1 hour and filtered. The precipitate was washed with cold ethanol to afford methyl 5-(1-cyano-2-ethoxy-2-oxo-ethyl)-3-ethylsulfanyl-pyridine-2-carboxylate.

LCMS (method 1): 309 (M+H)⁺; retention time: 0.85 min.

1H NMR (400 MHz, CHLOROFORM-D) δ ppm 1.33 (t, J=7.15 Hz, 3 H) 1.45 (t, J=7.34 Hz, 3 H) 2.98 - 3.05 (m, 2 H) 4.04 (s, 3 H) 4.28 - 4.35 (m, 2 H) 4.84 (s, 1 H) 7.83 (d, J=1.83 Hz, 1 H) 8.49 (d, J=1.83 Hz, 1 H).

### Step 2: Preparation of methyl 5-(cyanomethyl)-3-ethylsulfanyl-pyridine-2-carboxylate

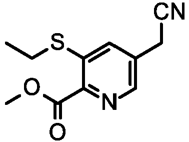

Methyl 5-(1-cyano-2-ethoxy-2-oxo-ethyl)-3-ethylsulfanyl-pyridine-2- (7.3 g, 24 mmol) was dissolved in dimethylsulfoxide (70 mL). To this was added NaCl (14 g, 240 mmol) and water (35 mL) successively at room temperature. The resulting suspension was stirred for 3 hours at 125°C. The reaction mixture was cooled to room temperature, diluted with 50 mL of water and 100mL of ethyl acetate. The aqueous layer was extracted with ethyl acetate three times. The organic layers were combined, dried over sodium sulfate and concentrated *in vacuo.* The crude product was purified over silica gel to afford methyl 5-(cyanomethyl)-3-ethylsulfanyl-pyridine-2-carboxylate.

LCMS (method 1): 237 (M+H)⁺; retention time: 0.72 min.

1H NMR (400 MHz, CHLOROFORM-D) δ ppm 1.45 (t, J=7.52 Hz, 3 H) 3.01 (q, J=7.34 Hz, 2 H) 3.87 (s, 2 H) 4.04 (s, 3 H) 7.72 (d, J=1.83 Hz, 1 H) 8.35 - 8.41 (m, 1 H).

### Step 3: Preparation of methyl 5-(1-cyanocyclopropyl)-3-ethylsulfanyl-pyridine-2-carboxylate

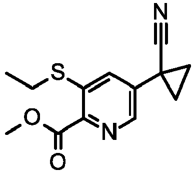

Methyl 5-(cyanomethyl)-3-ethylsulfanyl-pyridine-2-carboxylate (5 g, 21.16 mmol) was dissolved in acetonitrile (170 mL) and treated with cesium carbonate (20.7 g, 63.48 mmol) and 1,2-dibromoethane (2.19 mL, 25.39 mmol) at room temperature. The resulting mixture was stirred 3h 30 hours at 80°C and then at room temperature overnight. The reaction mixture was diluted with water and ethyl acetate. The aqueous layer was extracted 3 times with ethyl acetate. The combined organic layers were washed with brine, dried over sodium sulfate, filtered and concentrated *in vacuo* to afford the crude product which was purified by chromatography to afford methyl 5-(1-cyanocyclopropyl)-3-ethylsulfanyl-pyridine-2-carboxylate.

LCMS (method 1): 263 (M+H)⁺; retention time: 0.85 min.

1H NMR (400 MHz, CHLOROFORM-D) δ ppm 1.45 (t, J=7.34 Hz, 3 H) 1.54 - 1.62 (m, 2 H) 1.89 - 1.96 (m, 2 H) 3.01 (q, J=7.34 Hz, 2 H) 4.02 (s, 3 H) 7.74 (d, J=2.20 Hz, 1 H) 8.17 (d, J=1.83 Hz, 1 H).

### Step 4: Preparation of 5-(1-cyanocyclopropyl)-3-ethylsulfanyl-pyridine-2-carboxylic acid

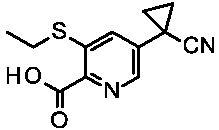

Methyl 5-(1-cyanocyclopropyl)-3-ethylsulfanyl-pyridine-2-carboxylate (2.63 g, 10.0 mmol) was dissolved in tetrahydrofurane (50 mL) and water (15 mL). Then lithium hydroxide (0.375 g, 15.0 mmol) was added and reaction was stirred one night at rt. After this time, a further portion of lithium hydroxide ( 0. 160 g, 7.0 mmol) was added and the reaction was stirred for a further 2 hours at RT. The reaction mixture was concentrated *in vacuo* and the residue was dissolved in dichloromethane. Aqueous HCl 1 M was added and the aqueous layer (pH 1) was extracted 3 times with dichloromethane. The combined organic layers were dried over sodium sulfate, filtered and concentrated *in vacuo* to afford 5-(1-cyanocyclopropyl)-3-ethylsulfanyl-pyridine-2-carboxylic acid.

LCMS (method 1): 249 (M+H)⁺; retention time: 0.67 min.

1H NMR (400 MHz, DMSO-d Solvent) δ ppm 1.26 (t, J=7.34 Hz, 3 H) 1.70 - 1.78 (m, 2 H) 1.83 - 1.92 (m, 2 H) 3.03 (q, J=7.34 Hz, 2 H) 7.63 (d, J=2.20 Hz, 1 H) 8.37 (d, J=1.83 Hz, 1 H) 13.16 - 13.40 (m, 1 H).

### Step 5. Preparation of 5-(1-cyanocyclopropyl)-3-ethylsulfanyl-N-[3-(methylamino)-6-(trifluoromethyl)pyridazin-4-yl]pyridine-2-carboxamide

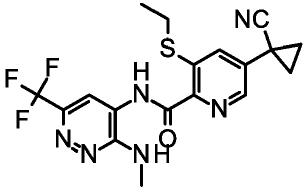

5-(1-cyanocyclopropyl)-3-ethylsulfanyl-pyridine-2-carboxylic acid (200 mg, 0.8055 mmol) was dissolved in dichloromethane (10 mL) and N,N-dimethylformamide (10.0 µL) was added. In this suspension, oxalyl dichloride (0.118 mL, 1.369 mmol,) was added dropwise via syringe. The resulting yellowish suspension was stirred at room temperature. After 1.5h, reaction mixture was concentrated *in vacuo* to afford 5-(1-cyanocyclopropyl)-3-ethylsulfanyl-pyridine-2-carbonyl chloride. The fresh 5-(1-cyanocyclopropyl)-3-ethylsulfanyl-pyridine-2-carbonyl chloride (250 mg, 0.9370 mmol) was dissolved in tetrahydrofurane (12 mL) and N,N-diethylethanamine (0.27 mL, 1.933 mmol) was added dropwise N3-methyl-6-(trifluoromethyl)pyridazine-3,4-diamine (651 mg, 3.222 mmol) in tetrahydrofurane (12 mL) was then added at 0°C. The resulting mixture was stirred at room temperature for 12 hours. The reaction mixture was then poured into water (20 ml) and extracted with dichloromethane (3x 20 ml ). The combined extracts were washed with brine (30 ml), dried with sodium sulfate, filtered and concentrated *in vacuo.* The crude product was purified by chromatography over silica gel to afford N-[4-amino-6-(trifluoromethyl)pyridazin-3-yl]-5-(1-cyanocyclopropyl)-3-ethylsulfanyl-N-methyl-pyridine-2-carboxamide

LCMS (method 2): retention time: 1.04min, 423 (M+H)⁺.

### Step 6: Preparation of 1-[5-ethylsulfanyl-6-[7-methyl-3-(trifluoromethyl)imidazo[4,5-c]pyridazin-6-yl]-3-pyridyl]cyclopropanecarbonitrile

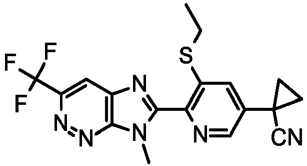

5-(1-cyanocyclopropyl)-3-ethylsulfanyl-N-[3-(methylamino)-6-(trifluoromethyl)pyridazin-4-yl]pyridine-2-carboxamide (240 mg, 0.5682 mmol) and glacial acetic acid (3 mL) were mixed and stirred one night at reflux and then cooled to room temperature. Acetic acid was removed under reduced pressure and the residue obtained was dissolved in ethyl acetate, and basified using aqueous bicarbonate-solution. The aqueous layer was extracted with ethyl acetate (3x 20 ml ) and the combined organic layer were dried with sodium sulfate, filtered and concentrated *in vacuo.* The crude product was purified by chromatography over silica gel to afford 1-[5-ethylsulfanyl-6-[7-methyl-3-(trifluoromethyl)imidazo[4,5-c]pyridazin-6-yl]-3-pyridyl]cyclopropanecarbonitrile.

LCMS (method 2): retention time: 1.05 min, (M+H)⁺ 405.

1H NMR (400 MHz, CHLOROFORM-d) d ppm 1.34 - 1.46 (m, 1 H) 1.38 (s, 2 H) 1.62 - 1.66 (m, 2 H) 1.89 - 2.02 (m, 2 H) 2.97 - 3.10 (m, 2 H) 4.26 (s, 3 H) 7.76 - 7.84 (m, 1 H) 8.22 - 8.27 (m, 1 H) 8.34 - 8.41 (m, 1 H).

### Step 7: Preparation of 1-[5-(ethylsulfonimidoyl)-6-[7-methyl-3-(trifluoromethyl)imidazo[4,5-c]pyridazin-6-vll-3-pyridyllcyclopropanecarbonitrile (Table P, Example P10)

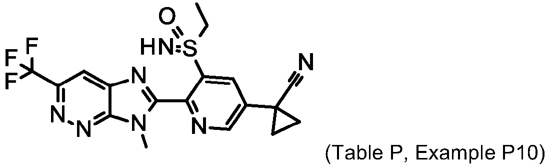

1-[5-ethylsulfanyl-6-[7-methyl-3-(trifluoromethyl)imidazo[4,5-c]pyridazin-6-yl]-3-pyridyl]cyclopropanecarbonitrile (120 mg, 0.2967 mmol) was dissolved in methanol (6 mL). Then ammonium carbamate (46 mg, 0.5935 mmol) and (diacetoxyiodo)benzene (243 mg, 0.7418 mmol) were added at room temperature. The suspension obtained became a solution after a few minutes and was stirred for 2 hours at room temperature. The reaction mixture was quenched with water (20 ml) and extracted with dichloromethane (3x 20 ml ). The combined organic layers were washed with brine (30 ml), dried with anhydrous sodium sulphate, filtered and concentrated *in vacuo.* The residue was purified by chromatography over silica gel using methanol and dichloromethane to afford the 1-[5-(ethylsulfonimidoyl)-6-[7-methyl-3-(trifluoromethyl)imidazo[4,5-c]pyridazin-6-yl]-3-pyridyl]cyclopropanecarbonitrile.

LCMS (method 2): retention time: 0.85 min, 436.36 (M+H)⁺.

1H NMR (400 MHz, CHLOROFORM-d) d ppm 1.14 - 1.32 (m, 3 H) 1.25 - 1.32 (m, 2 H) 1.37 - 1.44 (m, 3 H) 1.67 - 1.77 (m, 2 H) 2.03 - 2.12 (m, 1 H) 2.06 -2.10 (m, 1 H) 2.11 (br dd, J=3.48, 2.38 Hz, 1 H) 2.68 - 2.84 (m, 1 H) 3.71 - 3.81 (m, 1 H) 3.84 - 3.94 (m, 1 H) 4.00 - 4.14 (m, 3 H) 8.18 (s, 1 H) 8.36 (d,J=2.20 Hz, 1 H) 9.04 (d, J=2.20 Hz, 1 H)

### Example H2. Preparation of 1-[5-(ethylsulfonimidoyl)-6-[3-methyl-6-(trifluoromethyl)imidazo[4,5-b]pyridin-2-yl]-3-pyridyl]cyclopropanecarbonitrile (Example P6, Table P) - reference example

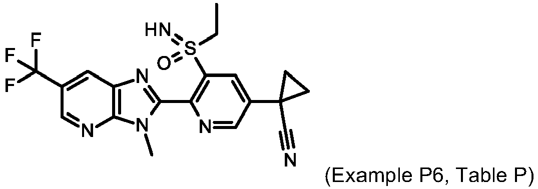

### Step 1:Preparation of 5-(1-cyanocyclopropyl)-3-ethylsulfanyl-N-[2-(methylamino)-5-(trifluoromethyl)-3-pyridyllpyridine-2-carboxamide

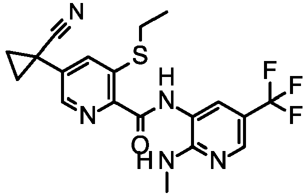

5-(1-cyanocyclopropyl)-3-ethylsulfanyl-pyridine-2-carboxylic acid (2.4 g, 9.7 mmol) was dissolved in dichloromethane (100 mL). N,N-dimethylformamide (10.0 µL) was added followed by the addition of oxalyl dichloride (1 mL, 12 mmol) dropwise via syringe. The resulting yellowish suspension was stirred at room temperature for 1 hour then the solvent was concentrated *in vacuo.* The resulting solid was dissolved in tetrahydrofurane (30 ml) and added to a solution of N2-methyl-5-(trifluoromethyl)pyridine-2,3-diamine (1.8 g, 9.7 mmol) and N,N-diethylethanamine (3.3 mL, 23 mmol) in tetrahydrofurane (75 mL) at 0°C. The resulting mixture was stirred 30' at 0°C then 2 hours at room temperature. The reaction mixture was treated with NH4Cl sat sol and diluted with ethyl acetate. The aqueous layer was extracted 3 times with ethyl acetate. The combined organic layers were washed with brine, dried over Na₂SO₄, filtered and concentrated *in vacuo* to afford 5-(1-cyanocyclopropyl)-3-ethylsulfanyl-N-[2-(methylamino)-5-(trifluoromethyl)-3-pyridyl]pyridine-2-carboxamide as crudewhich was used in the next step without further purification.

LCMS (method 1): 422 (M+H)⁺; retention time: 1.03 min.

1H NMR (400 MHz, CHLOROFORM-d) δ ppm 1.48 (t, J=7.34 Hz, 3 H) 1.58 - 1.63 (m, 2 H) 1.93 - 1.99 (m, 2 H) 3.01 (q, J=7.34 Hz, 2 H) 3.09 (d, J=4.77 Hz, 3 H) 5.02 (br d, J=4.03 Hz, 1 H) 7.71 (d, J=2.20 Hz, 1 H) 7.94 (d, J=2.20 Hz, 1 H) 8.16 (d, J=2.20 Hz, 1 H) 8.36 (d, J=0.73 Hz, 1 H) 9.53 - 9.60 (m, 1 H).

### Step 2: Preparation of 1-[5-ethylsulfanyl-6-[3-methyl-6-(trifluoromethyl)imidazo[4,5-b]pyridin-2-y]-3-pyridyl]cyclopropanecarbonitrile

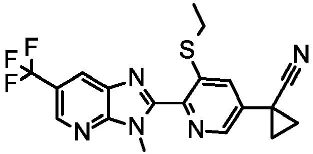

5-(1-cyanocyclopropyl)-3-ethylsulfanyl-N-[2-(methylamino)-5-(trifluoromethyl)-3-pyridyl]pyridine-2-carboxamide (4.2 g, 10 mmol) was dissolved in acetic acid (100 mL) and the resulting solution was stirred 18 hours at 110°C. Acetic acid was removed by *in vacuo* and the crude product was purified by chromatography over silica gel to afford 1-[5-ethylsulfanyl-6-[3-methyl-6-(trifluoromethyl)imidazo[4,5-b]pyridin-2-yl]-3-pyridyl]cyclopropanecarbonitrile, Mp 142-144 °C.

LCMS (method 1): 404 (M+H)⁺; retention time: 1.07 min.

1H NMR (400 MHz, CHLOROFORM-d) δ ppm 1.40 (t, J=7.34 Hz, 3 H) 1.59 - 1.64 (m, 2 H) 1.90 - 1.97 (m, 2 H) 3.03 (q, J=7.46 Hz, 2 H) 4.07 (s, 3 H) 7.77 (d, J=2.20 Hz, 1 H) 8.35 (d, J=2.20 Hz, 1 H) 8.42 (d, J=1.47 Hz, 1 H) 8.73 - 8.78 (m, 1 H).

### Step 3: Preparation of 1-[5-(ethylsulfonimidoyl)-6-[3-methyl-6-(trifluoromethylimidazo[4,5-b]pyridin-2-vll-3-pyridyllcyclopropanecarbonitrile (Example P6, Table P)

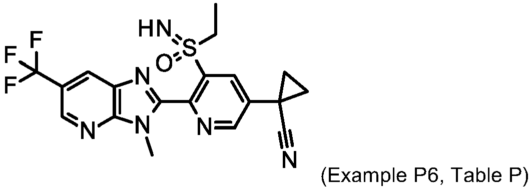

Sulfoximine formation analogous to Example H1, Step 7.

LCMS (method 1): 435 (M+H)⁺; retention time: 0.87 min.

¹H NMR (400 MHz, Chloroform) δ ppm 1.44 (t, *J*=7.34 Hz, 3 H) 1.75 - 1.80 (m, 2 H) 2.02 - 2.08 (m, 2 H) 3.94 (s, 3 H) 3.94 - 4.26 (m, 2 H) 8.33 (d, *J*=1.47 Hz, 1 H) 8.39 (d, *J*=2.20 Hz, 1 H) 8.79 (d, *J*=1.47 Hz, 1 H) 9.06 - 9.09 (m, 1 H).

### Example H3: Preparation of 1-[5-(ethylsulfonimidoyl)-6-[5-methoxy-3-methyl-4-oxo-6-(trifluoromethyl)imidazo[4.5-c]pyridin-2-yl]-3-pyridyl]cyclopropanecarbonitrile (Example P8, Table P) - reference example

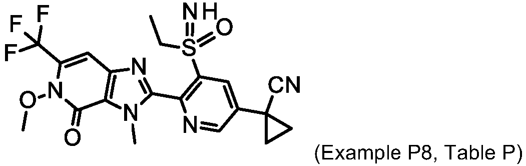

### Step 1: Preparation of N-[4-bromo-6-(difluoromethyl)-1-methoxy-2-oxo-3-pyridyl]-2,2,2-trifluoro-N-methyl-acetamide

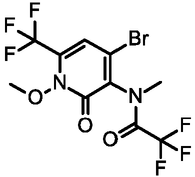

To a solution of 4-bromo-1-methoxy-3-(methylamino)-6-(trifluoromethyl)pyridin-2-one (5.0 g, 16.61 mmol) in dichloromethane (100 mL) was added trifluoroacetic anhydride (7.09 mL, 49.82 mmol) at room temperature. The reaction mixture was stirred for 30 minutes at room temperature and then concentrated *in vacuo.* Water (100 mL), then an aqueous saturated potassium carbonate solution (50 mL) were added and the aqueous layer was extracted with ethyl acetate (100 mL). The organic layer was washed with brine (100 mL), dried over sodium sulfate, filtered and concentrated *in vacuo.* The crude product was purified over silica gel to afford pure N-[4-bromo-6-(difluoromethyl)-1-methoxy-2-oxo-3-pyridyl]-2,2,2-trifluoro-N-methyl-acetamide.

This material was used in the next step without further purification.

LCMS (method 5): 397/399 (M+H)⁺, retention time 0.96 min. ¹H NMR (400 MHz, CDCl₃) δ ppm 3.27 (s, 3H), 4.16 (s, 3H), 6.84 (s, 1H).

### Step 2: Preparation of N-[4-azido-1-methoxy-2-oxo-6-(trifluoromethyl)-3-pyridyl]-2,2,2-trifluoro-N-methyl-acetamide

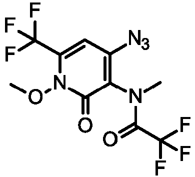

To a solution of N-[4-bromo-1-methoxy-2-oxo-6-(trifluoromethyl)-3-pyridyl]-2,2,2-trifluoro-N-methylacetamide (11.8 g, 29.7 mmol) in N,N-dimethylformamide (110 mL) was added sodium azide (2.9 g, 44.6 mmol) at room temperature. The reaction mixture was stirred at room temperature overnight. The above reaction was separately duplicated, then the combined reaction mixtures were diluted with cold water (500 mL) and extracted with ethyl acetate (3x 150 mL). The combined organic layers were washed with water (100 mL) and brine, dried over sodium sulfate, filtered and concentrated *in vacuo* below 40°C to afford N-[4-azido-1-methoxy-2-oxo-6-(trifluoromethyl)-3-pyridyl]-2,2,2-trifluoro-N-methyl-acetamide. This material was used in the next step without further purification.

LCMS (method 5): 360 (M+H)⁺, retention time 0.90 min. ¹H NMR (400 MHz, CDCl₃) δ ppm 3.23 (s, 3H), 4.15 (s, 3H), 6.40 (s, 1H).

### Step 3: Preparation of 4-azido-1-methoxy-3-(methylamino)-6-(trifluoromethyl)pyridin-2-one

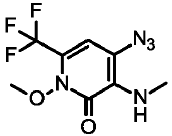

To a solution of N-[4-azido-1-methoxy-2-oxo-6-(trifluoromethyl)-3-pyridyl]-2,2,2-trifluoro-N-methylacetamide (4.6 g, 13.0 mmol) in methanol (100 mL) was added potassium carbonate (4.7 g, 33.0 mmol). The reaction mixture was stirred at room temperature overnight, then diluted with water (150 mL). The aqueous layer was extracted with ethyl acetate (2x 75 mL), the combined organic layers washed with brine (150 mL), dried over sodium sulfate, filtered and concentrated *in vacuo.* The crude product was purified on silica gel (40% ethyl acetate in cyclohexane) to afford 4-azido-1-methoxy-3-(methylamino)-6-(trifluoromethyl)pyridin-2-one (2.2 g, 8.4 mmol).

LCMS (method E): 264 (M+H)⁺, retention time 0.94 min. ¹H NMR (400 MHz, CDCl₃) δ ppm 3.18 (s, 3H), 4.11 (s, 3H), 6.46 (s, 1H).

### Step 4: Preparation of 4-amino-1-methoxy-3-(methylamino)-6-(trifluoromethyl)pyridin-2-one

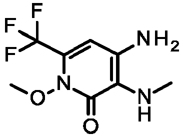

To a solution of 4-azido-1-methoxy-3-(methylamino)-6-(trifluoromethyl)pyridin-2-one (1.7 g, 6.5 mmol) in tetrahydrofurane (50 mL) and water (5 mL) at room temperature was added triphenylphosphine (5.1 g, 19 mmol) and the resulting mixture stirred at room temperature for 2 hours. A 2M aqueous hydrochloric acid solution (9 mL, 18 mmol, 2 mol/L) was added and stirring continued overnight at room temperature. The reaction mixture was concentrated and quenched using an aqueous saturated potassium carbonate solution (20 mL). The aqueous layer was extracted with ethyl acetate (2x75 mL), the combined organic layers washed with brine (100 mL),dried over sodium sulfate, filtered and concentrated *in vacuo.* The residue was purified on silica gel (50-60% ethyl acetate in cyclohexane) to afford 4-amino-1-methoxy-3-(methylamino)-6-(trifluoromethyl)pyridin-2-one.

LCMS (method 5): 238 (M+H)⁺, retention time 0.18 min. ¹H NMR (400 MHz, d₆-DMSO) δ ppm 2.60 (s, 3H), 3.98 (s, 3H), 5.75 (s, 2H), 6.42 (s, 1H).

### Step 5: Preparation of 5-(1-cyanocyclopropyl)-3-ethylsulfanyl-N-[1-methoxy-3-(methylamino)-2-oxo-6-(trifluoromethyl)-4-pyridyl]pyridine-2-carboxamide and N-[4-amino-1-methoxy-2-oxo-6-(trifluoromethyl)-3-pyridyl]-5-(1-cyanocyclopropyl)-3-ethylsulfanyl-N-methyl-pyridine-2-carboxamide (isomeric mixture)

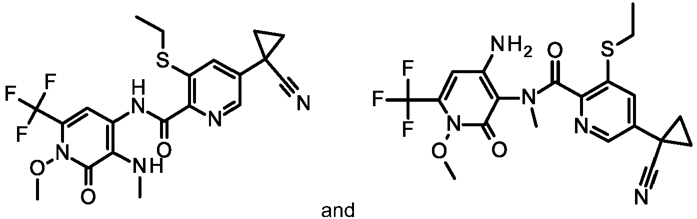

To a suspension of 5-(1-cyanocyclopropyl)-3-ethylsulfanyl-pyridine-2-carboxylic acid (400 mg, 1.61 mmol) in dichloromethane (16 mL) were added a catalytic amount of N,N-dimethylformamide (2 drops) and oxalyl chloride (3.22 mmol, 0.287 mL) dropwise. The reaction was stirred at room temperature for 6 hours and the solvent was removed *in vacuo* to afford 5-(1-cyanocyclopropyl)-3-ethylsulfanyl-pyridine-2-carbonyl chloride.

A solution of above 5-(1-cyanocyclopropyl)-3-ethylsulfanyl-pyridine-2-carbonyl chloride (428 mg, 1.60 mmol) in dry tetrahydrofurane (20 mL) was added slowly to a mixture of 4-amino-1-methoxy-3-(methylamino)-6-(trifluoromethyl)pyridin-2-one (456.6 mg, 1.92 mmol) and triethylamine (0.678 mL, 4.81 mmol) in tetrahydrofurane (9.6 mL). The reaction mixture was stirred at room temperature for 2 hours, then quenched with water and extracted with dichloromethane (100 ml). The combined organic layers were washed with water and brine, dried with anhydrous sodium sulfate and concentrated *in vacuo* to afford the desired isomeric mixture of 5-(1-cyanocyclopropyl)-3-ethylsulfanyl-N-[1-methoxy-3-(methylamino)-2-oxo-6-(trifluoromethyl)-4-pyridyl]pyridine-2-carboxamide and N-[4-amino-1-methoxy-2-oxo-6-(trifluoromethyl)-3-pyridyl]-5-(1-cyanocyclopropyl)-3-ethylsulfanyl-N-methyl-pyridine-2-carboxamide (750 mg, 1.60 mmol). This material was used in the next step without further purification. LCMS (method 5): 468 (M+H)⁺, retention time 0.86 min.

### Step 6: Preparation of 1-[5-ethylsulfanyl-6-[5-methoxy-3-methyl-4-oxo-6-(trifluoromethyl) imidazo[4,5-c]pyridin-2-yl]-3-pyridyl]cyclopropanecarbonitrile

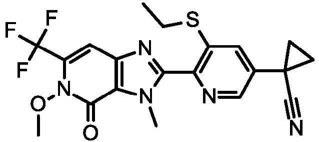

A solution of above isomeric mixture of 5-(1-cyanocyclopropyl)-3-ethylsulfanyl-N-[1-methoxy-3-(methylamino)-2-oxo-6-(trifluoromethyl)-4-pyridyl]pyridine-2-carboxamide and N-[4-amino-1-methoxy-2-oxo-6-(trifluoromethyl)-3-pyridyl]-5-(1-cyanocyclopropyl)-3-ethylsulfanyl-N-methyl-pyridine-2-carboxamide (750 mg, 1.60 mmol) in acetic acid (4.8 mL) was heated to 100°C for 48 hours. After cooling to room temperature, the reaction mixture was concentrated *in vacuo.* The residue was poured into water and extracted with ethyl acetate (3x 100 mL), the combined organic layers washed with brine, dried over sodium sulfate and concentrated *in vacuo.* The crude product was purified by chromatography on silica gel (40% ethyl acetate in cyclohexane) to afford the desired product 1-[5-ethylsulfanyl-6-[5-methoxy-3-methyl-4-oxo-6-(trifluoromethyl)imidazo[4,5-c]pyridin-2-yl]-3-pyridyl] cyclopropanecarbonitrile (350 mg) as a solid. LCMS (method E): 450 (M+H)⁺, retention time 1.03 min. ¹H NMR (400 MHz, CDCl₃) δ ppm 1.37 (t, 3H), 1.59 (m, 2H), 1.93 (m, 2H), 3.01 (q, 2H), 4.20 (s, 3H), 4.21 (s, 3H), 7.25 (s, 1H), 7.76 (d, *J*=2.0 Hz, 1H), 8.32 (d, *J*=2.0 Hz, 1H).

### Step 7: Preparation of 1-[5-(ethylsulfonimidoyl)-6-[5-methoxy-3-methyl-4-oxo-6-(trifluoromethyl)imidazo[4,5-c]pyridin-2-yl]-3-pyridyl]cyclopropanecarbonitrile (Example P8, Table P)

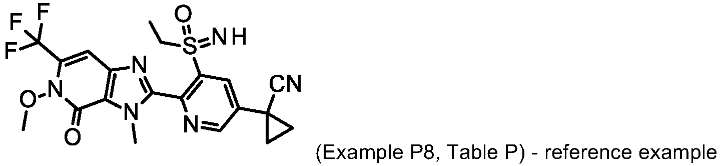

Sulfoximine formation analogous to Example H1, Step 7.

LCMS (method 5): 481 (M+H)⁺; retention time: 0.85 min.

¹H NMR (400 MHz, DMSO-d6) δ ppm 1.14 (t, J=7.40 Hz, 3 H) 1.83 - 1.94 (m, 2 H) 1.97 - 2.06 (m, 2 H) 3.44 - 3.66 (m, 2 H) 3.86 (s, 3 H) 4.12 (s, 3 H) 4.56 (s, 1 H) 7.41 (s, 1 H) 8.38 (d, J=2.20 Hz, 1 H) 8.90 (d, J=2.20 Hz, 1 H).

### Example H4: Preparation of 1-[3-(ethylsulfonimidoyl)-4-[3-methyl-6-(trifluoromethylsulfanylimidazo[4,5-c]pyridin-2-yl]phenyl]cyclopropanecarbonitrile (Example P9, Table P) - reference example

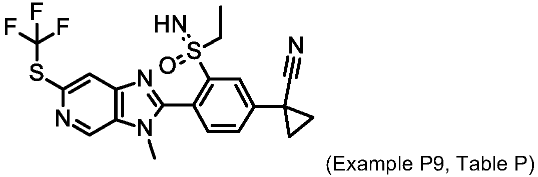

### Step 1: Preparation of N-[4-amino-6-(trifluoromethylsulfanyl)-3-pyridyl]-4-bromo-2-ethylsulfanyl-N-methyl-benzamide

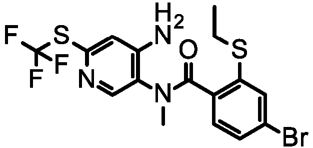

Under argon, 4-bromo-2-ethylsulfanyl-benzoic acid (prepared as described in patent WO 2016/120182 (1.96 g, 7.51 mmol)) was suspended in dichloromethane (30 mL) and 2 drops of DMF were added. oxalyl dichloride (1.24 g, 0.851 mL, 9.76 mmol) was added dropwise and gas evolution was observed. The mixture was stirred 4 hours at room temperature and then concentrated *in vacuo* to give 4-bromo-2-ethylsulfanyl-benzoyl chloride as product of the first step. Under argon, the crude 4-bromo-2-ethylsulfanyl-benzoyl chloride (2.07 g, 7.39 mmol) freshly prepared, was dissolved in tetrahydrofurane (20 mL) and N3-methyl-6-(trifluoromethylsulfanyl)pyridine-3,4-diamine prepared as described in patent WO 2016/169886 (1.50 g, 6.72 mmol) was added. The resulting mixture was stirred one night at room temperature then 8 hours at 70°C. Sodium bicarbonate and water were added after cooling at room temperature. The aqueous layer was extracted 2 times with ethyl acetate. The combined organic layer were dried over sodium sulfate, filtered and concentrated *in vacuo* to afford a mixture containing N-[4-amino-6-(trifluoromethylsulfanyl)-3-pyridyl]-4-bromo-2-ethylsulfanyl-N-methyl-benzamide.

LCMS (method 1): 450 (M+H)⁺; retention time: 1.14 min.

### Step 2: Preparation of 2-(4-bromo-2-ethylsulfanyl-phenyl)-3-methyl-6-(trifluoromethylsulfanyl)imidazo [4,5-c] pyridine

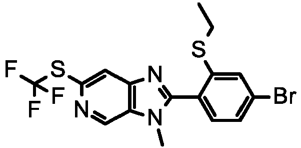

N-[4-amino-6-(trifluoromethylsulfanyl)-3-pyridyl]-4-bromo-2-ethylsulfanyl-N-methyl-benzamide (3.4 g, 5.1 mmol) was dissolved in acetic acid (51 mL). The resulting solution was stirred one night at 120°C and then cooled down at room temperature. Acetic acid was removed under reduced pressure and the crude was purified by chromatography over silica gel

The mixture obtained was dissolved in ethyl acetate, washed with bicarbonate, dried over sodium sulfate, filtered and concentrated *in vacuo* to afford 2-(4-bromo-2-ethylsulfanyl-phenyl)-3-methyl-6-(trifluoromethylsulfanyl)imidazo [4,5-c] pyridine.

LCMS (method 1): 450 (M+H)⁺; retention time: 1.14 min.

1H NMR (400 MHz, Chloroform) δ ppm 1.29 (t, J=7.34 Hz, 3 H) 2.92 (q, J=7.34 Hz, 2 H) 3.79 (s, 3 H) 7.32 (d, J=8.07 Hz, 1 H) 7.50 (dd, J=8.07, 1.83 Hz, 1 H) 7.61 (d, J=1.83 Hz, 1 H) 8.15 - 8.17 (m, 1 H) 8.90 (d, J=1.10 Hz, 1 H).

### Step 3:Preparation of 4-[3-ethylsulfanyl-4-[3-methyl-6-(trifluoromethylsulfanyl)imidazo[4,5-c]pyridin-2-yl]phenyl]isoxazole

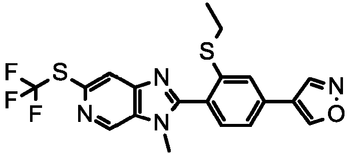

DMSO (1.11 mL) and water (0.53 mL) were added in a microwave vial under argon and the solution was purged with argon for 5 min. Then 2-(4-bromo-2-ethylsulfanyl-phenyl)-3-methyl-6-(trifluoromethylsulfanyl)imidazo[4,5-c]pyridine (0.2 g, 0.446mmol), 4-isoxazoleboronic acid pinacol ester (0.104 g, 0.535 mmol) and potassium fluoride (0.077 g, 1.33 mmol) were added. Dichloropalladium;triphenylphosphane (0.0031 g, 0.0044 mmol) was added, the resulting mixture was purged with argon for 5 min, stirred for 40 min at 90°C in a micro wave system, cooled down at room temperature and then poured in ice-water. The aqueous layer was extracted 3 times with dichloromethane. Then the milky aqueous layer was filtered and the solid obtained was dissolved in dichloromethane and added to the organic layer. The combined organic layers was dried over sodium sulfate, filtered and concentrated *in vacuo.* The oil obtained was washed with water. The precipitate formed in water was filtered then dissolved in dichloromethane again and the solvent was concentrated *in vacuo* to afford 4-[3-ethylsulfanyl-4-[3-methyl-6-(trifluoromethylsulfanyl)imidazo[4,5-c]pyridin-2-yl]phenyl]isoxazole, which was used as such for the next step.

LCMS (method 1): 437 (M+H)⁺; retention time: 0.95 min.

### Step 4: Preparation of 2-[3-ethylsulfanyl-4-[3-methyl-6-(trifluoromethylsulfanyl)imidazo[4,5-c]pyridin-2-yl]phenyl]acetonitrile

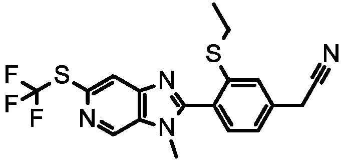

4-[3-ethylsulfanyl-4-[3-methyl-6-(trifluoromethylsulfanyl)imidazo[4,5-c]pyridin-2-yl]phenyl]isoxazole (236 mg, 0.3785 mmol, 70 mass%) was dissolved in methanol (2 mL) and water (1.14 mL) , and potassium fluoride (1.14 mL, 1.135 mmol) was added . The reaction mixture was stirred for 3 hours at 90°C. The dark red reaction mixture (suspension ) was filtered, washed with dichloromethane and concentrated *in vacuo.* The crude was purified by chromatography over silica gel column to afford 2-[3-ethylsulfanyl-4-[3-methyl-6-(trifluocromethylsulfanyl)imidazo[4,5-c]pyridin-2-yl]phenyl]acetonitrile.

LCMS (method 1): 409 (M+H)⁺; retention time: 0.98 min.

1H NMR (400 MHz, Chloroform) δ ppm 1.29 (t, J=7.34 Hz, 4 H) 2.93 (q, J=7.34 Hz, 2 H) 3.80 (s, 3 H) 3.89 (s, 2 H) 7.34 (dd, J=7.89, 1.65 Hz, 1 H) 7.47 - 7.48 (m, 1 H) 7.48 - 7.51 (m, 1 H) 8.16 - 8.18 (m, 1 H) 8.92 (d, J=0.73 Hz, 1 H).

### Step 5: Preparation of 1-[3-ethylsulfanyl-4-[3-methyl-6-(trifluoromethylsulfanyl)imidazo[4,5-c]pyridin-2-yl]phenyl]cyclopropanecarbonitrile

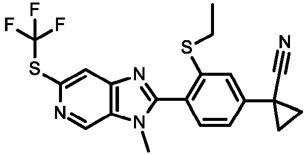

1-[3-ethylsulfanyl-4-[3-methyl-6-(trifluoromethylsulfanyl)imidazo[4,5-c]pyridin-2-yl]phenyl]cyclopropanecarbonitrile (0.108 g, 0.2644 mmol) was dissolved in acetonitrile (1.322 mL) . Then dicesium carbonate (0.2584 g, 0.7931 mmol) and 1,2-dibromoethane (0.1490 g, 0.0683 mL, 0.7931 mmol) were added. The reaction mixture was stirred under microwave system at 100°C for 2 hours. The reaction mixture was concentrated *in vacuo.* The residue was dissolved in ethyl acetate and washed several times with water and brine. Organic layer was dried over sodium sulfate, filtered and concentrated *in vacuo.* The crude product was purified chromatography over silica gel to afford 1-[3-ethylsulfanyl-4-[3-methyl-6-(trifluoromethylsulfanyl)imidazo[4,5-c]pyridin-2-yl]phenyl]cyclopropanecarbonitrile.

LCMS (method 1): 435 (M+H)⁺; retention time: 1.04 min.

¹H NMR (400 MHz, Chloroform) δ ppm 1.28 (t, *J*=7.34 Hz, 4 H) 1.55 (br d, *J*=2.20 Hz, 2 H) 1.86 - 1.91 (m, 2 H) 2.93 (q, *J*=7.58 Hz, 2 H) 3.79 (s, 3 H) 7.17 (dd, *J*=7.89, 2.02 Hz, 1 H) 7.44 - 7.47 (m, 1 H) 7.52 (d, *J*=1.83 Hz, 1 H) 8.16 (d, *J*=0.73 Hz, 1 H) 8.91 (d, *J*=0.73 Hz, 1 H).

### Step 6: Synthesis of 1-[3-(ethylsulfonimidoyl)-4-[3-methyl-6-(trifluoromethylsulfanyl)imidazo[4,5-c]pyridin-2-yl]phenyl]cyclopropanecarbonitrile (Example P9, Table P) - reference example

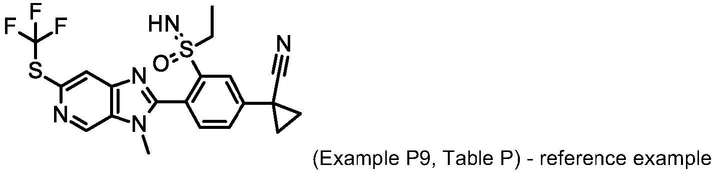

### Sulfoximine formation carried out as described in Example H1, step 7.

LCMS (method 1): 466 (M+H)⁺; retention time: 0.86 min.

### Example H5:Synthesis of 1-[3-(ethylsulfonimidoyl)-4-[3-methyl-6-(trifluoromethyl)imidazo[4,5-c]pyridin-2-yl]phenylcyclopropanecarbonitrile (Example P13, Table P) - reference example

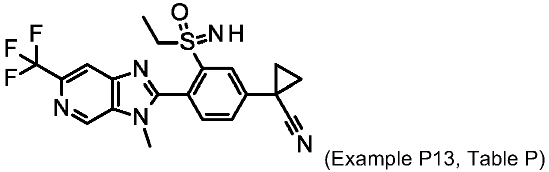

### Step 1: Preparation of 1-[3-ethylsulfanyl-4-[3-methyl-6-(trifluoromethyl)imidazo[4,5-c]pyridin-2-yl]phenyl]cyclopropanecarbonitrile

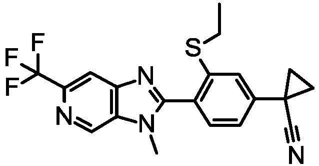

To a mixture of 2-(4-bromo-2-ethylsulfanyl-phenyl)-3-methyl-6-(trifluoromethyl)imidazo[4,5-c]pyridine prepared as described in patent WO 2016/030229 (0.5 g, 1.201 mmol), Pd₂dba₃ (0.056 g, 0.060 mmol) and BINAP (0.077 g, 0.120 mmol) in tetrahydrofurane (1.2 mL) were added cyclopropanecarbonitrile (0.090 g, 1.32 mmol) and cyclopropylmethylether (1.20 mL) under argon atmosphere at rt. The mixture was cooled down at -25°C and

Lithium bis(trimethylsilyl)amide (1.3 mL, 1.321 mmol) was added dropwise at -25°C, under argon atmosphere. The mixture was stirred at 80°C for 2 hours. After cooling at room temperature, the mixture was filtered over a pad of celite which was washed with ethyl acetate. The filtrate was washed with water. The aqueous layer was separated and extracted twice with ethyl acetate. The organic layers were combined, dried over anhydrous sodium sulfate, filtered, and concentrated *in vacuo.* The crude product was purified by chromatography over silica gel to afford 1-[3-ethylsulfanyl-4-[3-methyl-6-(trifluoromethyl)imidazo[4,5-c]pyridin-2-yl]phenyl]cyclopropanecarbonitrile.

LCMS (method 1): 403 (M+H)⁺; retention time: 1.00 min.

1H NMR (400 MHz, Chloroform) δ ppm 1.27 (t, J=7.34 Hz, 3 H) 1.52 - 1.57 (m, 2 H) 1.86 - 1.91 (m, 2 H) 2.91 (q, J=7.46 Hz, 2 H) 3.82 (s, 3 H) 7.18 (dd, J=8.07, 1.83 Hz, 1 H) 7.46 (d, J=8.07 Hz, 1 H) 7.52 (d, J=1.83 Hz, 1 H) 8.14 (d, J=0.73 Hz, 1 H) 8.95 (s, 1 H).

### Step 2: Synthesis of 1-[3-(ethylsulfonimidoyl)-4-[3-methyl-6-(trifluoromethyl)imidazo[4,5-c]pyridin-2-yl]phenyl]cyclopropanecarbonitrile (Example P13, Table P) - reference example

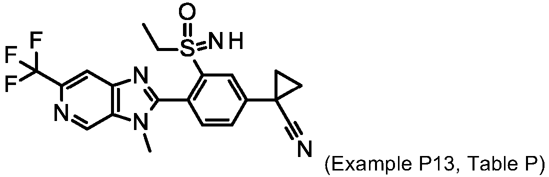

The desired product was prepared using the standard procedure described step 7, example H1. LCMS (method 1): 434 (M+H)⁺; retention time: 0.83 min.

1H NMR (400 MHz, Chloroform) δ ppm 1.28 (t, J=7.34 Hz, 3 H) 1.63 - 1.68 (m, 2 H) 1.96 - 2.01 (m, 2 H) 3.42 - 3.62 (m, 2 H) 3.75 (s, 3 H) 7.56 (d, J=8.07 Hz, 1 H) 7.89 (dd, J=7.89, 2.02 Hz, 1 H) 8.05 (d, J=1.83 Hz, 1 H) 8.10 (d, J=0.73 Hz, 1 H) 8.93 - 8.96 (m, 1 H).

### Example H5: Synthesis of 1-[3-(ethylsulfonimidoyl)-4-[3-methyl-6-(trifluoromethyl)imidazo[4,5-b]pyridin-2-yl]phenyl]cyclopropanecarbonitrile (Example P15, Table P) - reference example

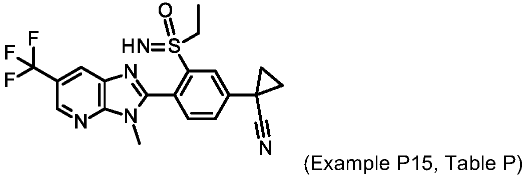

### Step 1: Synthesis of methyl 2-ethylsulfanyl-4-isoxazol-4-yl-benzoate

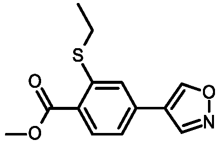

To a solution of methyl 4-bromo-2-ethylsulfanyl-benzoate (WO 2016/023954) (250 mg, 0.91 mmol) in dimethyl sulfoxide (8 mL) under argon were added water (4 mL), 4-isoxazoleboronic acid pinacol ester (213 mg, 1.09 mmol) and potassium fluoride (158 mg, 2.73 mmol). The thick reaction mixture was purged with argon for 5 minutes, then bis(triphenylphosphine)palladium(II) dichloride (6.4 mg, 0.009 mmol) was added. The vial was sealed and the mixture stirred in the microwave at 90°C for 40 minutes. The reaction mixture was poured onto iced-water, and the resulting yellowish suspension filtered and washed with cold water. This solid was dissolved in dichlormethane, the solution dried over sodium sulfate and reduced to dryness under vacuum to afford methyl 2-ethylsulfanyl-4-isoxazol-4-yl-benzoate as a yellowish solid. This material was used in the next step without further purification.

LCMS (method 1): 262 (M-H)⁻, retention time 0.88 min.

### Step 2:Synthesis of methyl 4-(cyanomethyl)-2-ethylsulfanyl-benzoate

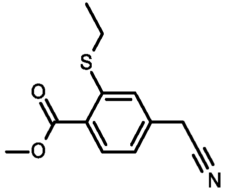

To a solution of methyl 2-ethylsulfanyl-4-isoxazol-4-yl-benzoate (760 mg, 2.89 mmol) in methanol (15 mL) was added a 1M potassium fluoride solution in water (8.66 mL, 8.66 mmol). The reaction mixture was stirred at reflux for 3 hours. After cooling, the suspension was filtered and the filtrate concentrated *in vacuo.* The residue was purified by Combiflash over silicagel to afford methyl 4-(cyano-methyl)-2-ethylsulfanyl-benzoate as a gum.

LCMS (method 5): 236 (M+H)⁺, retention time 0.90 min.

¹H NMR (400 MHz, CDCl₃) δ ppm 1.42 (t, 3H), 2.99 (q, 2H), 3.80 (s, 2H), 3.93 (s, 3H), 7.10 (dd, 1H), 7.28 (d, 1H), 7.99 (d, 1H).

### Step 3:Synthesis of methyl 4-(1-cyanocyclopropyl)-2-ethylsulfanyl-benzoate

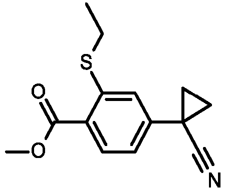

To a solution of methyl 4-(cyano-methyl)-2-ethylsulfanyl-benzoate (300 mg, 1.275 mmol) in acetonitrile (15 mL) were added cesium carbonate (1.24 g, 3.825 mmol) and 1,2-dibromoethane (719 mg, 3.825 mmol). The reaction mixture was stirred at reflux for 90 minutes. After cooling, the suspension was filtered and the filtrate concentrated *in vacuo.* The residue was purified by Combiflash over silicagel to afford methyl 4-(cyano-methyl)-2-ethylsulfanyl-benzoate as an oil.

LCMS (method 1): 262 (M+H)⁺, retention time 0.98 min. ¹H NMR (400 MHz, CDCl₃) δ ppm 1.43 (t, 3H), 1.48 (m, 2H), 1.82 (m, 2H), 3.01 (q, 2H), 3.92 (s, 3H), 6.88 (dd, 1H), 7.35 (d, 1H), 7.94 (d, 1H).

### Step 4: Synthesis of 4-(1-cyanocyclopropyl)-2-ethylsulfanyl-benzoic acid

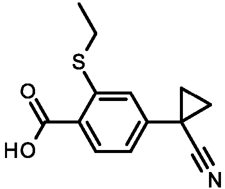

To a solution of methyl 4-(1-cyanocyclopropyl)-2-ethylsulfanyl-benzoate (198 mg, 0.758 mmol) in a mixture of tetrahydrofurane (9 mL) and water (3 mL) at 0-5°C was added lithium hydroxide (1.5 eq., 1.137 mmol) and the reaction mixture was stirred at room temperature overnight. The solution was concentrated *in vacuo,* the residue diluted with t-butyl methyl ether (10 mL) and acidified with a 1M aqueous hydrochloric acid solution (10 mL). The organic layer was separated, the aqueous layer extracted with t-butyl methyl ether, the combined organic layers washed with water and brine, dried over sodium sulfate, filtered and concentrated *in vacuo* to afford 4-(1-cyanocyclopropyl)-2-ethylsulfanyl-benzoic acid as a solid. This material was used in the next step without further purification.

LCMS (method 5): 246 (M-H)⁻, retention time 0.83 min. ¹H NMR (400 MHz, CDCl₃) δ ppm 1.44 (t, 3H), 1.51 (m, 2H), 1.85 (m, 2H), 3.03 (q, 2H), 6.90 (dd, 1H), 7.41 (d, 1H), 8.10 (d, 1H).

### Step 5 Preparation of 1-[3-ethylsulfanyl-4-[3-methyl-6-(trifluoromethyl)imidazo[4,5-b]pyridin-2-yl]phenyl]cyclopropanecarbonitrile

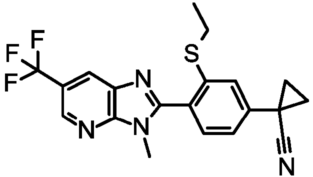

4-(1-cyanocyclopropyl)-2-ethylsulfanyl-benzoic acid (1.7 g, 6.9 mmol) was dissolved in nitrobenzene (14 mL) and N2-methyl-5-(trifluoromethyl)pyridine-2,3-diamine (WO 2017/043342) (1.6 g, 1.2 equiv) was added follow by the slow addition of phosphoryl chloride (1.6 mL, 17 mmol) at room temperature. The resulting solution was heated at 120 °C for 7 hours monitored by TLC and LC-MS. Reaction mass was quenched with 30% sodium hydroxide solution and water (100 mL) was added. The aqueous layer was extracted with ethyl acetate (3x 100 ml). The combined organic layer was dried over sodium sulfate, filtered and concentrated *in vacuo.* The crude product was purified by chromatography to afford1-[3-ethylsulfanyl-4-[3-methyl-6-(trifluoromethyl)imidazo[4,5-b]pyridin-2-yl]phenyl]cyclopropanecarbonitrile. LCMS (method 2): 403 (M+H)⁺; retention time: 1.18 min.

¹H NMR (400 MHz, CHLOROFORM-d) d ppm 1.26 (t, J=7.34 Hz, 3 H) 1.44 - 1.61 (m, 2 H) 1.80 - 1.92 (m, 2 H) 2.91 (q, J=7.42 Hz, 2 H) 3.78 (s,3 H) 7.16 (dd, J=8.01, 1.77 Hz, 1 H) 7.44 (d, J=7.60 Hz, 1 H) 7.50 (s, 1 H) 8.33 (s, 1 H) 8.72 (s, 1 H).

### Step 6: Preparation of of 1-[3-(ethylsulfonimidoyl)-4-[3-methyl-6-(trifluoromethyl)imidazo[4,5-b]pyridin-2-yl]phenyl]cyclopropanecarbonitrile (Example P15, Table P) - reference example

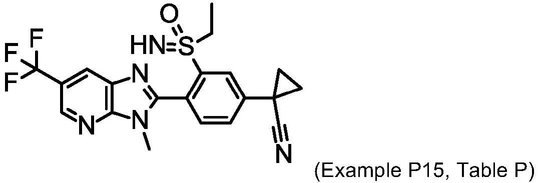

The desired product was prepared using the standard procedure described in example H1, step 7. LCMS (method 1): 434 (M+H)⁺; retention time: 0.88 min.

1H NMR (400 MHz, Chloroform) δ ppm 1.28 (t, J=7.34 Hz, 4 H) 1.63 - 1.68 (m, 2 H) 1.96 - 2.01 (m, 2 H) 3.41 - 3.62 (m, 2 H) 3.75 (s, 3 H) 7.56 (d, J=8.07 Hz, 1 H) 7.89 (dd, J=7.89, 2.02 Hz, 1 H) 8.05 (d, J=1.83 Hz, 1 H) 8.10 (d, J=0.73 Hz, 1 H) 8.94 - 8.97 (m, 1 H).

### Example H6: Synthesis of [5-cyclopropyl-2-[7-(trifluoromethyl)imidazo[1,2-b]pyridazin-2-yl]-3-pyridyl]-ethyl-imino-oxo-λ⁶-sulfane (Example P16, Table P).

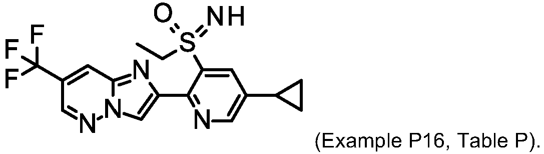

### Step 1.Preparation of 2-bromo-1-(5-bromo-3-ethylsulfanyl-2-pyridyl)ethanone

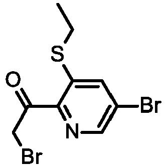

A sample of 1-(5-bromo-3-ethylsulfanyl-2-pyridyl)ethanone (preparation described in WO 2016/071214 (1 g, 3.8439 mmol) was suspended in acetonitrile (3 mL) and chloroform (3 mL). Dibromocopper (1.7171 g, 7.6879 mmol) was added and the reaction mixture was heated to 70°C and stirred for 22 hours, after which time LC-MS showed reaction completion. The reaction mixture was filtered through celite and with dichloromethane. The filtrate was concentrated *in vacuo* and the residue obtained was purified by chromatography over silica gel to afford 2-bromo-1-(5-bromo-3-ethylsulfanyl-2-pyridyl)ethanone.

LCMS (method 1): retention time 1.10 min; 339/341 (M+H)⁺.

¹H NMR (400 MHz, DMSO) δ ppm 1.28 (t, J=7.34 Hz, 3 H) 3.06 (q, J=7.34 Hz, 2 H) 4.93 (s, 2 H) 8.11 (d, J=1.83 Hz, 1 H) 8.58 - 8.60 (m, 1 H).

### Step 2: Preparation of 2-(5-bromo-3-ethylsulfanyl-2-pyridyl)-7-(trifluoromethyl)imidazo[1,2-b]pyridazine

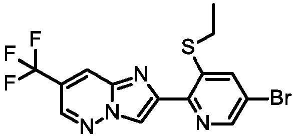

In a three neck flask fitted with reflux condenser, 2-bromo-1-(5-bromo-3-ethylsulfanyl-2-pyridyl)ethanone (0.50 g, 1.5 mmol) and 5-(trifluoromethyl)pyridazin-3-amine (CAS [1211591-88-6]) (0.27 g, 1.5 mmol) were suspended in acetonitrile (11 mL) and magnesium oxide (0.12 g, 2.9 mmol) was added. The resulting mixture was heated to 90°C and stirred overnight. The reaction mixture was filtered, and the filtrate was concentrated *in vacuo.* The resulting solid was dissolved in ethyl acetate and washed once with NaHCO3 sat. sol. The organic layer was pre-dried with brine, dried over sodium sulphate, filtered and concentrated *in vacuo.* The crude product was purified by chromatography over silica gel to afford 2-(5-bromo-3-ethylsulfanyl-2-pyridyl)-7-(trifluoromethyl)imidazo[1,2-b]pyridazine.

LCMS (method 1): retention time 1.15 min; 403/405 (M+H)⁺.

1H NMR (400 MHz, Chloroform) δ ppm 1.44 (t, J=7.34 Hz, 3 H) 3.04 (q, J=7.34 Hz, 2 H) 7.82 (d, J=1.83 Hz, 1 H) 8.33 - 8.39 (m, 1 H) 8.57 (dd, J=3.85, 2.02 Hz, 2 H) 8.81 - 8.86 (m, 1 H).

### Step 3: Preparation of 2-(5-cyclopropyl-3-ethylsulfanyl-2-pyridyl)-7-(trifluoromethyl)imidazo[1,2-b]pyridazine

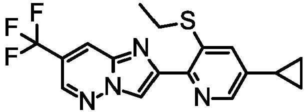

In a 100 ml-3-necked flask toluene (14 mL) and water (0.69 mL) was placed and flushed with argon 5 min. Under argon, 2-(5-bromo-3-ethylsulfanyl-2-pyridyl)-7-(trifluoromethyl)imidazo[1,2-b]pyridazine (0.30 g, 0.74 mmol), cyclopropylboronic acid (0.087 g, 0.97 mmol), tripotassium phosphate (0.58 g, 2.6 mmol), tricyclohexylphosphane (0.022 g, 0.074 mmol) and Palladium (II) acetate (0.0084 g, 0.050eq, 0.037 mmol) were added. The brown reaction mixture was heated up to 110°C and stirred one night. Then the mixture was cooled down at room temperature and water and ethyl acetate were added. The resulting mixture was filtered over celite and celite cake was washed by ethyl acetate. The organic layer was separated, dried over sodium sulfate, filtered and concentrated under vacuum. The crude was purified by chromatography over silica gel to afford 2-(5-cyclopropyl-3-ethylsulfanyl-2-pyridyl)-7-(trifluoromethyl)imidazo[1,2-b]pyridazine.

LCMS (method 1): retention time 1.08 min; 365 (M+H)⁺.

¹H NMR (400 MHz, Chloroform) δ ppm 0.81 - 0.86 (m, 2 H) 1.09 - 1.16 (m, 2 H) 1.39 (t, J=7.34 Hz, 3 H) 1.94 - 2.03 (m, 1 H) 3.00 (q, J=7.34 Hz, 2 H) 7.36 - 7.39 (m, 1 H) 8.33 (d, J=1.83 Hz, 1 H) 8.33 (s, 1 H) 8.54 (d, J=2.20 Hz, 1 H) 8.83 - 8.86 (m, 1 H).

### Step 4: Preparation of [5-cyclopropyl-2-[7-(trifluoromethylimidazo[1,2-b]pyridazin-2-yl]-3-pyridyl]-ethyl-imino-oxo-λ⁶-sulfane ((Example P16, Table P).)

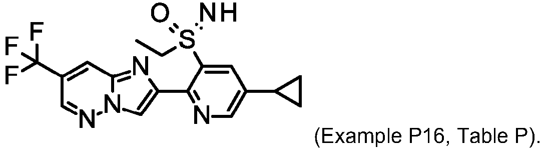

2-(5-cyclopropyl-3-ethylsulfanyl-2-pyridyl)-7-(trifluoromethyl)imidazo[1,2-b]pyridazine (0.07 g, 0.1921 mmol) was suspended in methanol (0.5763 mL) and Phl(OAc)2 (0.1894 g, 0.5763 mmol) and ammonium carbamate (0.03826 g, 0.4802 mmol) were added at room temperature .The reaction was stirred at room temperature and after 45 min, was quenched with iced water and sodium thiosulfate. The aqueous layer was extracted twice with acetate d'ethyl. The organic layers were combined washed with brine, dried over anhydrous sodium sulfate, filtered and concentrated under. The crude product was purified by chromatography over silica gel to afford [5-cyclopropyl-2-[7-(trifluoromethyl)imidazo[1,2-b]pyridazin-2-yl]-3-pyridyl]-ethyl-imino-oxo-λ⁶-sulfane.

LCMS (method 1): retention time 0.87 min; 396 (M+H)⁺.

1H NMR (400 MHz, Chloroform) δ ppm 0.89 - 0.95 (m, 2 H) 1.18 - 1.25 (m, 2 H) 1.36 (t, J=7.52 Hz, 3 H) 2.08 (tt, J=8.44, 5.14 Hz, 1 H) 3.21 - 3.37 (br s, 1 H) 3.71 - 3.98 (m, 2 H) 8.19 (d, J=2.20 Hz, 1 H) 8.26 - 8.29 (m, 1 H) 8.58 (d, J=2.20 Hz, 1 H) 8.64 (d, J=2.20 Hz, 1 H) 8.71 - 8.74 (m, 1 H).

### Example H7: Synthesis of [5-cyclopropyl-2-[6-(trifluoromethyl)pyrazolo[4,3-c]pyridin-2-yl]-3-pyridyl]-ethyl-imino-oxo- λ⁶-sulfane (Example P7, Table P)

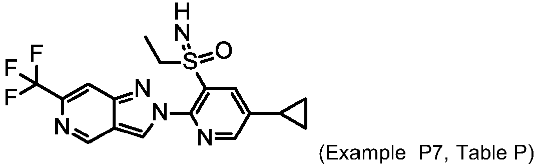

### Step 1: Preparation of 2,5-dibromo-3-ethylsulfanyl-pyridine

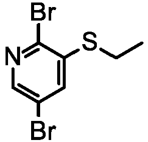

A solution of diethyldisulfide (7.76 g, 63.5 mmol, 2.00 equiv.) and tert-butyl nitrite (4.91 g, 47.6 mmol, 1.50 equiv.) in DCE (60 mL) and DCM (40 mL) was heated to 40 °C. To this mixture was slowly added a solution of 2,5-dibromopyridin-3-amine (8.00 g, 31.7 mmol, 1.00 equiv.) in dichloroethane (200 mL) slowly over 90 min and the reaction mixture was stirred for additional 1 h at 40 °C. After completion of the reaction, the reaction mass was cooled, diluted with water (100 mL), and extracted with dichloromethane (2 x 100 mL). The organic layer was separated, combined organic layers were dried over sodium sulfate, filtered and concentrated *in vacuo.* The crude product was purified by column chromatopgraphy (silica gel, 5-15% ethyl acetate/cyclohexane) to afford 2,5-dibromo-3-ethylsulfanyl-pyridine.

LCMS (method 4): 296 (M+H)⁺, retention time 1.16 min.

¹H NMR (400 MHz, CDCl₃) δ/ppm: 1.32 (t, 3 H), 2.98 (m, 2 H), 7.52 (s, 1 H) 8.19 (s, 1 H).

### Step 2: Preparation of (5-bromo-3-ethylsulfanyl-2-pyridyl)hydrazine

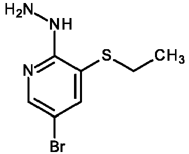

To a solution of 2,5-dibromo-3-ethylsulfanyl-pyridine (1 g, 3.3669 mmol) in1,4-dioxane (10.34 g) hydrazine monohydrate (1.0113 g, 20.201 mmol) was added and the resulting mixture was stirred at 120 °C for 10 hours. After completion of reaction, the mixture was diluted with water (30ml) and extracted with ethyl acetate. The combined organic layers were washed with water (20ml), dried with sodium sulfate, filtered and concentrated *in vacuo* to afford (5-bromo-3-ethylsulfanyl-2-pyridyl)hydrazine.

LCMS: 250 (M+H)⁺, retention time 0.6 min.

1H NMR (400 MHz, CHLOROFORM-d) d ppm 1.26 (t, 3 H) 2.81 (q, 2 H) 3.10 (br 2 H) 6.67 (br s, 1H) 7.65 (d, 1 H) 8.15 (d, 1 H).

### Step 3: Preparation of 4-[2-(5-bromo-3-ethylsulfanyl-2-pyridyl)hydrazinol-6-(trifluoro methyl)pyridine-3-carboxylic acid

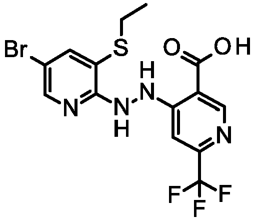

4-chloro-6-(trifluoromethyl)pyridine-3-carboxylic acid (CAS [1060810-66-3]) (20 g, 88.672 mmol), (5-bromo-3-ethylsulfanyl-2-pyridyl)hydrazine (33.005 g, 133.01 mmol) and pentan-1-ol (120 mL) were mixed and the mixture was stirred at 110 °C for 15 hours. After completion of reaction, the mixture was concentrated *in vacuo* to remove all of the pentanol. The residue obtained was co-evaporated with toluene. The crude product was diluted with water (100 ml), brine (100 ml) and extracted with ethyl acetate (3x 200 ml). The combined organic layer was dried over sodium sulfate, filtered and concentrated *in vacuo.* The solid obtained was triturated with cyclohexane to afford 4-[2-(5-bromo-3-ethylsulfanyl-2-pyridyl)hydrazino]-6-(trifluoromethyl)pyridine-3-carboxylic acid.

LCMS (method 5): retention time 1.50 min, 439 (M+2)⁺.

1H NMR (400 MHz, DMSO-d6) d ppm 1.26 (t, 3 H) 3.05 (q, 2 H) 7.16 (s, 1 H) 7.89 (d, 1 H) 8.11 (d, 1 H) 8.77 (s, 1 H) 8.88 (s, 1 H) 9.83 (s, 1 H) 13.38 - 14.36 (m, 1 H).

### Step 4: Preparation of 2-(5-bromo-3-ethylsulfanyl-2-pyridyl)-3-chloro-6-(trifluoromethyl)pyrazolo[4,3-c]pyridine

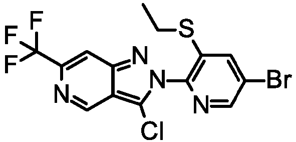

4-[2-(5-bromo-3-ethylsulfanyl-2-pyridyl)hydrazino]-6-(trifluoromethyl)pyridine-3-carboxylic acid (22.87 mmol, 10 g) was solved in phosphorus oxychloride (100 mL) and the resulting mixture was heated at 110 °C. The clear solution obtained at 110°C was refluxed for 50 min. After completion of reaction, the mixture was concentrated (phosphorus oxychloride distilled off under reduced pressure) and the reaction was diluted with dichloromethane (110 ml) poured into ice water (200 mL). The aqueous layer was extracted with dichlormethane(2x 100 ml). The combined organic layer was washed with water (200ml), dried over sodium sulfate, filtered and concentrated *in vacuo.* The crude product was purified by chromatography over silica gel to afford 2-(5-bromo-3-ethylsulfanyl-2-pyridyl)-3-chloro-6-(trifluoromethyl)pyrazolo[4,3-c]pyridine.

LCMS: 437 (M+H)⁺, retention time 1.21 min.

1H NMR (400 MHz, CHLOROFORM-d) d ppm 1.34 (m, 3 H) 2.85 (m, 2 H) 7.92 (S, 2 H) 8.02 (m, 1 H)8.60 (m, 1 H) 9.32 (m, 1 H).

### Step 5: Preparation of 2-(5-bromo-3-ethylsulfanyl-2-pyridyl)-6-(trifluoromethyl)pyrazolo[4,3-c]pyridine

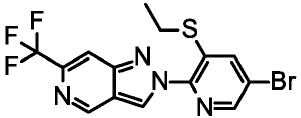

2-(5-bromo-3-ethylsulfanyl-2-pyridyl)-3-chloro-6-(trifluoromethyl)pyrazolo[4,3-c]pyridine (0.2 mmol, 0.1 g) was solved in acetic acid (2 mL) and zinc (0.5 mmol, 0.03 g) was added slowly to the mixture. The reaction mass was stirred at 55°C for 40 min. The reaction was monitored by LCMS & TLC. After completion, the reaction mixture was poured into water (30ml), and the resultant solution was extracted with ethyl acetate (20ml x 3). The combined organic layer was washed with aqueous saturated sodium chloride solution (30ml), dried over anhydrous sodium sulfate, filtered and concentrated *in vacuo.* The residue was subjected to silica gel column chromatography to afford 2-(5-bromo-3-ethylsulfanyl-2-pyridyl)-6-(trifluoromethyl)pyrazolo[4,3-c]pyridine.

H NMR (400 MHz, CHLOROFORM-d) d ppm 1.29 (m, 4 H) 2.95 (m, 2 H) 7.85 (m, 1 H) 8.35 (m, 1 H) 8.38 (d, 1 H) 8.95 (m, 1 H) 9.44 (m, 1 H).

LCMS: retention time 1.6 min, 403 (M+H)⁺.

### Step 6: Preparation of 2-(5-cyclopropyl-3-ethylsulfanyl-2-pyridyl)-6-(trifluoromethyl)pyrazolo[4,3-c]pyridine

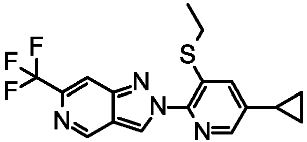

In a microwave vial were added 2-(5-bromo-3-ethylsulfanyl-2-pyridyl)-6-(trifluoromethyl)pyrazolo[4,3-c]pyridine (0.1 g, 0.2 mmol), cyclopropylboronic acid (0.03 g., 0.3 mmol), tripotassium phosphate (0.2 g, 0.9 mmol), tricyclohexylphosphane (0.007g, 0.02 mmol) in toluene (2 mL) and water (1 mL). The reaction mass was purged with nitrogen for 30 min. To this was then added palladium(II) acetate (0.003 g., 0.01 mmol) and the reaction mixture was stirred at 150 °C under microwave for 4 hours. Then at room temperature, reaction mixture was diluted with ethyl acetate (20 mL) and washed with water (20 mL). The organic layer was washed with water (20ml), dried organic layer over sodium sulfate, filtered and concentrated *in vacuo.* The compound was isolated by chromatography over silica gel to afford 2-(5-cyclopropyl-3-ethylsulfanyl-2-pyridyl)-6-(trifluoromethyl)pyrazolo[4,3-c]pyridine.

LCMS: 365 (M+H)⁺, retention time 1.12 min.

1H NMR (400 MHz, CHLOROFORM-d) d ppm 0.86 (m, 2 H) 1.13 (br , 2 H) 1.23 (m, 4 H) 2.90 (q, 2 H) 7.47 (d, 1 H) 8.13 (m, 2 H) 8.94 (s, 1 H) 9.40 (m, 1 H).

### Step 7: Synthesis of [5-cyclopropyl-2-[6-(trifluoromethyl)pyrazolo[4,3-c]pyridin-2-yl]-3-pyridyl]-ethyl-imino-oxo-λ⁶-sulfane Example P7, Table P)

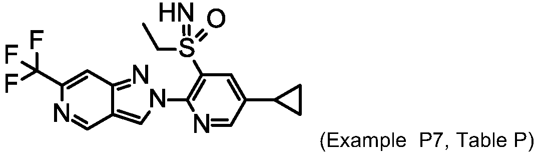

The desired product was prepared in using the standard method described in example H1, Step 7. LCMS (method 5): retention time 0.94 min, 396 (M+H)⁺.

1H NMR (400 MHz, CHLOROFORM-d) d ppm 1.25 (m, 4 H) 1.44 (m, 3 H) 2.16 (m, 1 H) 3.87(m, 2 H) 8.06 (s, 1 H) 8.29 (d, 1 H) 8.56 (d, 1 H) 8.87 (d, 1 H) 9.40(s, 1 H).

### Example H8: Synthesis of [5-cyclopropyl-2-[5-(trifluoromethylsulfanyl)-1,3-benzoxazol-2-yl]-3-pyridyl]-ethyl-imino-oxo- λ⁶-sulfane (Example P18, Table P) - reference example

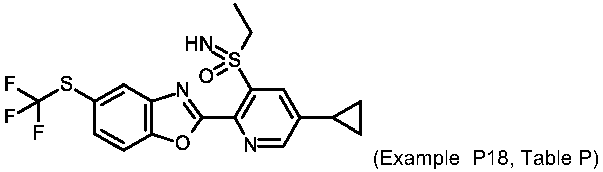

The desired product was prepared by using the standard method described in Example H1, Step 7 starting from 2-(5-cyclopropyl-3-ethylsulfanyl-2-pyridyl)-5-(trifluoromethylsulfanyl)-1,3-benzoxazole (known from WO19/009307).

LCMS (method 5): 428 (M+H)⁺, retention time: 1.03 min.

Further compounds of the invention can be prepared analogously to the method described above. Compounds P7, P8, P14, P16 and P17 prepared to further illustrate the invention are listed in Table P. Compounds P1 - P6, P9-P13, P15, P18 and P19 are reference examples.

| Entry | IUPAC name | **STRUCTURE** | RT (min) | [M+H] (meas ured) | Method | MP °C |
|---|---|---|---|---|---|---|
| P1 | 1-[5-(ethylsulfonimidoyl)-6-[7-methyl-3-(trifluoromethyl)imidazo[4,5-c]pyridazin-6-yl]-3-pyridyl]cyclopropanecarboxa mide | 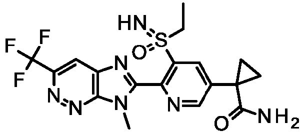 | 0.87 | 454 | 5 | 253 - 255 |
| P2 | 1-[3-(ethylsulfonimidoyl)-4-[3-methyl-6-(trifluoromethyl)imidazo[4,5-b]pyridin-2-yl]phenyl]cyclopropanecarbo xamide | 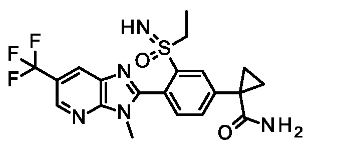 | 0.88 | 452 | 5 | 235 - 237 |
| P3 | [5-cyclopropyl-2-[3-methyl-6-(trifluoromethylsulfonyl)imida zo[4,5-c]pyridin-2-yl]-3-pyridyl]-ethyl-imino-oxo- λ⁶-sulfane | 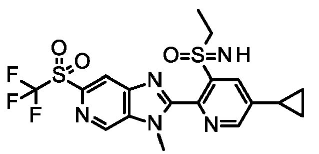 | 0.91 | 474 | 1 | - |
| P4 | [5-cyclopropyl-2-[3-methyl-6-(trifluoromethylsulfanyl)imida zo[4,5-c]pyridin-2-yl]-3-pyridyl]-ethyl-imino-oxo- λ⁶-sulfane | 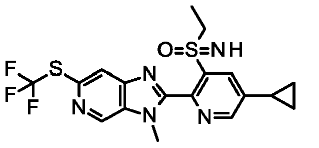 | 0.90 | 442 | 1 | - |
| P5 | [5-cyclopropyl-2-[3-methyl-6-(trifluoromethyl)imidazo[4,5-c]pyridin-2-yl]-3-pyridyl]-ethyl-imino-oxo-λ⁶-sulfane | 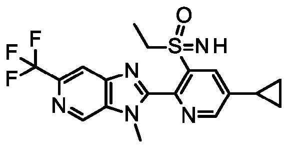 | 0.85 | 411 | 1 | - |
| P6 | 1-[5-(ethylsulfonimidoyl)-6-[3-methyl-6-(trifluoromethyl)imidazo[4,5-b]pyridin-2-yl]-3-pyridyl]cyclopropanecarbonitr ile | 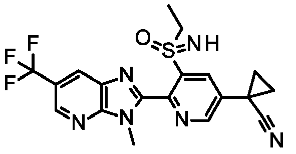 | 0.87 | 435 | 1 | - |
| P7 | [5-cyclopropyl-2-[6-(trifluoromethyl)pyrazolo[4,3-c]pyridin-2-yl]-3-pyridyl]-ethyl-imino-oxo-λ⁶-sulfane | 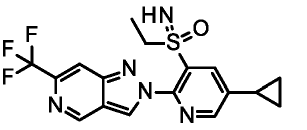 | 0.94 | 396 | 5 | 75 - 77 |
| P8 | 1-[5-(ethylsulfonimidoyl)-6-[5-methoxy-3-methyl-4-oxo-6-(trifluoromethyl)imidazo[4,5-c]pyridin-2-yl]-3-pyridyl]cyclopropanecarbonitr ile | 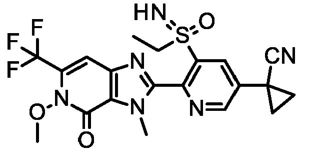 | 0.85 | 481 | 5 | 163 - 165 |
| P9 | 1-[3-(ethylsulfonimidoyl)-4-[3-methyl-6-(trifluoromethylsulfanyl)imida zo[4,5-c]pyridin-2-yl]phenyl]cyclopropanecarbo nitrile | 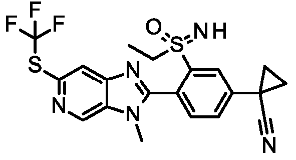 | 0.86 | 466 | 1 | 191 - 193 |
| P10 | 1-[5-(ethylsulfonimidoyl)-6-[7-methyl-3-(trifluoromethyl)imidazo[4,5-c]pyridazin-6-yl]-3-pyridyl]cyclopropanecarbonitr ile | 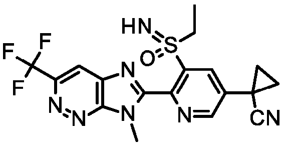 | 0.82 | 436 | 5 | 182 - 184 |
| P11 | [5-cyclopropyl-2-[7-methyl-3-(trifluoromethyl)imidazo[4,5-c]pyridazin-6-yl]-3-pyridyl]-ethyl-imino-oxo-λ⁶-sulfane | 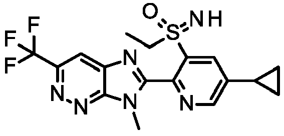 | 0.84 | 411 | 1 | - |
| P12 | [5-cyclopropyl-2-[3-methyl-6-(trifluoromethyl)imidazo[4,5-b]pyridin-2-yl]-3-pyridyl]-ethyl-imino-oxo-λ⁶-sulfane | 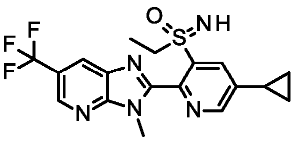 | 0.89 | 410 | 1 | - |
| P13 | 1-[3-(ethylsulfonimidoyl)-4-[3-methyl-6-(trifluoromethyl)imidazo[4,5-c]pyridin-2-yl]phenyl]cyclopropanecarbo nitrile | 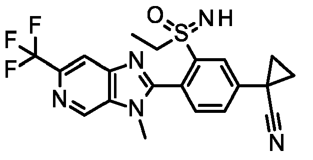 | 0.83 | 434 | 1 | - |
| P14 | 1-[6-[5-ethyl-3-methyl-4-oxo-6-(trifluoromethyl)imidazo[4,5-c]pyridin-2-yl]-5-(ethylsulfonimidoyl)-3-pyridyl]cyclopropanecarbonitr ile | 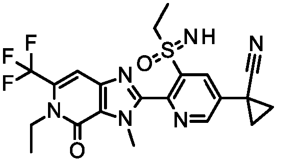 | 0.92 | 479 | 5 | 176 - 178 |
| P15 | 1-[3-(ethylsulfonimidoyl)-4-[3-methyl-6-(trifluoromethyl)imidazo[4,5-b]pyridin-2-yl]phenyl]cyclopropanecarbo nitrile | 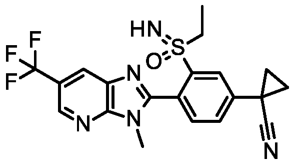 | 0.88 | 434 | 1 | 117 - 117 |
| P16 | [5-cyclopropyl-2-[7-(trifluoromethyl)imidazo[1,2-b]pyridazin-2-yl]-3-pyridyl]-ethyl-imino-oxo-λ⁶-sulfane | 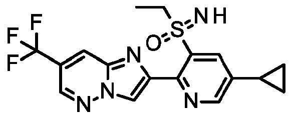 | 0.87 | 396 | 1 | 119-121 |
| P17 | 1-[5-(ethylsulfonimidoyl)-6-[6-(trifluoromethyl)pyrazolo[4,3-c]pyridin-2-yl]-3-pyridyl]cyclopropanecarbonitr ile | 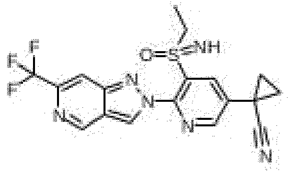 | 0.91 | 421 | 5 | 170 - 172 |
| P18 | [5-cyclopropyl-2-[5-(trifluoromethylsulfanyl)-1,3-benzoxazol-2-yl]-3-pyridyl]-ethyl-imino-oxo-λ⁶-sulfane | 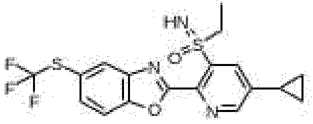 | 1.03 | 428 | 5 | 224 - 226 |
| P19 | [5-cyclopropyl-2-[5-(trifluoromethylsulfonyl)-1,3-benzoxazol-2-yl]-3-pyridyl]-ethyl-imino-oxo-λ⁶-sulfane | 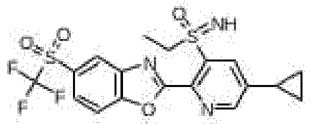 | 1.10 | 460 | 5 | - |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds of formula I with active ingredients are preferred (the abbreviation "TX" means "one compound selected from the group consisting of the compounds described in Tables Y-1 to Y-8, X-1 to X-8, U-1 to U-2 and V-1 to V-6 and Table P of the present invention"):
an adjuvant selected from the group of substances consisting of petroleum oils (alternative name) (628) + TX,
an acaricide selected from the group of substances consisting of 1,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dimpropyridaz + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (alternative name) (473) + TX, isopropyl O-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
a biological agent selected from the group of substances consisting of *Adoxophyes orana GV* (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and H. *megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinemema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX, an insect pheromone selected from the group of substances consisting of (*E*)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (*E,Z*)-tetradeca-4,10-dien-1-yl acetate (IUPAC name) (779) + TX, (*Z*)-dodec-7-en-1-yl acetate (IUPAC name) (285) + TX, (*Z*)-hexadec-11-enal (IUPAC name) (436) + TX, (Z)-hexadec-11-en-1-yl acetate (IUPAC name) (437) + TX, (*Z*)-hexadec-13-en-11-yn-1-yl acetate (IUPAC name) (438) + TX, (*Z*)-icos-13-en-10-one (IUPAC name) (448) + TX, (*Z*)-tetradec-7-en-1-al (IUPAC name) (782) + TX, (*Z*)-tetradec-9-en-1-ol (IUPAC name) (783) + TX, (*Z*)-tetradec-9-en-1-yl acetate (IUPAC name) (784) + TX, (7*E*,9*Z*)-dodeca-7,9-dien-1-yl acetate (IUPAC name) (283) + TX, (9*Z*,11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin S-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, *d*-limonene (alternative name) [CCN] + TX, d-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl O-(methoxy-aminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O-*5-dichloro-4-iodophenyl *O*-ethyl ethylphosphonothioate (IUPAC name) (1057) + TX, *O,O*-diethyl *O*-4-methyl-2-oxo-2H-chromen-7-yl phosphorothioate (IUPAC name) (1074) + TX, *O,O-*diethyl *O*-6-methyl-2-propylpyrimidin-4-yl phosphorothioate (IUPAC name) (1075) + TX, *O*,*O*,*O*',*O*'-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX, fluxametamide (WO 2007/026965) + TX, epsilon-metofluthrin [240494-71-7] + TX, epsilon-momfluorothrin [1065124-65-3] + TX, fluazaindolizine [1254304-22-7] + TX, chloroprallethrin [399572-87-3] + TX, fluxametamide [928783-29-3] + TX, cyhalodiamide [1262605-53-7] + TX, tioxazafen [330459-31-9] + TX, broflanilide [1207727-04-5] + TX, flufiprole [704886-18-0] + TX, cyclaniliprole [1031756-98-5] + TX, tetraniliprole [1229654-66-3] + TX, guadipyr (described in WO2010/060231) + TX, cycloxaprid (described in WO 2005/077934) + TX, spiropidion + TX, Afidopyropen + TX, flupyrimin + TX, Momfluorothrin + TX, kappa-bifenthrin + TX, kappa-tefluthrin + TX, Dichloromezotiaz + TX, Tetrachloraniliprole + TX, benzpyrimoxan + TX;
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX,
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (alternative name) (720) + TX,
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin (alternative name) [CCN] and ribavirin (alternative name) [CCN] + TX,
a wound protectant selected from the group of substances consisting of mercuric oxide (512) + TX, octhilinone (590) and thiophanate-methyl (802) + TX,
and biologically active compounds selected from the group of substances consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxicon-azole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebucon-azole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3 ] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil *[1897-45-6]* + TX, cyflufenamid *[180409-60-3]* + TX, cymoxanil *[57966-95-7]* + TX, dichlone *[117-80-6]* + TX, diclocymet [139920-32-4] + TX, diclomezine *[62865-36-5]* + TX, dicloran *[99-30-9]* + TX, diethofencarb *[87130-20-9]* + TX, dimethomorph *[110488-70-5] +* TX, SYP-LI90 (Flumorph) *[211867-47-9]* + TX, dithianon *[3347-22-6]* + TX, ethaboxam *[162650-77-3] +* TX, etridiazole *[2593-15-9]* + TX, famoxadone *[131807-57-3] + TX,* fenamidone *[161326-34-7]* + TX, fenoxanil *[115852-48-7] +* TX, fentin *[668-34-8]* + TX, ferimzone *[89269-64-7]* + TX, fluazinam *[79622-59-6]* + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[120116-88-3]* + TX, kasugamycin *[6980-18-3]* + TX, methasulfocarb *[66952-49-6]* + TX, metrafenone *[220899-03-6] +* TX, pencycuron *[66063-05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulfur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1,2,3,4-tetrahydro-1,4-methano-naphthalen-5-yl)-amide (disclosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3*S*,4*R*,4a*R*,6*S*,6a*S*,12*R*,12a*S*,12b*S*)-3-[(cyclopropylcarbonyl)oxy]- 1,3,4,4a,5,6,6a,12,12a,12b-decahydro-6,12-dihydroxy-4,6a,12b-trimethyl-11-oxo-9-(3-pyridinyl)-2*H*,11*H*naphtho[2,1-*b*]pyrano[3,4-e]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX, lancotrione [1486617-21-3] + TX, florpyrauxifen [943832-81-3] + TX, ipfentrifluconazole[1417782-08-1] + TX, mefentrifluconazole [1417782-03-6] + TX, quinofumelin [861647-84-9] + TX, chloroprallethrin [399572-87-3] + TX, cyhalodiamide [1262605-53-7] + TX, fluazaindolizine [1254304-22-7] + TX, fluxametamide [928783-29-3] + TX, epsilon-metofluthrin [240494-71-7] + TX, epsilon-momfluorothrin [1065124-65-3] + TX, pydiflumetofen [1228284-64-7] + TX, kappa-bifenthrin [439680-76-9] + TX, broflanilide [1207727-04-5] + TX, dicloromezotiaz [1263629-39-5] + TX, dipymetitrone [16114-35-5] + TX, pyraziflumid [942515-63-1] + TX and kappa-tefluthrin [391634-71-2] + TX, fenpicoxamid [517875-34-2] + TX; flufenpyrrolidone + TX, benzpyrimoxan [1449021-97-9] + TX; isocycloseram + TX, rescalure [64309-03-1] + TX; aminopyrifen [1531626-08-0] + TX; and
microbials including: *Acinetobacter Iwoffii +* TX, *Acremonium alternatum* + TX + TX, *Acremonium cephalosporium* + TX + TX, *Acremonium diospyri* + TX, *Acremonium obclavatum* + TX, *Adoxophyes orana granulovirus* (AdoxGV) (Capex^{®}) + TX, *Agrobacterium radiobacter* strain K84 (Galltrol-A^{®}) + TX, *Alternaria alternate* + TX, *Alternaria cassia* + TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans + TX, Azospirillum* + TX, (MicroAZ^{®} + TX, TAZO B^{®}) + TX, *Azotobacter +* TX, *Azotobacter chroocuccum* (Azotomeal^{®}) + TX, *Azotobacter* cysts (Bionatural Blooming Blossoms^{®}) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans* + TX, *Bacillus firmus* (BioSafe^{®} + TX, BioNem-WP^{®} + TX, VOTiVO^{®}) + TX, *Bacillus firmus* strain I-1582 + TX, *Bacillus macerans +* TX, *Bacillus marismortui* + TX, *Bacillus megaterium* + TX, *Bacillus mycoides* strain AQ726 + TX, *Bacillus papillae* (Milky Spore Powder^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ^{®}) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* Cry1Ab + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPel^{®}) + TX, bacteria spp. (GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis +* TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii* + TX, *Burkholderia gladioli +* TX, *Burkholderia* spp. + TX, Canadian thistle fungus (CBH Canadian Bioherbicide^{®}) + TX, *Candida butyri +* TX, *Candida famata +* TX, *Candida fructus +* TX, *Candida glabrata* + TX, *Candida guilliermondii +* TX, *Candida melibiosica* + TX, *Candida oleophila* strain O + TX, *Candida parapsilosis +* TX, *Candida pelliculosa* + TX, *Candida pulcherrima* + TX, *Candida reukaufii +* TX, *Candida saitoana* (Bio-Coat^{®} + TX, Biocure^{®}) + TX, *Candida sake* + TX, *Candida* spp. + TX, *Candida tenius +* TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides +* TX, *Cladosporium oxysporum* + TX, *Cladosporium chlorocephalum +* TX, *Cladosporium* spp. + TX, *Cladosporium tenuissimum* + TX, *Clonostachys rosea* (EndoFine^{®}) + TX, *Colletotrichum acutatum* + TX, *Coniothyrium minitans* (Cotans WG^{®}) + TX, *Coniothyrium* spp. + TX, *Cryptococcus albidus* (YIELDPLUS^{®}) + TX, *Cryptococcus humicola +* TX, *Cryptococcus infirmo-miniatus +* TX, *Cryptococcus laurentii +* TX, *Cryptophlebia leucotreta granulovirus* (Cryptex^{®}) + TX, *Cupriavidus campinensis +* TX, *Cydia pomonella granulovirus* (CYD-X^{®}) + TX, *Cydia pomonella granulovirus* (Madex^{®} + TX, Madex Plus^{®} + TX, Madex Max/ Carpovirusine^{®}) + TX, *Cylindrobasidium laeve* (Stumpout^{®}) + TX, *Cylindrocladium* + TX, *Debaryomyces hansenii +* TX, *Drechslera hawaiinensis* + TX, *Enterobacter cloacae* + TX, *Enterobacteriaceae* + TX, *Entomophtora virulenta* (Vektor^{®}) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean^{®} / Biofox C^{®}) + TX, *Fusarium proliferatum* + TX, *Fusarium* spp. + TX, *Galactomyces geotrichum* + TX, *Gliocladium catenulatum* (Primastop^{®} + TX, Prestop^{®}) + TX, *Gliocladium roseum* + TX, *Gliocladium* spp. (SoilGard^{®}) + TX, *Gliocladium virens* (Soilgard^{®}) + TX, *Granulovirus* (Granupom^{®}) + TX, *Halobacillus halophilus* + TX, *Halobacillus litoralis +* TX, *Halobacillus trueperi +* TX, *Halomonas* spp. + TX, *Halomonas subglaciescola* + TX, *Halovibrio variabilis +* TX, *Hanseniaspora uvarum* + TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata* + TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus +* TX, *Meira geulakonigii +* TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima* + TX, *Microdochium dimerum* (Antibot^{®}) + TX, *Micromonospora coerulea* + TX, *Microsphaeropsis ochracea* + TX, *Muscodor albus* 620 (Muscudor^{®}) + TX, *Muscodor roseus* strain A3-5 + TX, *Mycorrhizae* spp. (AMykor^{®} + TX, Root Maximizer^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus +* TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa +* TX, *Pantoea agglomerans* (BlightBan C9-1^{®}) + TX, *Pantoea* spp. + TX, *Pasteuria* spp. (Econem^{®}) + TX, *Pasteuria nishizawae* + TX, *Penicillium aurantiogriseum* + TX, *Penicillium billai* (Jumpstart^{®} + TX, TagTeam^{®}) + TX, *Penicillium brevicompactum* + TX, *Penicillium frequentans +* TX, *Penicillium griseofulvum* + TX, *Penicillium purpurogenum* + TX, *Penicillium* spp. + TX, *Penicillium viridicatum* + TX, *Phlebiopsis gigantean* (Rotstop^{®}) + TX, phosphate solubilizing bacteria (Phosphomeal^{®}) + TX, *Phytophthora cryptogea* + TX, *Phytophthora palmivora* (Devine^{®}) + TX, *Pichia anomala* + TX, *Pichia guilermondii +* TX, *Pichia membranaefaciens* + TX, *Pichia onychis +* TX, *Pichia stipites +* TX, *Pseudomonas aeruginosa* + TX, *Pseudomonas aureofasciens* (Spot-Less Biofungicide^{®}) + TX, *Pseudomonas cepacia* + TX, *Pseudomonas chlororaphis* (AtEze^{®}) + TX, *Pseudomonas corrugate +* TX, *Pseudomonas fluorescens* strain A506 (BlightBan A506^{®}) + TX, *Pseudomonas putida* + TX, *Pseudomonas reactans +* TX, *Pseudomonas* spp. + TX, *Pseudomonas syringae* (Bio-Save^{®}) + TX, *Pseudomonas viridiflava* + TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum +* TX, *Pythium oligandrum* (Polygandron^{®} + TX, Polyversum^{®}) + TX, *Pythium periplocum +* TX, *Rhanella aquatilis +* TX, *Rhanella* spp. + TX, *Rhizobia* (Dormal^{®} + TX, Vault^{®}) + TX, *Rhizoctonia +* TX, *Rhodococcus globerulus* strain AQ719 + TX, *Rhodosporidium diobovatum* + TX, *Rhodosporidium toruloides +* TX, *Rhodotorula* spp. + TX, *Rhodotorula glutinis +* TX, *Rhodotorula graminis +* TX, *Rhodotorula mucilagnosa +* TX, *Rhodotorula rubra +* TX, *Saccharomyces cerevisiae* + TX, *Salinococcus roseus* + TX, *Sclerotinia minor +* TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens* + TX, *Serratia plymuthica* + TX, *Serratia* spp. + TX, *Sordaria fimicola +* TX, *Spodoptera littoralis nucleopolyhedrovirus* (Littovir^{®}) + TX, *Sporobolomyces roseus* + TX, *Stenotrophomonas maltophilia* + TX, *Streptomyces ahygroscopicus +* TX, *Streptomyces albaduncus +* TX, *Streptomyces exfoliates +* TX, *Streptomyces galbus +* TX, *Streptomyces griseoplanus +* TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus +* TX, *Tilletiopsis minor +* TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycostar^{®}) + TX, *Trichoderma harzianum* T-22 (Trianum-P^{®} + TX, PlantShield HC^{®} + TX, RootShield^{®} + TX, Trianum-G^{®}) + TX, *Trichoderma harzianum* T-39 (Trichodex^{®}) + TX, *Trichoderma inhamatum* + TX, *Trichoderma koningii +* TX, *Trichoderma* spp. LC 52 (Sentinel^{®}) + TX, *Trichoderma lignorum* + TX, *Trichoderma longibrachiatum* + TX, *Trichoderma polysporum* (Binab T^{®}) + TX, *Trichoderma taxi +* TX, *Trichoderma virens* + TX, *Trichoderma virens* (formerly Gliocladium virens GL-21) (SoilGuard^{®}) + TX, *Trichoderma viride* + TX, *Trichoderma viride* strain ICC 080 (Remedier^{®}) + TX, *Trichosporon pullulans +* TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen^{®}) + TX, *Ustilago maydis +* TX, various bacteria and supplementary micronutrients (Natural II^{®}) + TX, various fungi (Millennium Microbes^{®}) + TX, *Verticillium chlamydosporium* + TX, *Verticillium lecanii* (Mycotal^{®} + TX, Vertalec^{®}) + TX, Vip3Aa20 (VIPtera^{®}) + TX, *Virgibaclillus marismortui +* TX, *Xanthomonas campestris pv. Poae* (Camperico^{®}) + TX, *Xenorhabdus bovienii* + TX, *Xenorhabdus nematophilus;* and
Plant extracts including: pine oil (Retenol^{®}) + TX, azadirachtin (Plasma Neem Oil^{®} + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina* + TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNIQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®}); and
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromone^{®}) + TX, Peachtree Borer Pheromone (Isomate-P^{®}) + TX, Tomato Pinworm Pheromone (3M Sprayable pheromone^{®}) + TX, Entostat powder (extract from palm tree) (Exosex CM^{®}) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate; and
Macrobials including: *Aphelinus abdominalis +* TX, *Aphidius ervi* (Aphelinus-System^{®}) + TX, *Acerophagus papaya* + TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola* + TX, *Ageniaspis fuscicollis +* TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum* + TX, *Anagrus atomus +* TX, *Anagyrus fusciventris* + TX, *Anagyrus kamali* + TX, *Anagyrus loecki* + TX, *Anagyrus pseudococci* (Citripar^{®}) + TX, *Anicetus benefices* + TX, *Anisopteromalus calandrae* + TX, *Anthocoris nemoralis* (Anthocoris-System^{®}) + TX, *Aphelinus abdominalis* (Apheline^{®} + TX, Aphiline^{®}) + TX, *Aphelinus asychis +* TX, *Aphidius colemani* (Aphipar^{®}) + TX, *Aphidius ervi* (Ervipar^{®}) + TX, *Aphidius gifuensis +* TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis +* TX, *Aphytis melinus +* TX, *Aprostocetus hagenowii +* TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis +* TX, *Chilocorus nigritus* + TX, *Chrysoperla carnea* (Chrysoline^{®}) + TX, *Chrysoperla carnea* (Chrysopa^{®}) + TX, *Chrysoperla rufilabris +* TX, *Cirrospilus ingenuus +* TX, *Cirrospilus quadristriatus +* TX, *Citrostichus phyllocnistoides* + TX, *Closterocerus chamaeleon* + TX, *Closterocerus* spp. + TX, *Coccidoxenoides perminutus* (Planopar^{®}) + TX, *Coccophagus cowperi +* TX, *Coccophagus lycimnia* + TX, *Cotesia flavipes +* TX, *Cotesia plutellae* + TX, *Cryptolaemus montrouzieri* (Cryptobug^{®} + TX, Cryptoline^{®}) + TX, *Cybocephalus nipponicus +* TX, *Dacnusa sibirica* + TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus +* TX, *Diachasmimorpha krausii +* TX, *Diachasmimorpha longicaudata* + TX, *Diaparsis jucunda* + TX, *Diaphorencyrtus aligarhensis +* TX, *Diglyphus isaea* + TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae* + TX, *Encarsia haitiensis* + TX, *Episyrphus balteatus* (Syrphidend^{®}) + TX, *Eretmoceris siphonini +* TX, *Eretmocerus californicus +* TX, *Eretmocerus eremicus* (Ercal^{®} + TX, Eretline e^{®}) + TX, *Eretmocerus eremicus* (Bemimix^{®}) + TX, *Eretmocerus hayati +* TX, *Eretmocerus mundus* (Bemipar^{®} + TX, Eretline m^{®}) + TX, *Eretmocerus siphonini* + TX, *Exochomus quadripustulatus* + TX, *Feltiella acarisuga* (Spidend^{®}) + TX, *Feltiella acarisuga* (Feltiline^{®}) + TX, *Fopius arisanus +* TX, *Fopius ceratifivorus +* TX, Formononetin (Wirless Beehome^{®}) + TX, *Franklinothrips vespiformis* (Vespop^{®}) + TX, *Galendromus occidentalis +* TX, *Goniozus legneri* + TX, *Habrobracon hebetor +* TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens* + TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides* + TX, *Lecanoideus floccissimus +* TX, *Lemophagus errabundus +* TX, *Leptomastidea abnormis +* TX, *Leptomastix dactylopii* (Leptopar^{®}) + TX, *Leptomastix epona* + TX, *Lindorus lophanthae* + TX, *Lipolexis oregmae* + TX, *Lucilia caesar* (Natufly^{®}) + TX, *Lysiphlebus testaceipes* + TX, *Macrolophus caliginosus* (Mirical-N^{®} + TX, Macroline c^{®} + TX, Mirical^{®}) + TX, *Mesoseiulus longipes +* TX, *Metaphycus flavus* + TX, *Metaphycus lounsburyi* + TX, *Micromus angulatus* (Milacewing^{®}) + TX, *Microterys flavus +* TX, *Muscidifurax raptorellus* and *Spalangia cameroni* (Biopar^{®}) + TX, *Neodryinus typhlocybae* + TX, *Neoseiulus californicus +* TX, *Neoseiulus cucumeris* (THRYPEX^{®}) + TX, *Neoseiulus fallacis +* TX, *Nesideocoris tenuis* (NesidioBug^{®} + TX, Nesibug^{®}) + TX, *Ophyra aenescens* (Biofly^{®}) + TX, *Orius insidiosus* (Thripor-I^{®} + TX, Oriline i^{®}) + TX, *Orius laevigatus* (Thripor-L^{®} + TX, Oriline 10) + TX, *Orius majusculus* (Oriline m^{®}) + TX, *Orius strigicollis* (Thripor-S^{®}) + TX, *Pauesia juniperorum* + TX, *Pediobius foveolatus* + TX, *Phasmarhabditis hermaphrodita* (Nemaslug^{®}) + TX, *Phymastichus coffea* + TX, *Phytoseiulus macropilus* + TX, *Phytoseiulus persimilis* (Spidex^{®} + TX, Phytoline p^{®}) + TX, *Podisus maculiventris* (Podisus^{®}) + TX, *Pseudacteon curvatus +* TX, *Pseudacteon obtusus +* TX, *Pseudacteon tricuspis +* TX, *Pseudaphycus maculipennis* + TX, *Pseudleptomastix mexicana +* TX, *Psyllaephagus pilosus +* TX, *Psyttalia concolor* (complex) + TX, *Quadrastichus* spp. + TX, *Rhyzobius lophanthae* + TX, *Rodolia cardinalis +* TX, *Rumina decollate* + TX, *Semielacher petiolatus* + TX, *Sitobion avenae* (Ervibank^{®}) + TX, *Steinemema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinemema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline sf^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinemema* spp. + TX, *Steinernematid* spp. (Guardian Nematodes^{®}) + TX, *Stethorus punctillum* (Stethorus^{®}) + TX, *Tamarixia radiate* + TX, *Tetrastichus setifer +* TX, *Thripobius semiluteus* + TX, *Torymus sinensis +* TX, *Trichogramma brassicae* (Tricholine b^{®}) + TX, *Trichogramma brassicae* (Tricho-Strip^{®}) + TX, *Trichogramma evanescens* + TX, *Trichogramma minutum* + TX, *Trichogramma ostriniae* + TX, *Trichogramma platneri +* TX, *Trichogramma pretiosum* + TX, *Xanthopimpla stemmator;* and
other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae* + TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo^{®}) + TX and Traps (Takitrapline y + b^{®}) + TX;
or a biologically active compound or agent selected from: Brofluthrinate + TX, Diflovidazine + TX, Flometoquin + TX, Fluhexafon + TX, Plutella xylostella Granulosis virus + TX, Cydia pomonella Granulosis virus + TX, Imicyafos + TX, Heliothis virescens Nucleopolyhedrovirus + TX, Heliothis punctigera Nucleopolyhedrovirus + TX, Helicoverpa zea Nucleopolyhedrovirus + TX, Spodoptera frugiperda Nucleopolyhedrovirus + TX, Plutella xylostella Nucleopolyhedrovirus + TX, p-cymene + TX, Pyflubumide + TX, Pyrafluprole + TX, QRD 420 + TX, QRD 452 + TX, QRD 460 + TX, Terpenoid blends + TX, Terpenoids + TX, Tetraniliprole + TX, and α-terpinene + TX;
or an active substance referenced by a code + TX, such as code AE 1887196 (BSC-BX60309) + TX, code NNI-0745 GR + TX, code IKI-3106 + TX, code JT-L001 + TX, code ZNQ-08056 + TX, code IPPA152201 + TX, code HNPC-A9908 (CAS: [660411-21-2]) + TX, code HNPC-A2005 (CAS: [860028-12-2]) + TX, code JS118 + TX, code ZJ0967 + TX, code ZJ2242 + TX, code JS7119 (CAS: [929545-74-4]) + TX, code SN-1172 + TX, code HNPC-A9835 + TX, code HNPC-A9955 + TX, code HNPC-A3061 + TX, code Chuanhua 89-1 + TX, code IPP-10 + TX, code ZJ3265 + TX, code JS9117 + TX, code ZJ3757 + TX, code ZJ4042 + TX, code ZJ4014 + TX, code ITM-121 + TX, code DPX-RAB55 (DKI-2301) + TX, code NA-89 + TX, code MIE-1209 + TX, code MCI-8007 + TX, code BCS-CL73507 + TX, code S-1871 + TX, code DPX-RDS63 + TX, code AKD-1193 + TX;
or other biologically active compounds or agents selected from: Quinofumelin + TX, mefentrifluconazol + TX, fenpicoxamid + TX, fluindapyr + TX, inpyrfluxam + TX or indiflumetpyr + TX, isoflucypram + TX, pyrapropoyne + TX, florylpicoxamid + TX, metyltetraprole + TX, ipflufenoquin + TX, pyridachlometyl + TX or chlopyridiflu + TX, tetrachlorantraniliprole + TX, tetrachloraniliprole + TX, Tetflupyrolimet + TX, Triflufenpyrrolidone + TX, Tyclopyrazoflor + TX, flupyrimin + TX or pyrifluramide + TX, benzpyrimoxan + TX, beflubutamid-M + TX, Benzosufyl + TX or oxazosulfyl + TX, etpyrafen + TX, acynonapyr + TX or pyrinonafen + TX, oxotrione + TX, bixlozone + TX or clofendizone + TX or dicloroxizone + TX, cyclopyranil + TX or pyrazocyclonil + TX or cyclopyrazonil + TX , alpha-bromadiolone + TX, Oxathiapiprolin + TX, Fluopyram + TX, Penflufen+ TX, Fluoxopyrosad+ TX, fluoxapiprolin + TX and Flupyradifurone + TX.

The references in brackets behind the active ingredients, e.g. [3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The active ingredient mixture of the compounds of formula I selected from Tables Y-1 to Y-8, X-1 to X-8, U-1 to U-2 and V-1 to V-6 and Table P with active ingredients described above comprises a compound selected from Tables Y-1 to Y-8, X-1 to X-8, U-1 to U-2 and V-1 to V-6 and Table P and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula I selected from Tables Y-1 to Y-8, X-1 to X-8, U-1 to U-2 and V-1 to V-6 and Table P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables Y-1 to Y-8, X-1 to X-8, U-1 to U-2 and V-1 to V-6 and Table P and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula I. The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

### Biological Examples

(Biology data relating to compounds P1 - P6, P9-P13, P15, P18 and P19 are reference examples.)

### Example B1: Activity on Bemisia tabaci (Cotton white fly)

Cotton leaf discs were placed on agar in 24-well microtiter plates and sprayed with aqueous test solutions prepared from 10'000 ppm DMSO stock solutions. After drying the leaf discs were infested with adult white flies. The samples were checked for mortality 6 days after incubation.

The following compounds resulted in at least 80% mortality at an application rate of 200 ppm: P3, P4, P6, P8, P10, P13, P14, and P15.

### Example B2: Activity on Diabrotica balteata (Corn root worm)

Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% in at least one of the two categories (mortality or growth inhibition) at an application rate of 200 ppm:
P3, P4, P5, P7, P8, P10, P11, P12, P13, P14 and P16.

### Example B3: Activity on Euschistus heros (Neotropical Brown Stink Bug)

Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.

The following compounds gave an effect of at least 80% in at least one of the two categories (mortality or growth inhibition) at an application rate of 200 ppm:
P3, P4, P8, P9, P10, P13, P14, and P15.

### Example B4: Activity on Myzus persicae (Green peach aphid):

Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.

The following compounds resulted in at least 80% mortality at an application rate of 200 ppm:
P3, P4, P5, P6, P7, P8, P9, P10, P11, P12, P13, P14, P15, P16, and P17.

### Example B5: Activity on Myzus persicae (Green peach aphid).

Roots of pea seedlings infested with an aphid population of mixed ages were placed directly into aqueous test solutions prepared from 10'000 DMSO stock solutions. The samples were assessed for mortality 6 days after placing seedlings into test solutions.

The following compounds resulted in at least 80% mortality at a test rate of 24 ppm:
P3, P4, P5, P6, P7, P8, P9, P10, P11, P12, P13, P14, P15, P16, and P17.

### Example B6: Activity on Plutella xylostella (Diamond back moth)

24-well microtiter plates with artificial diet were treated with aqueous test solutions prepared from 10'000 ppm DMSO stock solutions by pipetting. After drying, the plates were infested with L2 larvae (10 to 15 per well). The samples were assessed for mortality and growth inhibition in comparison to untreated samples 5 days after infestation.

The following compounds gave an effect of at least 80% in at least one of the two categories (mortality or growth inhibition) at an application rate of 200 ppm:
P3, P4, P5, P6, P7, P8, P11, P12, P14, and P15.

### Example B7: Activity on Spodoptera littoralis (Egyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality, anti-feeding effect, and growth inhibition in comparison to untreated samples 3 days after infestation. Control of Spodoptera littoralis by a test sample is given when at least one of the categories mortality, anti-feedant effect, and growth inhibition is higher than the untreated sample.

The following compounds resulted in at least 80% control at an application rate of 200 ppm:
P3, P4, P5, P7, P8, P11, P12, P14, P15, and P16.

### Example B8: Activity on Nilaparvata Iugens (Brown plant hopper)

Rice plants were treated with the diluted test solutions in a spray chamber. After drying plants were infested with ~20 N3 nymphs. 7 days after the treatment samples were assessed for mortality and growth regulation.
P6, P10, P11, P12, P13, and P15

### Example B9: Activity on Nilaparvata Iugens (Brown plant hopper)

Rice plants cultivated in a nutritive solution were treated with the diluted test solutions into nourishing cultivation system. 1 day after application plants were infested with ~20 N3 nymphs. 7 days after infestation samples were assessed for mortality and growth regulation.
P6, P10, P11, P12, P13, and P15

### Example B10: Activity on Tetranychus urticae (Two-spotted spider mite):

Bean leaf discs on agar in 24-well microtiter plates were sprayed with aqueous test solutions prepared from 10'000 ppm DMSO stock solutions. After drying the leaf discs were infested with a mite population of mixed ages. The samples were assessed for mortality on mixed population (mobile stages) 8 days after infestation.

The following compound resulted in at least 80% mortality at an application rate of 200 ppm:
P3.

### Example B11: Activity on Plutella xylostella (Diamond back moth)

24-well microtiter plates with artificial diet were treated with aqueous test solutions prepared from 10'000 ppm DMSO stock solutions by pipetting. After drying, Plutella eggs were pipetted through a plastic stencil onto a gel blotting paper and the plate was closed with it. The samples were assessed for mortality and growth inhibition in comparison to untreated samples 8 days after infestation.

The following compounds gave an effect of at least 80% in at least one of the two categories (mortality or growth inhibition) at an application rate of 200 ppm:
P16, P17, P18, and P19.

### Example B12: Activity on Heterodera schachtii Juvenile mobility in vitro profiling in 96 well plate

Test solutions are prepared from 10'000 ppm DMSO stock solutions with a TECAN robot to achieve 20 µL of 500, 100, 50, 25, 12.5 and 6.25 ppm. For each concentration three replicates are produced. Per well, 80 µL nematode solution is added containing 100 to 150 freshly harvested second stage juveniles of Heterodera schachtii. The plates are covered and stored at room temperature in the dark and incubated for 48 h. Mobility of the exposed juveniles in a treated well is measured using an imaging tool and compared to an average of 12 untreated replicates.

The following compounds achieved at least 60% control at 100 ppm after 48 h:
P10, P15.

## Claims

1. A compound of formula (I) 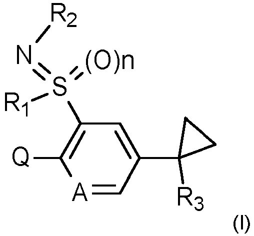 wherein
A is CH or N
R₁ is C₁-C₄ alkyl
R₂ is hydrogen, cyano, -C(O)R7, -C(O)OR8, C₁-C₆alkyl or -CONR₉R₁₀, SO₂R₁₁
wherein
R₇ is hydrogen, C₁-C₆alkyl or C₁-C₆haloalkyl and R₈ is C₁-C₆alkyl or C₁-C₆haloalkyl;
R₉, R₁₀ independently of one another are hydrogen or C₁-C₆alkyl;
R₁₁ is C₁-C₆alkyl;
R₃ is hydrogen, C₁-C₃alkyl, C₁-C₃haloalkyl, cyano, -CO₂H, -CO₂NH₂, C₁-C₄alkoxycarbonyl, C₁-C₄alkylaminocarbonyl, C₁-C₄dialkylaminocarbonyl
n is 0 or 1;
Q is a radical selected from the group consisting of formulae Q₂, Q₃, Q₄ and Q₅ 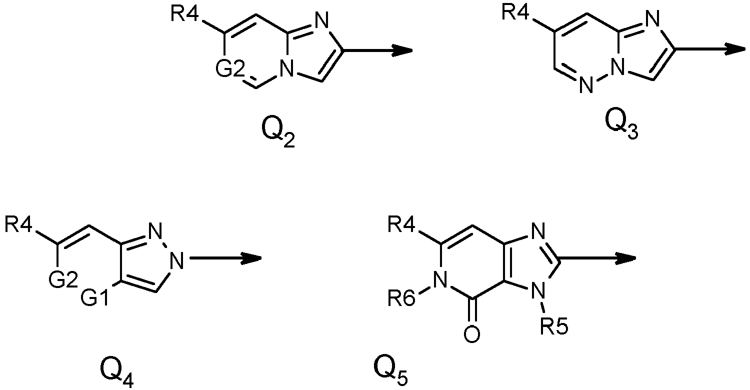
wherein the arrow denotes the point of attachment to the ring incorporating the radical A;
and wherein
R₄ is trifluoromethyl, trifluoromethylsulfanyl or trifluoromethylsulfonyl;
R₅ is C₁-C₄alkyl;
R₆ is OCH₃, CH₂CH₃ or cyclopropyl;
G₁ and G₂ are, independently from each other, N or CH;
or an agrochemically acceptable salt, stereoisomer, enantiomer, tautomer or N-oxide of a compound of formula I.

2. A compound according to claim 1, wherein:
A is CH or N;
R₁ is ethyl, propyl or isopropyl;
R₂ is is hydrogen, cyano, C₁-C₃ alkyl, C₁-C₃alkylcarbonyl, C₁-C₃alkoxycarbonyl, C₁-C₃haloalkylcarbonyl;
R₃ is hydrogen, C₁-C₃haloalkyl, cyano, -CO₂H, -CO₂NH₂, C₁-C₄dialkylaminocarbonyl; and
n is 1.

3. A compound according to claim 1, wherein:
A is CH or N;
R₁ is ethyl;
R₂ is hydrogen;
R₃ is hydrogen, C₁-C₂haloalkyl, cyano, -CO₂NH₂, C₁-C₂dialkylaminocarbonyl; and
n is 1.

4. A compound according to claim 1, wherein:
A is CH or N;
R₁ is ethyl;
R₂ is hydrogen;
R₃ is hydrogen, cyano or CO₂NH₂; and
n is 1.

5. A compound according to claim 1, wherein:
A is CH or N;
R₁ is ethyl;
R₂ is hydrogen;
R₃ is hydrogen or cyano; and
n is 1.

6. A compound according to any one of claims 1 - 5, wherein:
Q is a radical selected from Q₅ 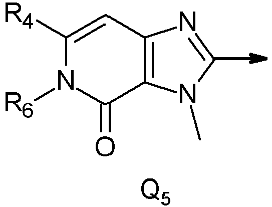
wherein the arrow denotes the point of attachment to the ring incorporating the radical A;
R₄ is trifluoromethyl, trifluoromethylsulfanyl or trifluoromethylsulfonyl; and
R₆ is OCH₃, CH₂CH₃ or cyclopropyl.

7. A compound according to any one of claims 1 - 5, wherein:
Q is a radical selected from Q₄ 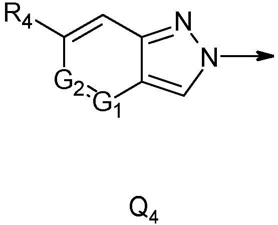
wherein the arrow denotes the point of attachment to the ring incorporating the radical A;
R₄ is trifluoromethyl, trifluoromethylsulfanyl or trifluoromethylsulfonyl; and
G₁ is N and G₂ is CH, G₁ is CH and G₂ is N, G₁ and G₂ are CH, or G₁ and G₂ are N; preferably G₁ is CH and
G₂ is N.

8. A compound of formula (I) according to claim 1 selected from the group consisting of:
[5-cyclopropyl-2-[6-(trifluoromethyl)pyrazolo[4,3-c]pyridin-2-yl]-3-pyridyl]-ethyl-imino-oxo-λ⁶-sulfane (compound P7);
1-[5-(ethylsulfonimidoyl)-6-[5-methoxy-3-methyl-4-oxo-6-(trifluoromethyl)imidazo[4,5-c]pyridin-2-yl]-3-pyridyl]cyclopropanecarbonitrile (compound P8);
1-[6-[5-ethyl-3-methyl-4-oxo-6-(trifluoromethyl)imidazo[4,5-c]pyridin-2-yl]-5-(ethylsulfonimidoyl)-3-pyridyl]cyclopropanecarbonitrile (compound P14);
[5-cyclopropyl-2-[7-(trifluoromethyl)imidazo[1,2-b]pyridazin-2-yl]-3-pyridyl]-ethyl-imino-oxo-λ⁶-sulfane (compound P16); and
1-[5-(ethylsulfonimidoyl)-6-[6-(trifluoromethyl)pyrazolo[4,3-c]pyridin-2-yl]-3-pyridyl]cyclopropanecarbonitrile (compound P17);

9. A composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 8 and, optionally, an auxiliary or diluent.

10. A non-therapeutic method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 8 or a composition as defined claim 9.

11. A method for the protection of plant propagation material from the attack by insects, acarines, nematodes or molluscs, which comprises treating the propagation material or the site, where the propagation material is planted, with a composition according to claim 9.

## Patentansprüche

1. Verbindung der Formel (I) 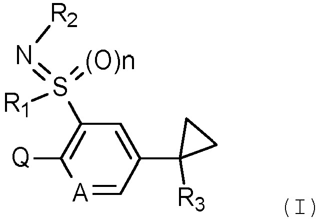 wobei
A für CH oder N steht;
R₁ für C₁-C₄-Alkyl steht;
R₂ für Wasserstoff, Cyano, -C(O)R7, -C(O)OR8, C₁-C₆-Alkyl oder -CONR₉R₁₀ oder SO₂R₁₁ steht;
wobei
R₇ für Wasserstoff, C₁-C₆-Alkyl oder C₁-C₆-Halogenalkyl steht und R₈ für C₁-C₆-Alkyl oder C₁-C₆-Halogenalkyl steht; R₉, R₁₀ unabhängig voneinander für Wasserstoff oder C₁-C₆-Alkyl stehen;
R₁₁ für C₁-C₆-Alkyl steht;
R₃ für Wasserstoff, C₁-C₃-Alkyl, C₁-C₃-Halogenalkyl, Cyano, -CO₂H, -CO₂NH₂, C₁-C₄-Alkoxycarbonyl, C₁-C₄-Alkylaminocarbonyl oder C₁-C₄-Dialkylaminocarbonyl steht; n für 0 oder 1 steht;
Q für einen Rest aus der Gruppe bestehend aus den Formeln Q₂, Q₃, Q₄ und Q₅ 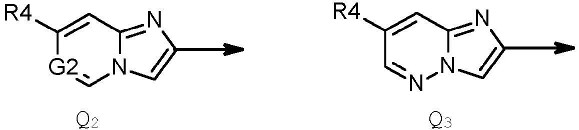 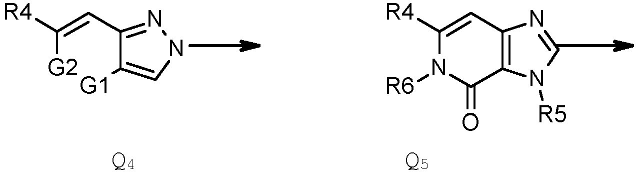
steht, wobei der Pfeil den Verknüpfungspunkt mit dem den Rest A enthaltenden Ring markiert;
und wobei
R₄ für Trifluormethyl, Trifluormethylsulfanyl oder Trifluormethylsulfonyl steht;
R₅ für C₁-C₄-Alkyl steht;
R₆ für OCH₃, CH₂CH₃ oder Cyclopropyl steht;
G₁ und G₂ unabhängig voneinander für N oder CH stehen; oder ein agrochemisch unbedenkliches Salz, Stereoisomer, Enantiomer, Tautomer oder N-Oxid einer Verbindung der Formel I.

2. Verbindung nach Anspruch 1, wobei:
A für CH oder N steht;
R₁ für Ethyl, Propyl oder Isopropyl steht;
R₂ für Wasserstoff, Cyano, C₁-C₃-Alkyl, C₁-C₃-Alkylcarbonyl, C₁-C₃-Alkoxycarbonyl oder C₁-C₃-Halogenalkylcarbonyl steht;
R₃ für Wasserstoff, C₁-C₃-Halogenalkyl, Cyano, -CO₂H, - CO₂NH₂ oder C₁-C₄-Dialkylaminocarbonyl steht; und
n für 1 steht.

3. Verbindung nach Anspruch 1, wobei:
A für CH oder N steht;
R₁ für Ethyl steht;
R₂ für Wasserstoff steht;
R₃ für Wasserstoff, C₁-C₂-Halogenalkyl, Cyano, -CO₂NH₂ oder C₁-C₂-Dialkylaminocarbonyl steht; und
n für 1 steht.

4. Verbindung nach Anspruch 1, wobei:
A für CH oder N steht;
R₁ für Ethyl steht;
R₂ für Wasserstoff steht;
R₃ für Wasserstoff, Cyano oder CO₂NH₂ steht; und
n für 1 steht.

5. Verbindung nach Anspruch 1, wobei:
A für CH oder N steht;
R₁ für Ethyl steht;
R₂ für Wasserstoff steht;
R₃ für Wasserstoff oder Cyano steht; und
n für 1 steht.

6. Verbindung nach einem der Ansprüche 1 - 5, wobei:
Q für einen Rest steht, der aus Q₅ 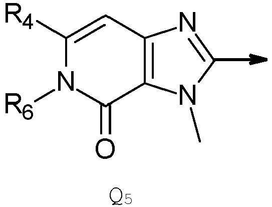
ausgewählt ist, wobei der Pfeil den Verknüpfungspunkt mit dem den Rest A enthaltenden Ring markiert;
R₄ für Trifluormethyl, Trifluormethylsulfanyl oder Trifluormethylsulfonyl steht; und
R₆ für OCH₃, CH₂CH₃ oder Cyclopropyl steht.

7. Verbindung nach einem der Ansprüche 1 - 5, wobei:
Q für einen Rest steht, der aus Q₄ 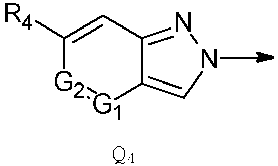
ausgewählt ist, wobei der Pfeil den Verknüpfungspunkt mit dem den Rest A enthaltenden Ring markiert;
R₄ für Trifluormethyl, Trifluormethylsulfanyl oder Trifluormethylsulfonyl steht; und
G₁ für N steht und G₂ für CH steht, G₁ für CH steht und G₂ für N steht, G₁ und G₂ für CH stehen oder G₁ und G₂ für N stehen; vorzugsweise G₁ für CH steht und G₂ für N steht.

8. Verbindung der Formel (I) nach Anspruch 1, ausgewählt aus der Gruppe bestehend aus:
[5-Cyclopropyl-2-[6-(trifluormethyl)pyrazolo[4,3-c]pyridin-2-yl]-3-pyridyl]ethyliminooxo-λ⁶-sulfan (Verbindung P7);
1-[5-(Ethylsulfonimidoyl)-6-[5-methoxy-3-methyl-4-oxo-6-(trifluormethyl)imidazo[4,5-c]pyridin-2-yl]-3-pyridyl]cyclopropancarbonitril (Verbindung P8);
1-[6-[5-Ethyl-3-methyl-4-oxo-6-(trifluormethyl)imidazo[4,5-c]pyridin-2-yl]-5-(ethylsulfonimidoyl)-3-pyridyl]cyclopropancarbonitril (Verbindung P14);
[5-Cyclopropyl-2-[7-(trifluormethyl)imidazo[1,2-b]pyridazin-2-yl]-3-pyridyl]ethyliminooxo-λ⁶-sulfan (Verbindung P16); und
1-[5-(Ethylsulfonimidoyl)-6-[6-(trifluormethyl)pyrazolo[4,3-c]pyridin-2-yl]-3-pyridyl]cyclopropancarbonitril (Verbindung P17);

9. Zusammensetzung, umfassend eine insektizid, akarizid, nematizid oder molluskizid wirksame Menge einer Verbindung der Formel (I) oder eines agrochemisch unbedenklichen Salzes, Stereoisomers, Enantiomers, Tautomers oder N-Oxids davon gemäß einem der Ansprüche 1-8 und gegebenenfalls einen Hilfsstoff oder ein Verdünnungsmittel.

10. Nichttherapeutisches Verfahren zur Bekämpfung und Kontrolle von Insekten, Acarina, Nematoden oder Mollusken, bei dem man auf einen Schädling, einen Ort eines Schädlings oder eine Pflanze, die gegenüber Schädlingsbefall anfällig ist, eine insektizid, akarizid, nematizid oder molluskizid wirksame Menge einer Verbindung der Formel (I) oder eines agrochemisch unbedenklichen Salzes, Stereoisomers, Enantiomers, Tautomers oder N-Oxids davon gemäß einem der Ansprüche 1 - 8 oder einer Zusammensetzung gemäß Anspruch 9 aufbringt.

11. Verfahren zum Schutz von Pflanzenfortpflanzungsmaterial gegen Befall durch Insekten, Acarina, Nematoden oder Mollusken, bei dem man das Fortpflanzungsmaterial oder die Stelle, an der das Fortpflanzungsmaterial angepflanzt ist, mit einer Zusammensetzung nach Anspruch 9 behandelt.

## Revendications

1. Composé de formule (I) 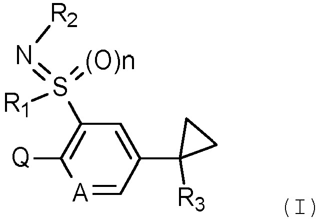
A étant CH ou N
R₁ étant C₁-C₄ alkyle
R₂ étant hydrogène, cyano, -C(O)R7, -C(O)OR8, C₁-C₆alkyle ou -CONR₉R₁₀, SO₂R₁₁
R₇ étant hydrogène, C₁-C₆alkyle ou C₁-C₆halogénoalkyle et R₈ étant C₁-C₆alkyle ou C₁-C₆halogénoalkyle ;
R₉, R₁₀ indépendamment l'un de l'autre étant hydrogène ou C₁-C₆alkyle ;
R₁₁ étant C₁-C₆alkyle ;
R₃ étant hydrogène, C₁-C₃alkyle, C₁-C₃halogénoalkyle, cyano, -CO₂H, -CO₂NH₂, C₁-C₄alcoxycarbonyle, C₁-C₄alkylaminocarbonyle, C₁-C₄dialkylaminocarbonyle n étant 0 ou 1 ;
Q étant un radical choisi dans le groupe constitué par les formules Q₂, Q_{3,} Q₄ et Q₅ 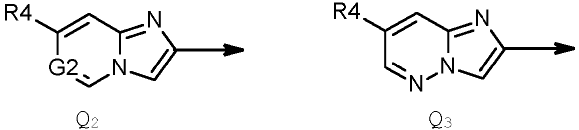 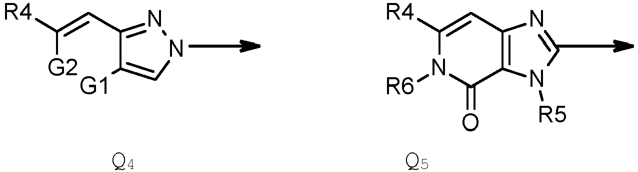
la flèche désignant le point de fixation au cycle incorporant le radical A ;
et
R₄ étant trifluorométhyle, trifluorométhylsulfanyle ou trifluorométhylsulfonyle ;
R₅ étant C₁-C₄alkyle ;
R₆ étant OCH₃, CH₂CH₃ ou cyclopropyle ;
G₁ et G₂ étant, indépendamment l'un de l'autre, N ou CH ; ou sel, stéréoisomère, énantiomère, forme tautomère ou N-oxyde acceptables sur le plan agrochimique d'un composé de formule I.

2. Composé selon la revendication 1,
A étant CH ou N ;
R₁ étant éthyle, propyle ou isopropyle ;
R₂ étant hydrogène, cyano, C₁-C₃ alkyle, C₁-C₃alkylcarbonyle, C₁-C₃alcoxycarbonyle, C₁-C₃halogénoalkylcarbonyle ;
R₃ étant hydrogène, C₁-C₃halogénoalkyle, cyano, -CO₂H, - CO₂NH₂, C₁-C₄dialkylaminocarbonyle ; et
n étant 1.

3. Composé selon la revendication 1,
A étant CH ou N ;
R₁ étant éthyle ;
R₂ étant hydrogène ;
R₃ étant hydrogène, C₁-C₂halogénoalkyle, cyano, -CO₂NH₂, C₁-C₂dialkylaminocarbonyle ; et
n étant 1.

4. Composé selon la revendication 1,
A étant CH ou N ;
R₁ étant éthyle ;
R₂ étant hydrogène ;
R₃ étant hydrogène, cyano ou CO₂NH₂ ; et
n étant 1.

5. Composé selon la revendication 1,
A étant CH ou N ;
R₁ étant éthyle ;
R₂ étant hydrogène ;
R₃ étant hydrogène ou cyano ; et
n étant 1.

6. Composé selon l'une quelconque des revendications 1 - 5,
Q étant un radical choisi parmi Q₅ 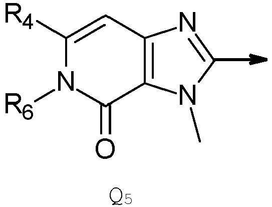
la flèche désignant le point de fixation au cycle incorporant le radical A ;
R₄ étant trifluorométhyle, trifluorométhylsulfanyle ou trifluorométhylsulfonyle ; et
R₆ étant OCH₃, CH₂CH₃ ou cyclopropyle.

7. Composé selon l'une quelconque des revendications 1 - 5,
Q étant un radical choisi parmi Q₄ 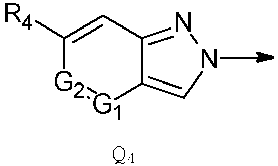
la flèche désignant le point de fixation au cycle incorporant le radical A ;
R₄ étant trifluorométhyle, trifluorométhylsulfanyle ou trifluorométhylsulfonyle ; et
G₁ étant N et G₂ étant CH, G₁ étant CH et G₂ étant N, G₁ et G₂ étant CH, ou G₁ et G₂ étant N ; préférablement G₁ étant CH et G₂ étant N.

8. Composé de formule (I) selon la revendication 1, choisi dans le groupe constitué par :
[5-cyclopropyl-2-[6-(trifluorométhyl)pyrazolo[4,3-c]pyridin-2-y1]-3-pyridiny1]-éthyl-imino-oxo-λ⁶-sulfane (composé P7) ;
1-[5-(éthylsulfonimidoyl)-6-[5-méthoxy-3-méthyl-4-oxo-6-(trifluorométhyl)imidazo[4,5-c]pyridin-2-yl]-3-pyridinyl]cyclopropanecarbonitrile (composé P8) ;
1-[6-[5-éthyl-3-méthyl-4-oxo-6-(trifluorométhyl)imidazo[4,5-c]pyridin-2-yl]-5-(éthylsulfonimidoyl)-3-pyridinyl]cyclopropanecarbonitrile (composé P14) ;
[5-cyclopropyl-2-[7-(trifluorométhyl)imidazo[1,2-b]pyridazin-2-yl]-3-pyridinyl]-éthyl-imino-oxo-λ⁶-sulfane (composé P16) ; et
1-[5-(éthylsulfonimidoyl)-6-[6-(trifluorométhyl)pyrazolo[4,3-c]pyridin-2-yl]-3-pyridinyl]cyclopropanecarbonitrile (composé P17) ;

9. Composition comprenant une quantité efficace sur le plan insecticide, sur le plan acaricide, sur le plan nématicide ou sur le plan molluscicide d'un composé de formule (I) ou d'un sel, d'un stéréoisomère, d'un énantiomère, d'une forme tautomère ou d'un N-oxyde acceptable sur le plan agrochimique correspondant(e), tel que défini dans l'une quelconque des revendications 1-8 et, éventuellement, un auxiliaire ou un diluant.

10. Procédé non thérapeutique de lutte contre des insectes, des acariens, des nématodes ou des mollusques et de régulation d'insectes, d'acariens, de nématodes ou de mollusques qui comprend une application sur un organisme nuisible, sur un site d'un organisme nuisible ou sur un végétal susceptible d'être attaqué par un organisme nuisible d'une quantité efficace sur le plan insecticide, sur le plan acaricide, sur le plan nématicide ou sur le plan molluscicide d'un composé de formule (I) ou d'un sel, d'un stéréoisomère, d'un énantiomère, d'une forme tautomère ou d'un N-oxyde acceptable sur le plan agrochimique correspondant(e), tel que défini dans l'une quelconque des revendications 1 à 8 ou d'une composition telle que définie dans la revendication **9.**

11. Procédé pour la protection d'un matériel de propagation végétale contre l'attaque par des insectes, des acariens, des nématodes ou des mollusques, qui comprend un traitement du matériel de propagation ou du site, où le matériel de propagation est planté, par une composition selon la revendication **9.**
